(19) Europäisches Patentamt
European Patent Office
Office européen des brevets

(11) EP 3 494 113 B1

(12) EUROPÄISCHE PATENTSCHRIFT

(45) Veröffentlichungstag und Bekanntmachung des Hinweises auf die Patenterteilung:
**06.10.2021 Patentblatt 2021/40**

(21) Anmeldenummer: **17758053.7**

(22) Anmeldetag: **01.08.2017**

(51) Int Cl.:
***C07D 403/10*** (2006.01) ***H01L 51/50*** (2006.01)

(86) Internationale Anmeldenummer:
**PCT/EP2017/069433**

(87) Internationale Veröffentlichungsnummer:
**WO 2018/024724 (08.02.2018 Gazette 2018/06)**

(54) **ORGANISCHE MOLEKÜLE ZUR VERWENDUNG IN OPTOELEKTRONISCHEN VORRICHTUNGEN**

ORGANIC MOLECULES FOR USE IN OPTOELECTRONIC DEVICES

MOLECULES ORGANIQUES POUR UTILISATION DANS DES DISPOSITIFS OPTOELECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.08.2016 EP 16182871**

(43) Veröffentlichungstag der Anmeldung:
**12.06.2019 Patentblatt 2019/24**

(73) Patentinhaber: **cynora GmbH**
**76646 Bruchsal (DE)**

(72) Erfinder:
- **SEIFERMANN, Stefan**
  **77815 Bühl (DE)**
- **DANZ, Michael**
  **76344 Eggenstein-Leopoldshafen (DE)**

(74) Vertreter: **Hoppe, Georg Johannes**
**Darani Anwaltskanzlei**
**Beuckestrasse 20**
**14163 Berlin (DE)**

(56) Entgegenhaltungen:
**WO-A1-2016/042070**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 494 113 B1

**Beschreibung**

Die Erfindung betrifft rein organische Moleküle und deren Verwendung in organischen lichtemittierenden Dioden (OLEDs) und in anderen organischen optoelektronischen Vorrichtungen.

**Hintergrund**

Organische optoelektronische Vorrichtungen zeichnen sich dadurch aus, dass entweder elektrische Energie in Photonen umgewandelt wird (Organische Lichtemittierende Dioden, OLED oder Lichtemittierende Elektrochemische Zellen, LEEC) oder der umgekehrte Prozess abläuft (Organische Photovoltaik, OPV). Dabei ist es wichtig, dass diese Prozesse möglichst effizient ablaufen. Für den Bereich von OLEDs müssen daher idealerweise Materialien mit möglichst hoher photolumineszenter Quantenausbeute verwendet werden. Begrenzte Effizienzen von OLED-Materialien können durch Verwendung effizienter Materialien, die thermisch aktivierte verzögerte Fluoreszenz (TADF) zeigen, verbessert werden, da im Gegensatz zu rein fluoreszenten Materialien statt 25 % der in einer OLED gebildeten Exzitonen bis zu 100 % der Exzitonen genutzt werden können. Hierbei können auch die entstandenen Triplett-Excitonen in Singulett-Excitonen überführt werden, aus welchem Zustand dann Photonen emittiert werden können. Voraussetzung für eine solche thermische Rückbesetzung ist dabei ein geringer energetischer Abstand zwischen dem niedrigsten angeregten Singulett-(S1) und Triplett-Niveau (T1). Dies kann beispielweise durch Verwendung von Kupfer-(I)-Komplexen (siehe hierzu z. B.: H. Yersin, U. Monkowius, T. Fischer, T. Hofbeck, WO 2010/149748 A1) aber auch durch rein organische Materialien (siehe hierzu z. B.: Q. Zhang et al., J. Am. Chem. Soc. 2012, 134, 14706, WO 2013161437 A1) erreicht werden WO 2016/042070 offenbart blaues Licht emittierende organische Moleküle zur Verwendung in OLEDs, welche aus einem Carbazolteil und einem Phthalimidteil bestehen. Das unterscheidende Merkmal ist der Rest Rb.

Die intensive Forschung in diesem Bereich zeigt, dass es weiterhin einen großen Bedarf an neuen Materialien gibt. So besteht zum Beispiel nach wie vor Bedarf an tiefblauen und himmelblauen TADF-OLEDs. Bestehende blaue TADF-Materialien zeigen oft hohe Exzitonenlebensdauern und/oder geringe Photolumineszenquantenzausbeuten, welche schlecht für effiziente und langlebige OLEDs sind. Als Maß für eine effiziente blaue OLED kann der Quotient aus der Stromeffizienz in cd/A und dem y-Wert der CIE-Farbkoordinate ($CIE_y$) des von der OLED emittierten Lichts verwendet werden, also eine auf den $CIE_y$-Wert normierte Effizienz. Neben den erwähnten Eigenschaften der Materialien ist für eine Kommerzialisierung ebenso die Zugänglichkeit relevant. Dies schließt die Verfügbarkeit von Synthesebausteinen, als auch den Aufwand für die eigentliche Synthese des funktionellen Materials, insbesondere dessen Aufreinigung mit ein.

**Beschreibung**

Der vorliegenden Erfindung lag die Aufgabe zu Grunde, Moleküle bereitzustellen, die sich zur Verwendung als Emittermaterialien in OLEDs eignen.

Überraschenderweise wurde festgestellt, dass die erfindungsgemäßen organischen Moleküle hohe Quantenausbeuten und geringe Exzitonenlebensdauern aufweisen. Da die Effizienz des Bauteils nach Optimierung des Stack-Designs typischerweise direkt mit der Photolumineszenquantenzausbeute (PLQY) des Emittermaterials korreliert, wurde für die erfindungsgemäßen Moleküle ein technischer Index analog zu dem oben beschriebenen bekannten Effizienzindexes für blaue OLEDs ermittelt. Dieser Blaumaterialindex ("blue material index", BMI) ergibt sich als Quotient aus der PLQY (in %) und der $CIE_y$-Farbkoordinate des von dem erfindungsgemäßen Molekül emittierten Lichts.

In einem ersten Aspekt betrifft die Erfindung ein organisches Molekül, aufweisend

- eine erste chemische Einheit aufweisend eine oder bestehend aus einer Struktur der Formel A1

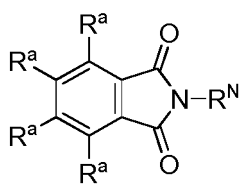

Formel A1

und

- eine zweite chemische Einheit oder zwei zweite chemische Einheiten aufweisend eine oder bestehend aus einer Struktur der Formel D1

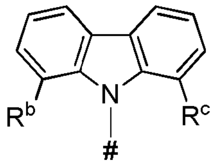

Formel D1

mit

# = Anknüpfungspunkt der zweiten chemischen Einheit an die erste chemische Einheit;

$R^N$ ist Methyl, Phenyl, Xylyl, Mesityl, Naphtyl, Biphenyl, Naphthylphenyl, Terphenyl oder 2,4,6-Triphenylphenyl;

$R^a$ ist bei jedem Auftreten gleich oder verschieden ausgewählt aus der Gruppe bestehend aus Anknüpfungspunkt der ersten chemischen Einheit an die zweite chemische Einheit, H, Deuterium, eine Alkylgruppe und eine unsubstituierte oder mit einem oder mehreren $R^4$ substituierte Arylgruppe, wobei ein oder zwei $R^a$ einen Anknüpfungspunkt der ersten chemischen Einheit an die zweite chemische Einheit darstellt;

$R^b$ ist ausgewählt aus der Gruppe bestehend aus $CF_3$, $C(=O)R^1$, CN, unsubstituiertem oder mit einem oder mehreren $R^2$ substituiertem Carbazolyl, einer unsubstituierten oder mit einem oder mehreren $R^2$ substituierten Arylgruppe, einer unsubstituierten oder mit einem oder mehreren $R^2$ substituierten Heteroarylgruppe;

$R^c$ ist H, Deuterium oder $R^b$;

$R^1$ ist bei jedem Auftreten eine unsubstituierte oder mit einer oder mehreren Resten $R^2$ substituierte Arylgruppe;

$R^2$ ist bei jedem Auftreten unabhängig voneinander ausgewählt aus der Gruppe bestehend aus $CF_3$, $C(=O)R^1$, CN, einer unsubstituierten oder mit einem oder mehreren $R^3$ substituierten Alkylgruppe, einer unsubstituierten oder mit einem oder mehreren $R^3$ substituierten Arylgruppe und einer unsubstituierten oder mit einem oder mehreren $R^3$ substituierten Heteroarylgruppe;

$R^3$ ist bei jedem Auftreten unabhängig voneinander ausgewählt aus der Gruppe bestehend aus $CF_3$, $C(=O)R^1$, CN, einer unsubstituierten Alkylgruppe, einer unsubstituierten Arylgruppe und einer unsubstituierten Heteroarylgruppe;

$R^4$ ist bei jedem Auftreten unabhängig voneinander ausgewählt aus der Gruppe bestehend aus F, $CF_3$, $C(=O)R^1$, CN, einer unsubstituierten Alkylgruppe, einer unsubstituierten Arylgruppe und einer unsubstituierten Heteroarylgruppe.

Es ist möglich, dass zwei oder mehrere Vertreter aus der Gruppe der Substituenten $R^1$, $R^2$ und $R^3$ miteinander ein mono- oder polycyclisches, aliphatisches, aromatisches und/oder benzoannelliertes Ringsystem bilden. Wenn ein organisches Molekül zwei zweite chemische Einheiten aufweist, so sind diese in einer Ausführungsform der Erfindung identisch, in einer anderen Ausführungsform nicht-identisch.

In einer Ausführungsform weist das organisches Molekül eine Struktur der Formel A2 auf oder besteht aus einer Struktur der Formel A2.

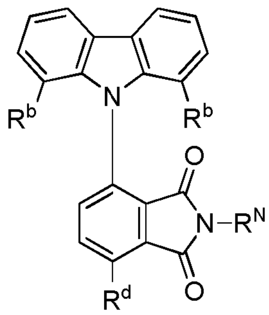

Formel A2

Darin ist

$R^d$ ausgewählt ist aus der Gruppe bestehend aus H und einem Anknüpfungspunkt an eine Gruppe der Formel A2a

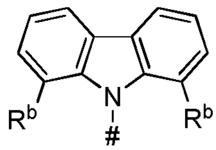

Formel A2a

mit # = Anknüpfungspunkt der chemischen Einheit der Formel A2a an die Einheit der Formel A2; und

$R^b$ und $R^N$ sind wie oben definiert.

In einer weiteren Ausführungsform weist das organisches Molekül eine Struktur der Formel A3 aufweist oder besteht aus einer Struktur der Formel A3.

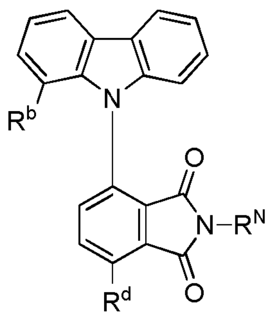

Formel A3

Darin ist

$R^d$ ausgewählt ist aus der Gruppe bestehend aus H und einem Anknüpfungspunkt an eine Gruppe der Formel A2b

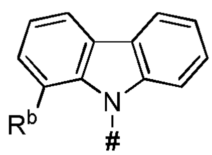

Formel A2b

mit

# = Anknüpfungspunkt der chemischen Einheit der Formel A2b an die Einheit der Formel A3;

$R^b$ und $R^N$ sind wie oben definiert.

In einer weiteren Ausführungsform weist das organisches Molekül eine Struktur der Formel A4 auf oder besteht aus einer Struktur der Formel A4.

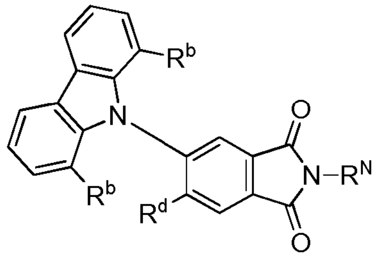

Formel A4

Darin ist

$R^d$ ausgewählt ist aus der Gruppe bestehend aus H und einem Anknüpfungspunkt an eine Gruppe der Formel A2a

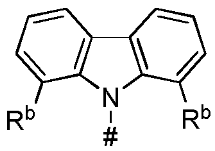

Formel A2a

mit

# = Anknüpfungspunkt der chemischen Einheit der Formel A2a an die Einheit der Formel A4;

$R^b$ und $R^N$ sind wie oben definiert.

In einer weiteren Ausführungsform besteht das organische Molekül aus einer Struktur der Formel A5 oder weist eine Struktur der Formel A5 auf.

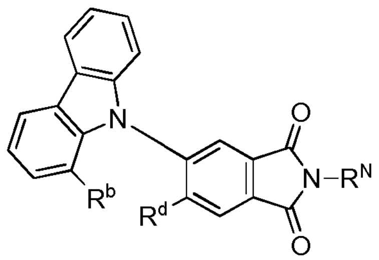

Formel A5

Darin ist

$R^d$ ausgewählt ist aus der Gruppe bestehend aus H und einem Anknüpfungspunkt an eine Gruppe der Formel A2b

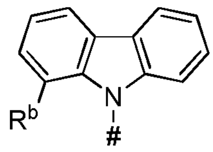

Formel A2b

mit

# = Anknüpfungspunkt der chemischen Einheit der Formel A2b an die Einheit der Formel A5;

$R^b$ und $R^N$ sind wie oben definiert.

Das erfindungsgemäße organische Molekül zeichnet sich in einer Ausführungsform dadurch aus, dass $R^b$ bei jedem Auftreten unabhängig voneinander ausgewählt ist aus der Gruppe bestehend aus einer unsubstituierten Arylgruppe, einer mit einem oder mehreren $R^2$ substituierten Arylgruppe, einer unsubstituierten Hetreroarylgruppe und einer mit $R^2$ substituierten Heteroarylgruppe, wobei die oben angegebenen Definitionen gelten.

In einer weiteren Ausführungsform des organisches Molekül ist $R^b$ bei jedem Auftreten unabhängig voneinander ausgewählt ist aus der Gruppe bestehend aus einer unsubstituierten Arylgruppe, einer mit einem oder mehreren $R^5$ substituierten Arylgruppe, einer unsubstituierten Heteroarylgruppe und einer mit $R^5$ substituierten Heteroarylgruppe, wobei $R^5$ bei jedem Auftreten unabhängig voneinander ausgewählt ist aus der Gruppe bestehend aus $CF_3$, CN, einer unsubstituierten Alkylgruppe, einer unsubstituierten Arylgruppe und einer unsubstituierten Heteroarylgruppe, wobei ansonsten die in Anspruch 1 angegebenen Definitionen gelten.

Beispiele für Strukturen, die die erfindungsgemäßen organischen Moleküle aufweisen oder aus denen die erfindungsgemäßen Moleküle bestehen, sind mit den Formeln A6 und A7 angegeben, wobei hinsichtlich der in den Formeln angegebenen Reste die oben genannten Definitionen gelten.

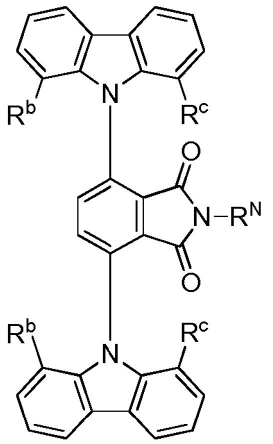

Formel A6

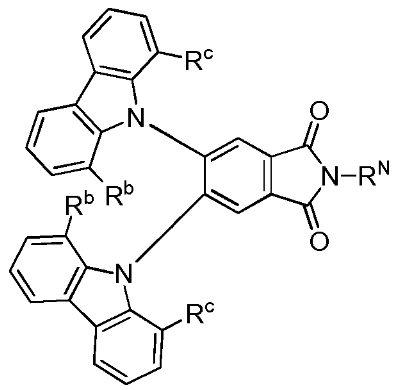

Formel A7

Im Sinne dieser Erfindung enthält eine Arylgruppe 6 bis 60 aromatische Ringatome; eine Heteroarylgruppe enthält 5 bis 60 aromatische Ringatome, von denen mindestens eines ein Heteroatom darstellt. Die Heteroatome sind insbesondere N, O und/oder S. Werden in der Beschreibung bestimmter Ausführungsformen der Erfindung andere, von der genannten Definition abweichende Definitionen angegeben, beispielsweise bezüglich der Zahl der aromatischen Ringatome oder der enthaltenen Heteroatome, so gelten diese.

Unter einer Arylgruppe bzw. Heteroarylgruppe wird ein einfacher aromatischer Cyclus, also Benzol, bzw. ein einfacher heteroaromatischer Cyclus, beispielsweise Pyridin, Pyrimidin oder Thiophen, oder ein heteroaromatischer Polycyclus, beispielsweise Phenanthren, Chinolin oder Carbazol verstanden. Ein kondensierter (annelierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen; Pyrrol, Indol, Isoindol, Carbazol, Pyridin, Chinolin, Isochinolin, Acridin, Phenanthridin, Benzo-5,6-chinolin, Isochinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benztriazol, 1,2,3-Oxadiazol, 1,2,4-Oxadiazol, 1,2,5-Oxadiazol, 1,2,3,4-Tetrazin, Purin, Pteridin, Indolizin und Benzothiadiazol oder Kombinationen der genannten Gruppen.

Unter einer cyclischen Alkyl-, Alkoxy- oder Thioalkoxygruppe wird hier eine monocyclische, eine bicyclische oder eine polycyclische Gruppe verstanden.

Im Rahmen der vorliegenden Erfindung werden unter einer Alkylgruppe beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-0ctyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Die Anbindung der Reste $R^N$ der ersten chemischen Einheit kann über eine beliebige Position erfolgen. Im Sinne dieser Erfindung werden unter Biphenyl beispielsweise ortho-Biphenyl, para-Biphenyl, meta-Biphenyl, unter Terphenyl 1,2-Diphenylphenyl, 1,3-Diphenylphenyl und 1,4-Diphenylphenyl, und unter Naphthylphenyl beispielsweise ortho-Naphthylphenyl, meta-Naphthylphenyl, para-Naphthylphenyl verstanden. Beispielhafte, Ausführungsformen sind:

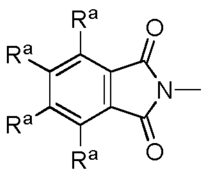 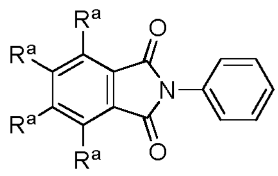 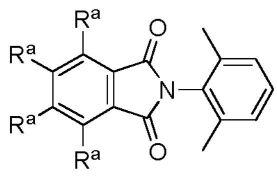 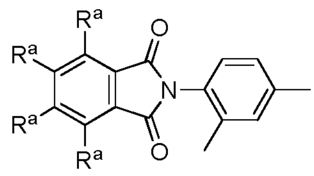

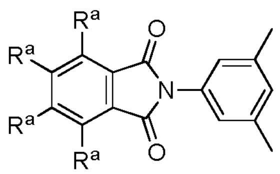 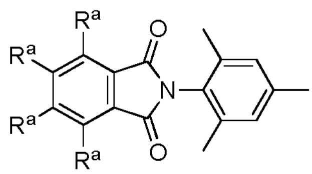 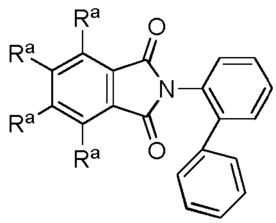

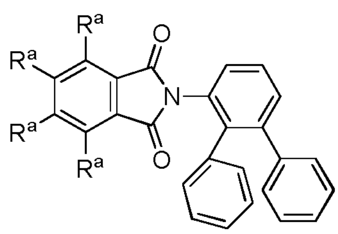 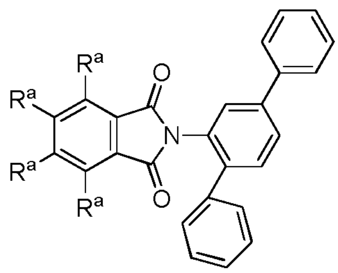 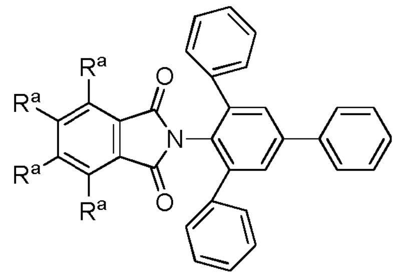

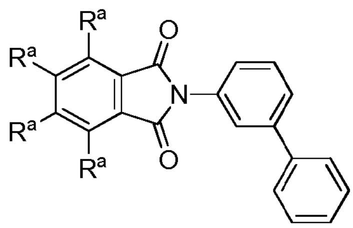 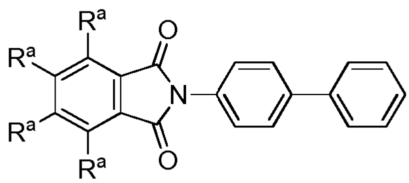 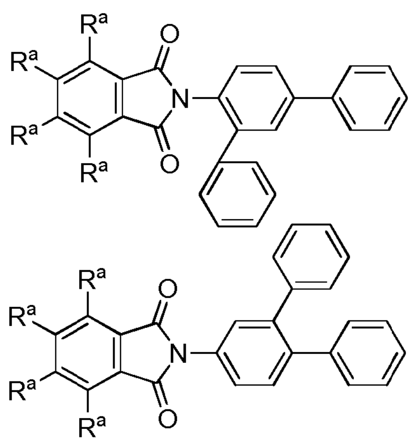

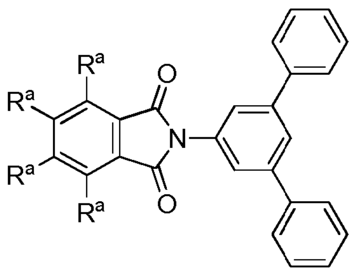 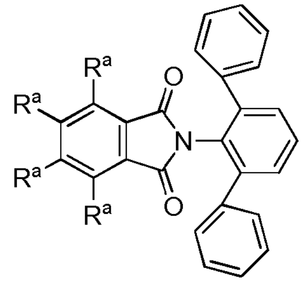

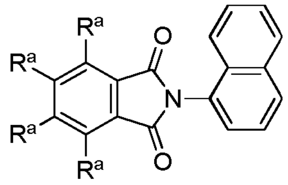
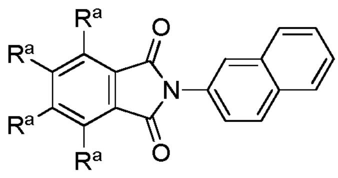
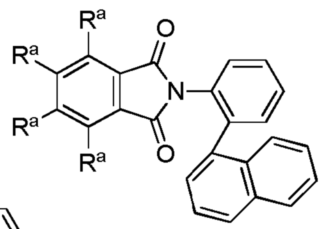
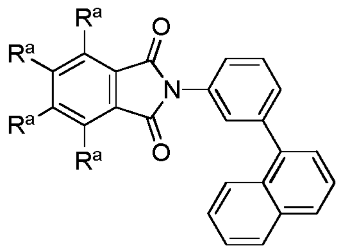
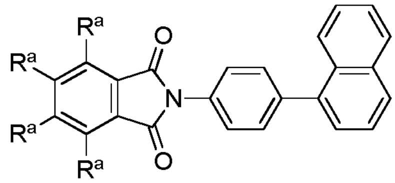

Die erfindungsgemäßen Moleküle weisen hohe Photolumineszenz-Quantenausbeuten (PLQY) und geringe Exzitonenlebensdauern auf und stellen daher vorteilhafte Emittermaterialien für grüne, himmelblaue oder blaue OLEDs dar.

Eine Ausführungsform der Erfindung betrifft organische Moleküle, welche ein Emissionsmaximum bei zwischen 400 und 500 nm, insbesondere zwischen 420 und 481 nm, bevorzugt zwischen 430 und 470 nm, oder zwischen 440 und 460 nm aufweisen.

Insbesondere haben die organischen Moleküle einen $\Delta E(S_1-T_1)$-Wert zwischen dem untersten angeregten Singulett ($S_1$)- und dem darunter liegenden Triplett ($T_1$)-Zustand von nicht höher als 5000 $cm^{-1}$, insbesondere nicht höher als 3000 $cm^{-1}$, oder nicht höher als 1500 $cm^{-1}$ oder 1000 $cm^{-1}$ und/oder eine Emissionslebensdauer von höchstens 150 $\mu s$, insbesondere von höchstens 100 $\mu s$, von höchsten 50 $\mu s$, oder von höchstens 10 $\mu s$ und/oder eine Hauptemissionsbande mit einer Halbwertsbreite kleiner als 120 nm, insbesondere kleiner als 100 nm, kleiner als 80 nm, oder kleiner als 60 nm und/oder eine PhotolumineszenzQuantenausbeute (PLQY) von größer 40 %, insbesondere von größer 50 %, von größer 55 %, von größer 60 %, von größer 65 %, von größer 70 %, oder von größer 80 %.

Die Bestimmung des $\Delta E(S_1-T_1)$-Wertes kann sowohl durch quantenmechanische Berechnungen mittels im Stand der Technik bekannten Computerprogrammen durchgeführt werden (z. B. mittels Turbomole-Programmen unter Ausführung von TD-DFT- und unter Berücksichtigung von CC2-Rechnungen) oder-wie weiter unten erläutert wird - experimentell bestimmt werden.

Die Energiedifferenz $\Delta E(S_1-T_1)$ lässt sich näherungsweise quantenmechanisch durch das mit dem Faktor 2 multiplizierte sogenannte Austauschintegral beschreiben. Dessen Wert hängt direkt ab von der Überlappung der Molekülorbitale. Diese Molekülorbitale sind über unterschiedliche Raumbereiche verteilt (teil-delokalisiert über $\pi$- bzw. $\pi^*$-Molekülorbitale). Das heißt, ein elektronischer Übergang zwischen den verschiedenen Molekülorbitalen repräsentiert einen sogenannten Charge-Transfer (CT)-Übergang. Je geringer die Überlappung der oben beschriebenen Molekülorbitale ist, desto ausgeprägter ist der elektronische Charge-Transfer Charakter. Das ist dann mit einer Abnahme des Austausch-Integrals und somit einer Abnahme der Energiedifferenz $\Delta E(S_1-T_1)$ verbunden.

Die Bestimmung des $\Delta E(S_1-T_1)$-Wertes kann experimentell folgendermaßen erfolgen:
Für ein vorgegebenes organisches Molekül lässt sich der Energieabstand $\Delta E(S_1-T_1) = \Delta E$ unter Verwendung der oben angegebenen Gleichung (1) einfach bestimmen. Eine Umformung ergibt:

$$\ln\{Int(S_1 \rightarrow S_0)/Int(T_1 \rightarrow S_0)\} = \ln\{k(S_1)/k(T_1)\} - (\Delta E/k_B)(1/T) \qquad (3)$$

Für die Messung der Intensitäten $Int(S_1 \rightarrow S_0)$ und $Int(T_1 \rightarrow S_0)$ kann jedes handelsübliche Spektralphotometer verwendet werden. Eine graphische Auftragung der bei verschiedenen Temperaturen gemessenen (logarithmierten) Intensitätsverhältnisse $\ln\{Int(S_1 \rightarrow S_0)/Int(T_1 \rightarrow S_0)\}$ gegen den Kehrwert der absoluten Temperatur T ergibt in der Regel eine Gerade. Die Messung wird in einem Temperaturbereich von Raumtemperatur (300 K) bis 77 K oder bis 4,2 K durchgeführt, wobei die Temperatur mittels eines Kryostaten eingestellt wird. Die Intensitäten werden aus den (korrigierten) Spektren bestimmt, wobei $Int(S_1 \rightarrow S_0)$ bzw. $Int(T_1 \rightarrow S_0)$ die integrierten Fluoreszenz- bzw. Phosphoreszenz-Bandenintensitäten repräsentieren, welche sich mittels der zum Spektralphotometer gehörenden Programme bestimmen lassen. Die jeweiligen Übergänge (Bandenintensitäten) lassen sich leicht identifizieren, da die Triplett-Bande bei niedrigerer Energie liegt als die Singulett-Bande und mit sinkender Temperatur an Intensität gewinnt. Dabei werden die Messungen in sauerstofffreien verdünnten Lösungen (ca. $10^{-2}$ mol/L) oder an dünnen Filmen aus den entsprechenden

Molekülen oder an mit den entsprechenden Molekülen dotierten Filmen durchgeführt. Verwendet man als Probe eine Lösung, so empfiehlt es sich, ein Lösemittel bzw. Lösemittelgemisch zu verwenden, das bei tiefen Temperaturen Gläser bildet, wie 2-Methyl-THF, THF (Tetrahydrofuran) oder aliphatische Kohlenwasserstoffe. Verwendet man als Probe einen Film, so eignet sich die Verwendung einer Matrix mit einer deutlich größeren Singulettsowie Triplett-Energie als die der organischen Emittermoleküle, z. B. PMMA (Polymethylmethacrylat). Dieser Film kann aus Lösung aufgebracht werden.

Die Geradensteigung beträgt $—\Delta E/k_B$. Mit $k_B = 1{,}380\ 10^{-23}\ JK^{-1} = 0{,}695\ cm^{-1}\ K^{-1}$ lässt sich der Energieabstand direkt bestimmen.

Eine äquivalente Betrachtungsweise zeigt, dass mittels der Messung der Temperaturabhängigkeit der Emissionsabklingzeit der $\Delta E(S_1\text{-}T_1)$-Wert auch bestimmt werden kann.

Eine einfache, näherungsweise Abschätzung des $\Delta E(S_1\text{-}T_1)$-Wertes kann auch dadurch vorgenommen werden, dass bei tiefer Temperatur (z. B. 77 K oder 4,2 K unter Verwendung eines Kryostaten) die Fluoreszenz- und Phosphoreszenz-Spektren registriert werden. Der $\Delta E(S_1\text{-}T_1)$-Wert entspricht dann in Näherung der Energiedifferenz zwischen den hochenergetischen Anstiegsflanken der Fluoreszenz- bzw. Phosphoreszenz-Bande.

Je ausgeprägter der CT-Charakter eines organischen Moleküls ist, desto stärker verändern sich die elektronischen Übergangsenergien als Funktion der Lösungsmittelpolarität. So gibt bereits eine ausgeprägte Polaritätsabhängigkeit der Emissionsenergien einen Hinweis auf das Vorliegen kleiner $\Delta E(S_1\text{-}T_1)$-Werte.

In einem weiteren Aspekt betrifft die Erfindung die Verwendung der organischen Moleküle als lumineszierender Emitter oder als Hostmaterial in einer organischen optoelektronischen Vorrichtung, insbesondere wobei die organische optoelektronische Vorrichtung ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gasund Dampf-Sensoren,
- organischen Dioden,
- organischen Solarzellen,
- organischen Transistoren,
- organischen Feldeffekttransistoren,
- organischen Lasern und
- Down-Konversions-Elementen.

In einem weiteren Aspekt betrifft die Erfindung eine Zusammensetzung aufweisend oder bestehend aus:

(a) mindestens einem erfindungsgemäßen organischen Molekül, insbesondere als Emitter und/oder Host, und

(b) mindestens einem, d. h. einem oder mehreren Emitter- und/oder Hostmaterialien, die von dem erfindungsgemäßen organischen Molekül verschiedenen ist bzw. sind und

(c) optional einem oder mehreren Farbstoffen und/oder einem oder mehreren organischen Lösungsmitteln.

In einer Ausführungsform besteht die erfindungsgemäße Zusammensetzung aus einem erfindungsgemäßen organischen Molekül und einem oder mehreren Hostmaterialien. Das oder die Hostmaterialen weisen insbesondere Triplett ($T_1$)- und Singulett ($S_1$)- Energieniveaus auf, die energetisch höher liegen als die Triplett ($T_1$)- und Singulett ($S_1$)-Energieniveaus des erfindungsgemäßen organischen Moleküls. In einer Ausführungsform weist die Zusammensetzung neben dem erfindungsgemäßen organischen Molekül ein elektronendominantes und ein lochdominantes Hostmaterial auf. Das höchste besetzte Orbital (HOMO) und das niedrigste unbesetzte Orbital (LUMO) des lochdominanten Hostmaterials liegen energetisch insbesondere höher als das jeweilige des elektronendominanten Hostmaterials. Das HOMO des lochdominanten Hostmaterials liegt energetisch unter dem HOMO des erfindungsgemäßen organischen Moleküls, während das LUMO des elektronendominanten Hostmaterials energetisch über dem LUMO des erfindungsgemäßen organischen Moleküls liegt. Um Exciplex-Formation zwischen Emitter und Hostmaterial oder Hostmaterialien zu vermeiden, sollten die Materialien so gewählt sein, dass die Energieabstände zwischen den jeweiligen Orbitalen gering sind. Der Abstand zwischen dem LUMO des elektronendominanten Hostmaterials und dem LUMO des erfindungsgemäßen organischen Moleküls beträgt insbesondere weniger als 0,5 eV, bevorzugt weniger als 0,3 eV, noch bevorzugter weniger als 0,2 eV. Der Abstand zwischen dem HOMO des lochdominanten Hostmaterials und dem HOMO des erfindungsgemäßen organischen Moleküls beträgt insbesondere weniger als 0,5 eV, bevorzugt weniger als 0,3 eV, noch bevorzugter weniger als 0,2 eV.

In einem weiteren Aspekt betrifft die Erfindung eine organische optoelektronische Vorrichtung, die ein erfindungsgemäßes organisches Molekül oder eine erfindungsgemäße Zusammensetzung aufweist. Die organische optoelektronische Vorrichtung ist insbesondere ausgeformt als eine Vorrichtung ausgewählt aus der Gruppe bestehend aus

organischer lichtemittierender Diode (OLED); lichtemittierender elektrochemischer Zelle; OLED-Sensor, insbesondere nicht hermetisch nach außen abgeschirmten Gas- und Dampf-Sensoren; organischer Diode; organischer Solarzelle; organischem Transistor; organischem Feldeffekttransistor; organischem Laser und Down-Konversion-Element.

Eine organische optoelektronische Vorrichtung aufweisend

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die ein erfindungsgemäßes organisches Molekül aufweist, stellt einen weitere Ausführungsform der Erfindung dar.

In einer Ausführungsform handelt es sich bei der optoelektronischen Vorrichtung um eine OLED. Eine typische OLED weist beispielsweise folgenden Schichtaufbau auf:

1. Substrat (Trägermaterial)
2. Anode
3. Lochinjektionsschicht (hole injection layer, HIL)
4. Lochtransportschicht (hole transport layer, HTL)
5. Elektronenblockierschicht (electron blocking layer, EBL)
6. Emitterschicht (emitting layer, EML)
7. Lochblockierschicht (hole blocking layer, HBL)
8. Elektronenleitschicht (electron transport layer, ETL)
9. Elektroneninjektionsschicht (electron injection layer, EIL)
10. Kathode.

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.

Gemäß einer Ausführungsform ist mindestens eine Elektrode des organischen Bauelements transluzent ausgebildet. Hier wird mit "transluzent" eine Schicht bezeichnet, die durchlässig für sichtbares Licht ist. Dabei kann die transluzente Schicht klar durchscheinend, also transparent, oder zumindest teilweise Licht absorbierend und/oder teilweise Licht streuend sein, so dass die transluzente Schicht beispielsweise auch diffus oder milchig durchscheinend sein kann. Insbesondere ist eine hier als transluzent bezeichnete Schicht möglichst transparent ausgebildet, so dass insbesondere die Absorption von Licht so gering wie möglich ist.

Gemäß einer weiteren Ausführungsform weist das organische Bauelement, insbesondere eine OLED, einen invertierten Aufbau auf. Der invertierte Aufbau zeichnet sich dadurch aus, dass sich die Kathode auf dem Substrat befindet und die anderen Schichten entsprechend invertiert aufgebracht werden:

1. Substrat (Trägermaterial)
2. Kathode
3. Elektroneninjektionsschicht (electron injection layer, EIL)
4. Elektronenleitschicht (electron transport layer, ETL)
5. Lochblockierschicht (hole blocking layer, HBL)

6. Emissionsschicht bzw. Emitterschicht (emitting layer, EML)

7. Elektronenblockierschicht (electron blocking layer, EBL)

8. Lochtransportschicht (hole transport layer, HTL)

9. Lochinjektionsschicht (hole injection layer, HIL)

10. Anode.

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.

In einer Ausführungsform wird bei der invertierten OLED die Anodenschicht des typischen Aufbaus, z. B. eine ITO-Schicht (Indium-Zinn-Oxid), als Kathode geschaltet.

Gemäß einer weiteren Ausführungsform weist das organische Bauelement, insbesondere eine OLED, einen gestapelten Aufbau auf. Hierbei werden die einzelnen OLEDs übereinander und nicht wie üblich nebeneinander angeordnet. Durch einen gestapelten Aufbau kann die Erzeugung von Mischlicht ermöglicht werden. Beispielsweise kann dieser Aufbau bei der Erzeugung von weißem Licht eingesetzt werden, für dessen Erzeugung das gesamte sichtbare Spektrum typischerweise durch die Kombination des emittierten Lichts von blauen, grünen und roten Emittern abgebildet wird. Weiterhin können bei praktisch gleicher Effizienz und identischer Leuchtdichte signifikant längere Lebensdauern im Vergleich zu üblichen OLEDs erzielt werden. Für den gestapelten Aufbau wird optional eine sogenannte Ladungserzeugungsschicht (charge generation layer, CGL) zwischen zwei OLEDs eingesetzt. Diese besteht aus einer n-dotierten und einem p-dotierten Schicht, wobei die n-dotierte Schicht typischerweise näher an der Anode aufgebracht wird.

In einer Ausführungsform - einer sogenannten Tandem-OLED - treten zwei oder mehr Emissionsschichten zwischen Anode und Kathode auf. In einer Ausführungsform sind drei Emissionsschichten übereinander angeordnet, wobei eine Emissionsschicht rotes Licht emittiert, eine Emissionsschicht grünes Licht emittiert und eine Emissionsschicht blaues Licht emittiert und optional weitere Ladungserzeugungs-, Blockier- oder Transportschichten zwischen den einzelnen Emissionsschichten aufgebracht sind. In einer weiteren Ausführungsform werden die jeweiligen Emissionsschichten direkt angrenzend aufgebracht. In einer weiteren Ausführungsform befindet sich jeweils eine Ladungserzeugungsschicht zwischen den Emissionsschichten. Weiterhin können in einer OLED direkt angrenzende und durch Ladungserzeugungsschichten getrennte Emissionsschichten kombiniert werden.

Über den Elektroden und den organischen Schichten kann weiterhin noch eine Verkapselung angeordnet sein. Die Verkapselung kann beispielsweise in Form eines Glasdeckels oder in Form einer Dünnschichtverkapselung ausgeführt sein.

Als Trägermaterial der optoelektronischen Vorrichtung kann beispielsweise Glas, Quarz, Kunststoff, Metall, ein Siliziumwafer oder jedes andere geeignete feste oder flexible, optional durchsichtige Material dienen.

Das Trägermaterial kann beispielsweise ein oder mehrere Materialien in Form einer Schicht, einer Folie, einer Platte oder einem Laminat aufweisen.

Als Anode der optoelektronischen Vorrichtung können beispielsweise transparente leitende Metalloxide wie beispielsweise ITO (Indium-Zinn-Oxid), Zinkoxid, Zinnoxid, Cadmiumoxid, Titanoxid, Indiumoxid oder Aluminiumzinkoxid (AZO), $Zn_2SnO_4$, $CdSnO_3$, $ZnSnO_3$, $MgIn_2O_4$, $GaInO_3$, $Zn_2In_2O_5$ oder $In_4Sn_3O_{12}$ oder Mischungen unterschiedlicher transparenter leitender Oxide dienen.

Als Materialien einer HIL können beispielsweise PEDOT:PSS (Poly-3,4-ethylendioxythiophen:Polystyrolsulfonsäure), PEDOT (Poly-3,4-ethylendioxythiophen), m-MTDATA (4,4',4"-Tris[phenyl(m-tolyl)amino]triphenylamin), Spiro-TAD (2,2',7,7'-Tetrakis(N,N-diphenylamino)-9,9-spirobifluoren), DNTPD (4,4'-Bis[N-[4-{N,N-bis(3-methylphenyl)amino}phenyl]-N-phenylamino]biphenyl), NPB (N,N'-Bis-(1-naphthalenyl)-N,N'-bisphenyl-(1,1'-biphenyl)-4,4'-diamin), NPNPB (N,N'-Diphenyl-N,N'-di-[4-(N,N-diphenyl-amino)phenyl]benzol), MeO-TPD (N,N,N',N'-Tetrakis(4-methoxyphenyl)benzol), HAT-CN (1,4,5,8,9,11-Hexaazatriphenylen-hexacarbonitril) oder Spiro-NPD (N,N'-diphenyl-N,N'-Bis-(1-naphthyl)-9,9'-spirobifluorene-2,7-diamin) dienen. Beispielhaft ist die Schichtdicke 10-80 nm. Des Weiteren können kleine Moleküle verwendet werden (z. B. Kupfer-Phthalocyanin (CuPc z. B. 10 nm dick)) oder Metalloxide wie beispielhaft $MoO_3$, $V_2O_5$.

Als Materialien einer HTL können tertiäre Amine, Carbazolderivate, mit Polystyrolsulfonsäure dotiertes Polyethylendioxythiophen, mit Camphersulfonsäure dotiertes Polyanilin poly-TPD (Poly(4-butylphenyl-diphenyl-amin)), [alpha]-NPD (Poly(4-butylphenyl-diphenyl-amin)), TAPC (4,4'-Cyclohexyliden-bis[*N*,*N*-bis(4-methylphenyl)benzenamin]), TCTA (Tris(4-carbazoyl-9-ylphenyl)amin), 2-TNATA (4,4',4"-Tris[2-naphthyl(phenyl)amino]triphenylamin), Spiro-TAD, DNTPD, NPB, NPNPB, MeO-TPD, HAT-CN oder TrisPcz (9,9'-Diphenyl-6-(9-phenyl-9H-carbazol-3-yl)-9H,9'H-3,3'-bicarbazol) dienen. Beispielhaft ist die Schichtdicke 10 nm bis100 nm.

Die HTL kann eine p-dotierte Schicht aufweisen, die einen anorganischen oder organischen Dotierstoff in einer

organischen löcherleitenden Matrix aufweist. Als anorganischer Dotierstoff können beispielsweise Übergangsmetalloxide wie etwa Vanadiumoxid, Molybdänoxid oder Wolframoxid genutzt werden. Als organische Dotierstoffe können beispielsweise Tetrafluorotetracyanoquinodimethan (F4-TCNQ), Kupfer-Pentafluorobenzoat (Cu(I)pFBz) oder Übergangsmetallkomplexe verwendet werden. Beispielhaft ist die Schichtdicke 10 nm bis 100 nm.

Als Materialien einer Elektronenblockierschicht können beispielsweise mCP (1,3-Bis(carbazol-9-yl)benzol), TCTA, 2-TNATA, mCBP (3,3-Di(9H-carbazol-9-yl)biphenyl), tris-Pcz (9,9'-Diphenyl-6-(9-phenyl-9H-carbazol-3-yl)-9H,9'H-3,3'-bicarbazol), CzSi (9-(4-tert-Butylphenyl)-3,6-bis(triphenylsilyl)-9H-carbazol) oder DCB (N,N'-Dicarbazolyl-1,4-dimethylbenzol) dienen. Beispielhaft ist die Schichtdicke 10 nm bis 50 nm.

Die Emitter-Schicht EML oder Emissionsschicht besteht aus oder enthält Emittermaterial oder eine Mischung aufweisend mindestens zwei Emittermaterialien und optional ein oder mehreren Hostmaterialien. Geeignete Hostmaterialien sind beispielsweise mCP, TCTA, 2-TNATA, mCBP, CBP (4,4'-Bis-(N-carbazolyl)-biphenyl), Sif87 (Dibenzo[b,d]thiophen-2-yltriphenylsilan), Sif88 (Dibenzo[b,d]thiophen-2-yl)diphenylsilan) oder DPEPO (Bis[2-((oxo)diphenylphosphino)phenyl]ether). Für im Grünen oder im Roten emittierendes Emittermaterial oder einer Mischung aufweisend mindestens zwei Emittermaterialien eignen sich die gängigen Matrixmaterialien wie CBP. Für im Blauen emittierendes Emittermaterial oder einer Mischung aufweisend mindestens zwei Emittermaterialien können UHG-Matrixmaterialien (Ultra-High energy Gap Materialien) (siehe z. B. M.E. Thompson et al., Chem. Mater. 2004, 16, 4743) oder andere sogenannten Wide-Gap-Matrixmaterialien eingesetzt werden. Beispielhaft ist die Schichtdicke 10 nm bis 250 nm.

Die Lochblockierschicht HBL kann beispielsweise BCP (2,9-Dimethyl-4,7-diphenyl-1,10-phenanthrolin = Bathocuproin), Bis-(2-methyl-8-hydroxychinolinato)-(4-phenylphenolato)-aluminium(III) (BAlq), Nbphen (2,9-Bis(naphthalen-2-yl)-4,7-diphenyl-1,10-phenanthrolin), Alq3 (Aluminium-tris(8-hydroxychinolin)), TSPO1 (Diphenyl-4-triphenylsilylphenyl-phosphinoxid) oder TCB/TCP (1,3,5-Tris(N-carbazolyl)benzol/ 1,3,5-tris(carbazol)-9-yl) benzol) aufweisen. Beispielhaft ist die Schichtdicke 10 nm bis 50 nm.

Die Elektronentransportschicht ETL kann beispielsweise Materialien auf Basis von $AlQ_3$, TSPO1, BPyTP2 (2,7-Di(2,2'-bipyridin-5-yl)triphenyl) ), Sif87, Sif88, BmPyPhB (1,3-Bis[3,5-di(pyridin-3-yl)phenyl]benzol) oder BTB (4,4'-Bis-[2-(4,6-diphenyl-1,3,5-triazinyl)]-1,1'-biphenyl) aufweisen. Beispielhaft ist die Schichtdicke 10 nm bis 200 nm.

Als Materialien einer dünnen Elektroneninjektionsschicht EIL können beispielsweise CsF, LiF, 8-Hydroxyquinolinolatolithium (Liq), $Li_2O$, $BaF_2$, MgO oder NaF verwendet werden.

Als Materialien der Kathodenschicht können Metalle oder Legierungen dienen, beispielsweise Al, Al > AlF, Ag, Pt, Au, Mg, Ag:Mg. Typische Schichtdicken betragen 100 nm bis 200 nm. Insbesondere werden ein oder mehrere Metalle verwendet, die stabil an der Luft sind und/oder die selbstpassivierend, beispielsweise durch Ausbildung einer dünnen schützenden Oxidschicht, sind.

Als Materialien zu Verkapselung sind beispielsweise Aluminiumoxid, Vanadiumoxid, Zinkoxid, Zirkoniumoxid, Titanoxid, Hafniumoxid, Lanthanoxid, Tantaloxid geeignet.

In einer Ausführungsform der erfindungsgemäßen organischen optoelektronischen Vorrichtung ist das erfindungsgemäße organische Molekül als Emissionsmaterial in einer lichtemittierenden Schicht EML eingesetzt, wobei es entweder als Reinschicht oder in Kombination mit einem oder mehreren Hostmaterialien eingesetzt ist.

Der Massenanteil des erfindungsgemäßen organischen Moleküls an der Emitter-Schicht EML beträgt in einer weiteren Ausführungsform in einer lichtemittierenden Schicht in optischen Licht emittierenden Vorrichtungen, insbesondere in OLEDs, zwischen 1 % und 80 %. In einer Ausführungsform der erfindungsgemäßen organischen optoelektronischen Vorrichtung ist die lichtemittierende Schicht auf ein Substrat aufgebracht, wobei bevorzugt eine Anode und eine Kathode auf das Substrat aufgebracht sind und die lichtemittierende Schicht zwischen Anode und Kathode aufgebracht ist.

Die lichtemittierende Schicht kann ausschließlich ein erfindungsgemäßes organisches Molekül in 100 % Konzentration aufweisen, wobei die Anode und die Kathode auf das Substrat aufgebracht sind, und die lichtemittierende Schicht zwischen Anode und Kathode aufgebracht ist.

In einer Ausführungsform der erfindungsgemäßen organischen optoelektronischen Vorrichtung sind eine löcher- und elektroneninjizierende Schicht zwischen Anode und Kathode, und eine löcher- und elektronentransportierende Schicht zwischen löcher- und elektroneninjizierende Schicht, und die lichtemittierende Schicht zwischen löcher- und elektronentransportierender Schicht aufgebracht.

Die organische optoelektronische Vorrichtung weist in einer weiteren Ausführungsform der Erfindung auf: ein Substrat, eine Anode, eine Kathode und mindestens je eine löcher- und elektroneninjizierende Schicht, und mindestens je eine löcher- und elektronentransportierende Schicht, und mindestens eine lichtemittierende Schicht, die ein erfindungsgemäßes organisches Molekül und ein oder mehrere Hostmaterialen aufweist, deren Triplett (T1)- und Singulett (S1)-Energieniveaus energetisch höher liegen als die Triplett (T1)- und Singulett (S1)-Energieniveaus des organischen Moleküls, wobei die Anode und die Kathode auf das Substrat aufgebracht ist, und die löcher- und elektroneninjizierende Schicht zwischen Anode und Kathode aufgebracht ist, und die löcher- und elektronentransportierende Schicht zwischen löcher- und elektroneninjizierende Schicht aufgebracht ist, und die lichtemittierende Schicht zwischen löcher- und elektronentransportierende Schicht aufgebracht ist.

In einem weiteren Aspekt betrifft die Erfindung ein Verfahren zur Herstellung eines optoelektronischen Bauelements. Dabei wird ein erfindungsgemäßes organisches Molekül verwendet.

In einer Ausführungsform umfasst das Herstellungsverfahren die Verarbeitung des erfindungsgemäßen organischen Moleküls mittels eines Vakuumverdampfungsverfahrens oder aus einer Lösung.

Erfindungsgemäß ist auch ein Verfahren zur Herstellung einer erfindungsgemäßen optoelektronischen Vorrichtung, bei dem mindestens eine Schicht der optoelektronischen Vorrichtung

- mit einem Sublimationsverfahren beschichtet wird,
- mit einem OVPD (Organic Vapor Phase Deposition) Verfahren beschichtet wird,
- mit einer Trägergassublimation beschichtet wird, und/oder
- aus Lösung oder mit einem Druckverfahren hergestellt wird.

Bei der Herstellung der erfindungsgemäßen optoelektronischen Vorrichtung werden bekannte Verfahren eingesetzt. Generell werden die Schichten einzeln auf ein geeignetes Substrat in aufeinanderfolgenden Abscheideverfahrensschritten aufgebracht. Bei der Gasphasenabscheidung können die gebräuchlichen Methoden, wie thermische Verdampfung, chemische Gasphasenabscheidung (CVD), physikalische Gasphasenabscheidung (PVD) angewendet werden. Für active matrix OLED Displays erfolgt die Abscheidung auf eine AMOLED Backplane als Substrat.

Alternativ können Schichten aus Lösungen oder Dispersionen in geeigneten Lösungsmitteln aufgebracht werden. Beispielhafte geeignete Beschichtungsverfahren sind Rotationsbeschichtung (spin-coating), Tauchbeschichtung (dip-coating) und Strahldruckmethoden. Die einzelnen Schichten können erfindungsgemäß sowohl über dasselbe als auch über jeweils verschiedene Beschichtungsmethoden hergestellt werden.

Durch die nachfolgenden Beispiele wird die Erfindung wird nun näher erläutert, ohne sie dadurch einschränken zu wollen.

**Beispiele**

**Allgemeine** Vorschriften

AAV1:

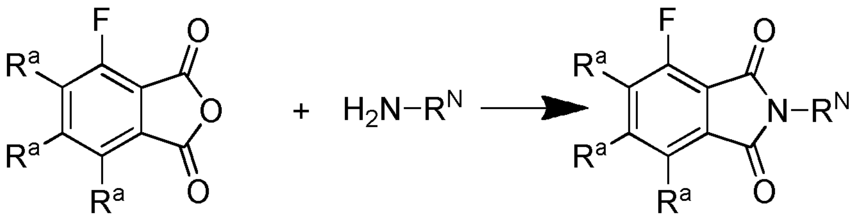

**E1** **A1** **E2**

In einem Rundkolben mit Rückflusskühler wird **E1** (1 Äquivalent) in Eisessig suspendiert. Nach Zugabe von **A1** (1,1 Äquivalente) wird 3 Stunden bei 100 °C gerührt. Nach abkühlen wird die Reaktionslösung am Rotationsverdampfer soweit möglich eingeengt. Der Rückstand wird in $CH_2Cl_2$ aufgenommen und zweimal mit ges. $Na_2CO_3$ gewaschen. Die vereinigten organischen Phasen werden über $MgSO_4$ getrocknet. Das Lösungsmittel wird am Rotationsverdampfer entfernt. Nach Trocknen am Hochvakuum wird **E2** als Produkt erhalten, welches in der Regel ohne weitere Aufreinigung eingesetzt werden kann. Bei Bedarf kann das Produkt **E2** durch Umkristallisation weiter aufgereinigt werden.

**Beispielhafte A1 und E2 Kombinationen**

| A1 | E2 |
|---|---|
| 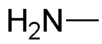 | 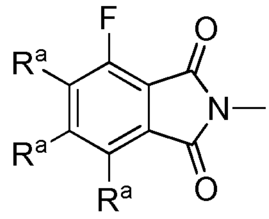 |
| 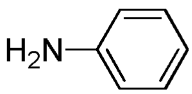 | 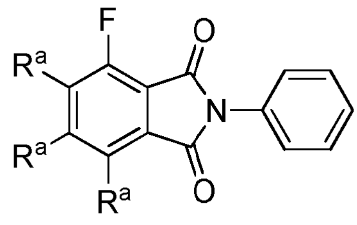 |
| 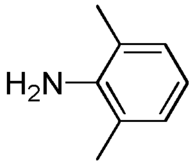 | 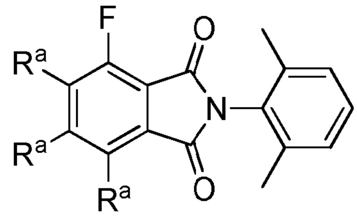 |
| 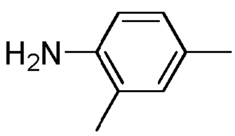 | 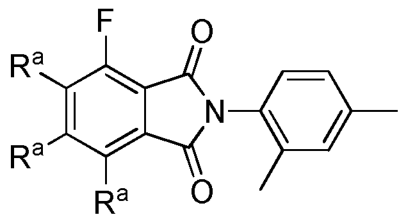 |
| 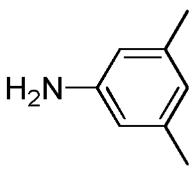 | 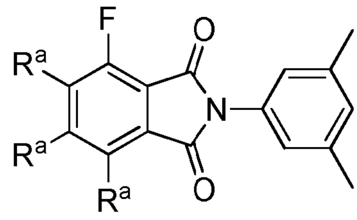 |
| 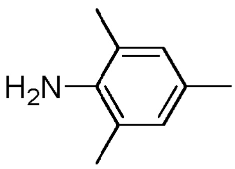 | 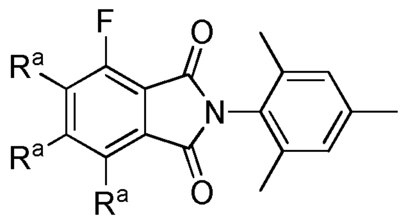 |

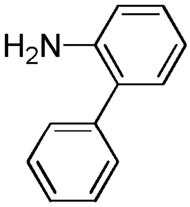

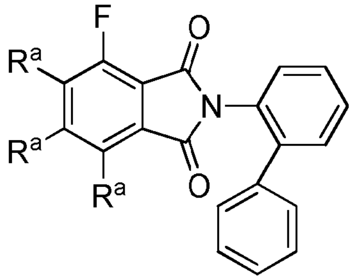

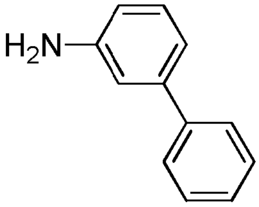

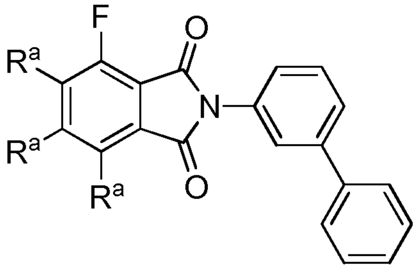

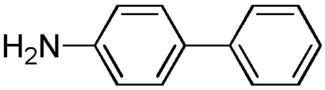

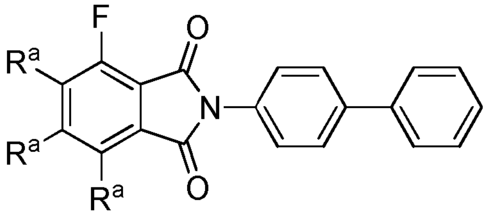

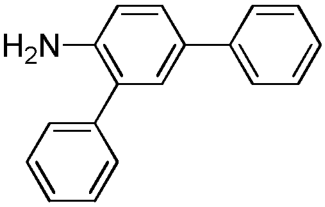

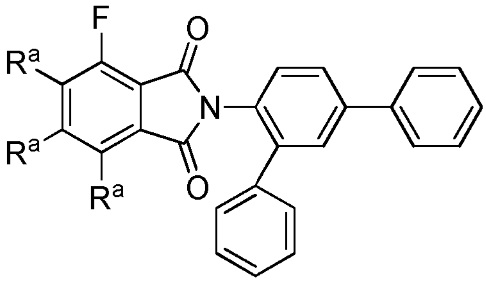

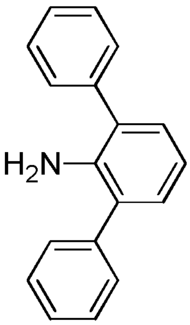

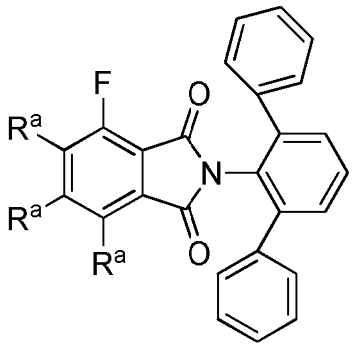

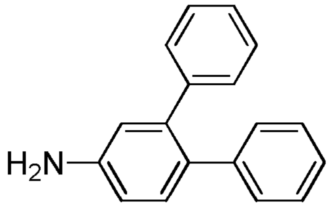

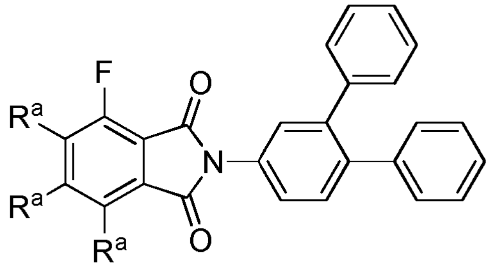

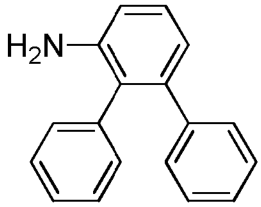

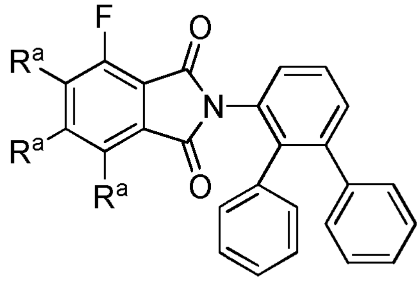

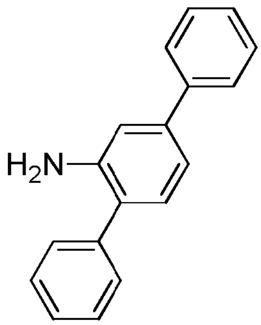

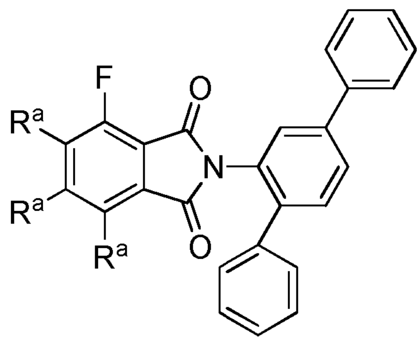

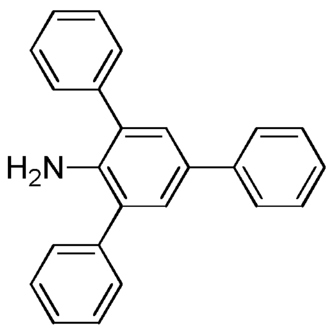

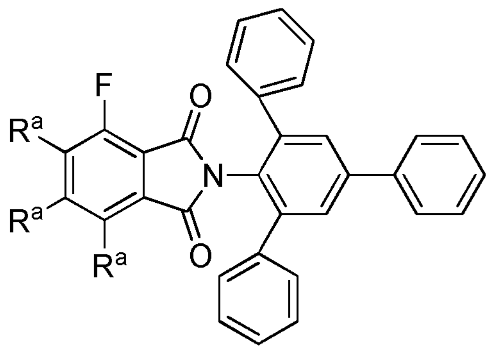

AAV1':

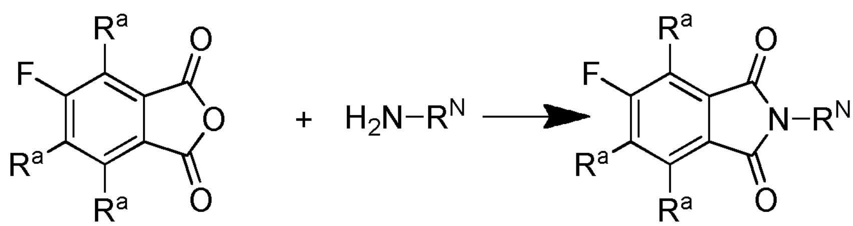

**E1'** **A1** **E2'**

In einem Rundkolben mit Rückflusskühler wird **E1'** (1 Äquivalent) in Eisessig suspendiert. Nach Zugabe von **A1** (1,1 Äquivalente) wird 3 Stunden bei 100 °C gerührt. Nach abkühlen wird die Reaktionslösung am Rotationsverdampfer soweit möglich eingeengt. Der Rückstand wird in $CH_2Cl_2$ aufgenommen und zweimal mit ges. $Na_2CO_3$ gewaschen. Die vereinigten organischen Phasen werden über $MgSO_4$ getrocknet. Das Lösungsmittel wird am Rotationsverdampfer entfernt. Nach Trocknen am Hochvakuum wird **E2'** als Produkt erhalten, welches in der Regel ohne weitere Aufreinigung eingesetzt werden kann. Bei Bedarf kann das Produkt **E2'** durch Umkristallisation weiter aufgereinigt werden.

**Beispielhafte A1 und E2' Kombinationen**

| A1 | E2 |
|---|---|
| 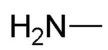 | 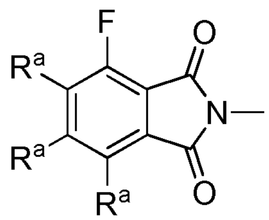 |
| 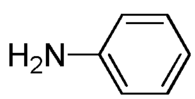 | 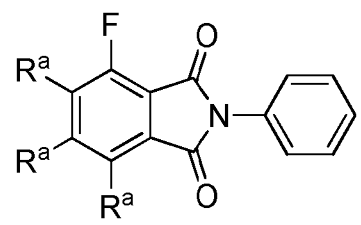 |
| 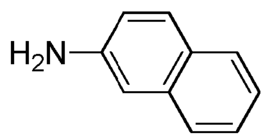 | 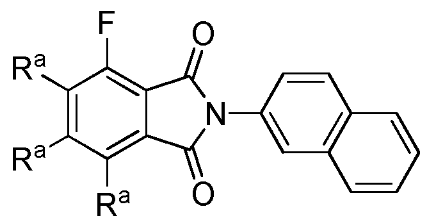 |
| 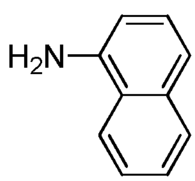 | 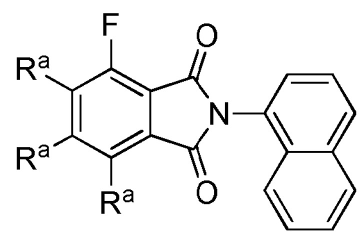 |
| 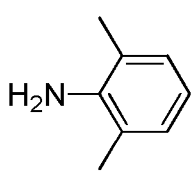 | 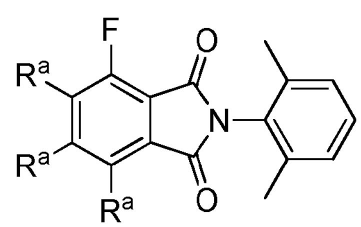 |

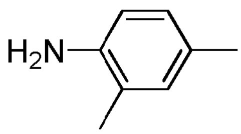

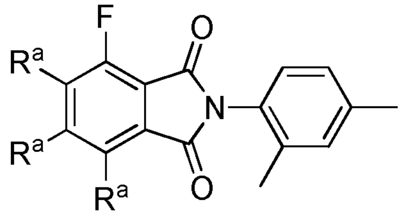

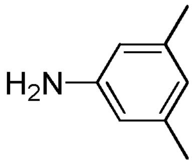

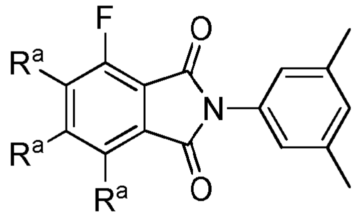

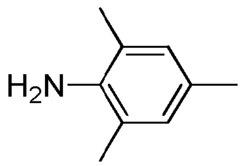

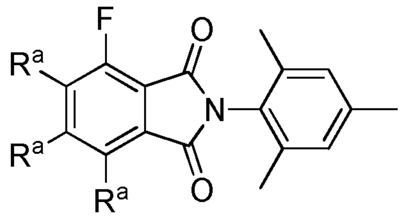

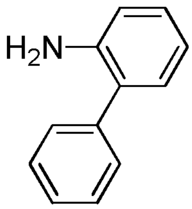

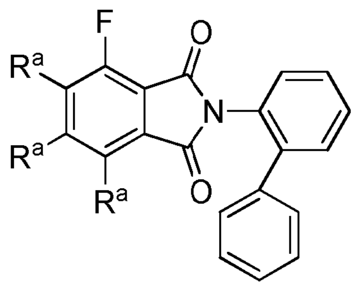

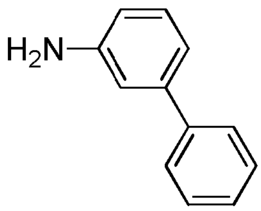

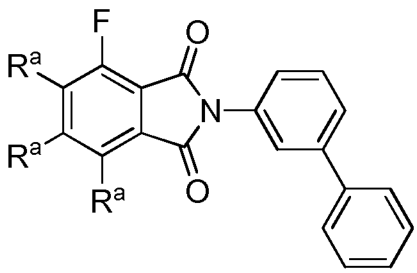

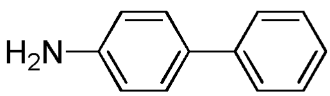

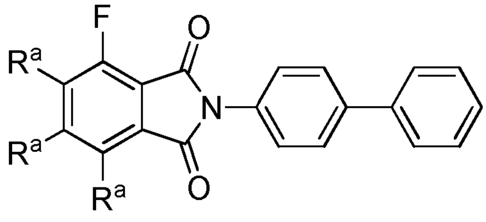

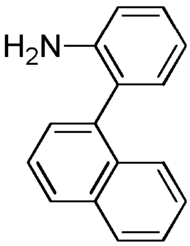

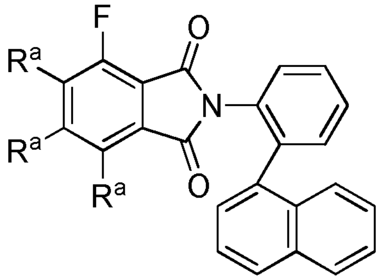

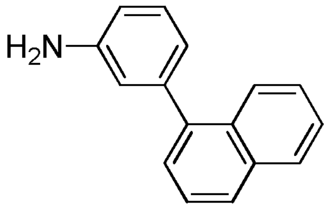

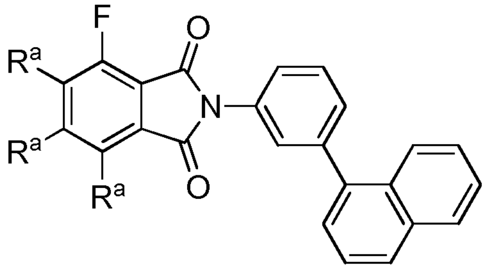

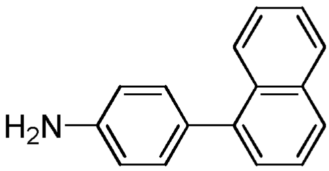

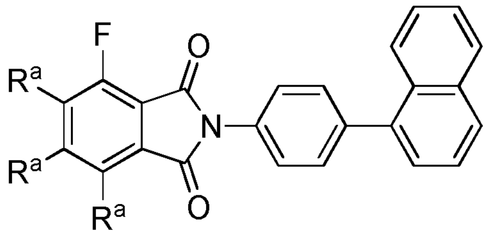

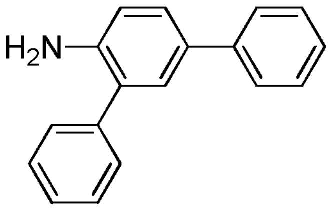

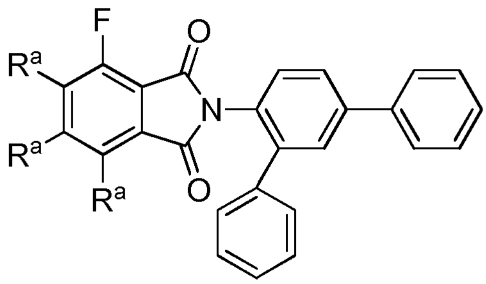

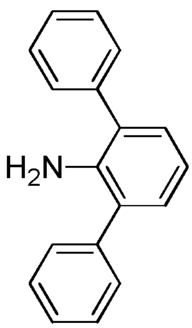

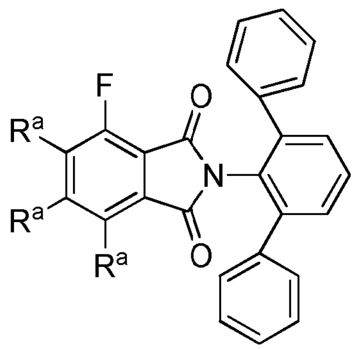

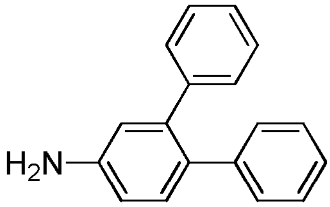

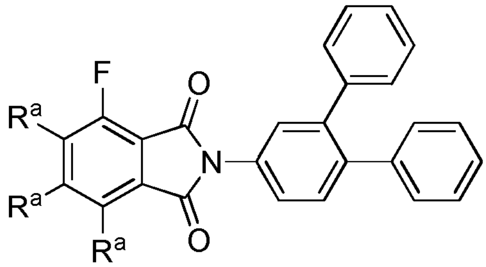

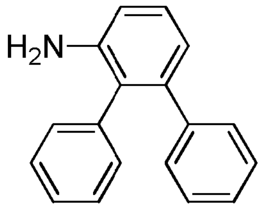

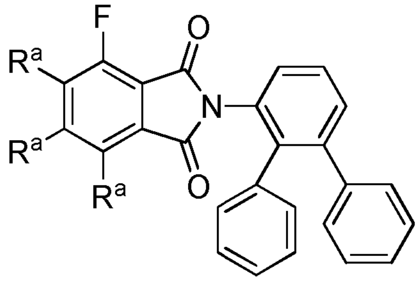

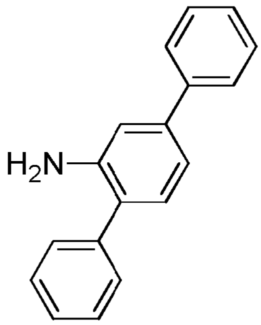

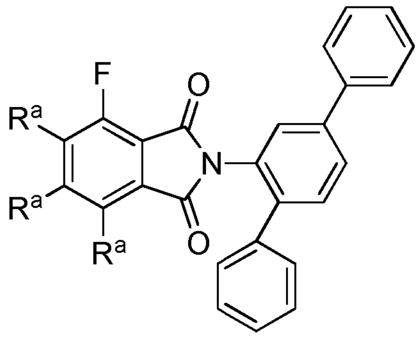

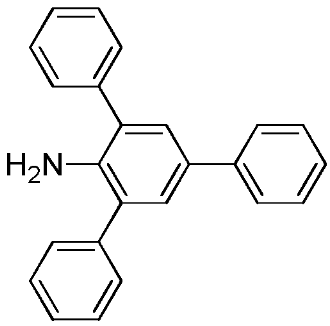

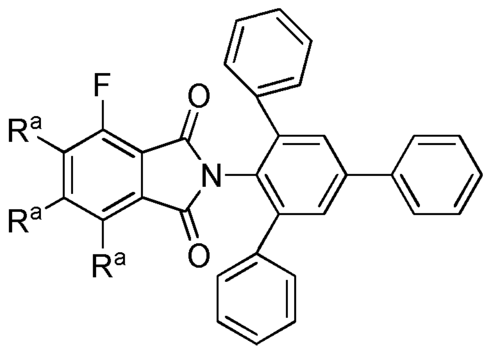

AAV2:

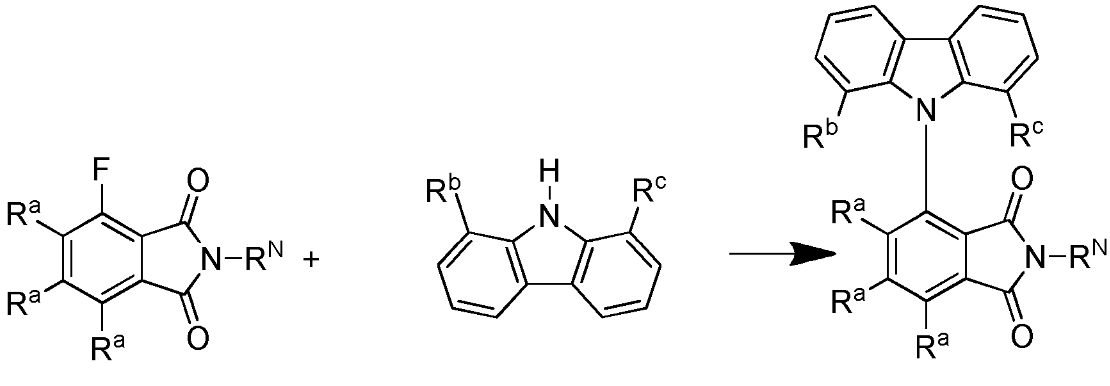

**E2** **E3**

In einem Rundkolben werden Phthalimid **E2** (1 Äquivalent), ein Carbazol-Derivat **E3** (1 Äquivalent) und $K_3PO_4$ (2 Äquivalente) vorgelegt und für 5 min evakuiert. Unter Inertgasatmosphäre wird DMSO (trocken) zugegeben und die Reaktionslösung für 16 h bei 100 °C gerührt. Nach abkühlen wird die Reaktionslösung auf Wasser geschüttet und mit $CH_2Cl_2$ extrahiert. Nach erneuter Extraktion mit $CH_2Cl_2$ werden die vereinigten organischen Phasen 2x mit Wasser und mit gesättigter NaCl-Lösung gewaschen. Anschließend wird über $MgSO_4$ getrocknet und das Lösemittel am Rotationsverdampfer entfernt. Das jeweilige Produkt kann durch Umkristallisation gereinigt werden.

AAV2':

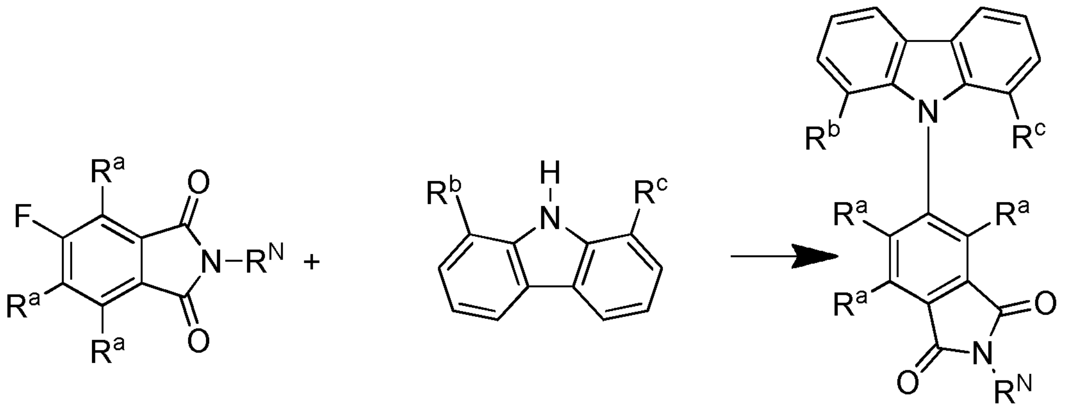

**E2'** **E3**

In einem Rundkolben werden Phthalimid **E2'** (1 Äquivalent), ein Carbazol-Derivat **E3** (1 Äquivalent) und $K_3PO_4$ (2 Äquivalente) vorgelegt und für 5 min evakuiert. Unter Inertgasatmosphäre wird DMSO (trocken) zugegeben und die Reaktionslösung für 16 h bei 100 °C gerührt. Nach abkühlen wird die Reaktionslösung auf Wasser geschüttet und mit $CH_2Cl_2$ extrahiert. Nach erneuter Extraktion mit $CH_2Cl_2$ werden die vereinigten organischen Phasen 2x mit Wasser und mit gesättigter NaCl-Lösung gewaschen. Anschließend wird über $MgSO_4$ getrocknet und das Lösemittel am Rotationsverdampfer entfernt. Das jeweilige Produkt kann durch Umkristallisation gereinigt werden.

**AAV3:**

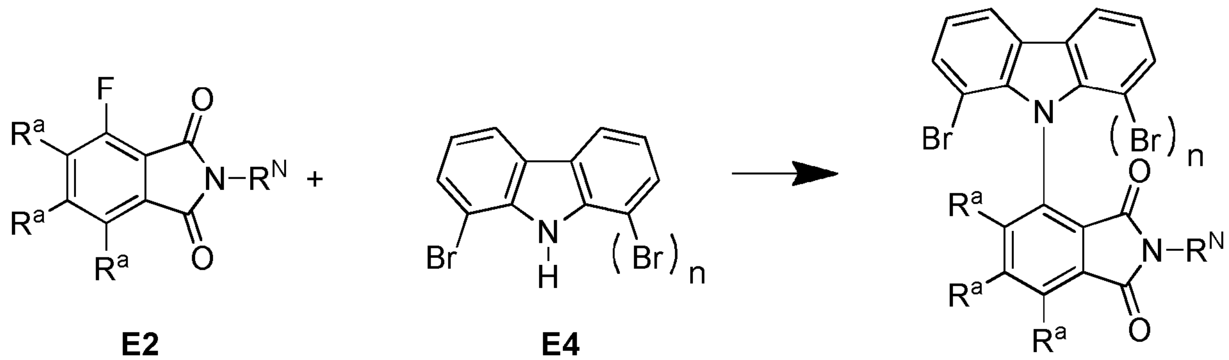

E2 E4

E5

n = 0 oder 1

In einem Rundkolben werden **E2** (1,2 Äquivalente), ein Brom-substituiertes Carbazol **E4** (1 Äquivalent) und $K_3PO_4$ (2 Äquivalente) vorgelegt und für 5 min evakuiert. Unter Inertgasatmosphäre wird DMSO (trocken) zugegeben und die Reaktionslösung für 16 h bei 100 °C gerührt. Nach abkühlen wird die Reaktionslösung auf Wasser geschüttet und mit $CH_2Cl_2$ extrahiert. Nach erneuter Extraktion mit $CH_2Cl_2$ werden die vereinigten organischen Phasen 2x mit Wasser und mit gesättigter NaCl-Lösung gewaschen. Anschließend wird über $MgSO_4$ getrocknet und das Lösemittel am Rotationsverdampfer entfernt. Das jeweilige Produkt kann durch Umkristallisation gereinigt werden.

Erfindungsgemäß kann statt Brom-substituiertem Carbazol auch Chlor- oder Iodsubstituiertes Carbazol verwendet werden.

**Beispielhafte E4 und E5 Kombinationen**

| E4 | E5 |
|---|---|
| 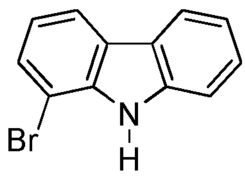 | 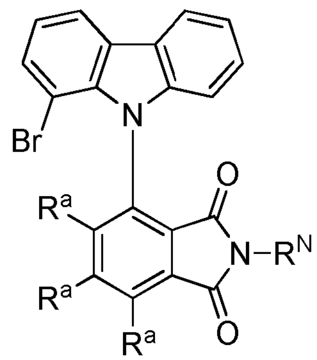 |
| 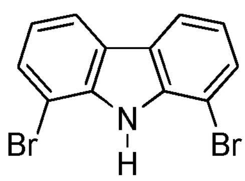 | 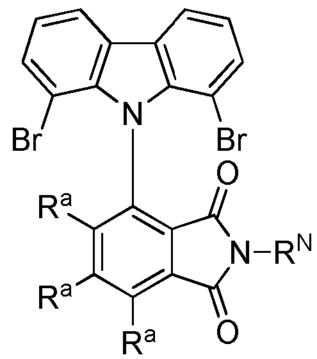 |

AAV3':

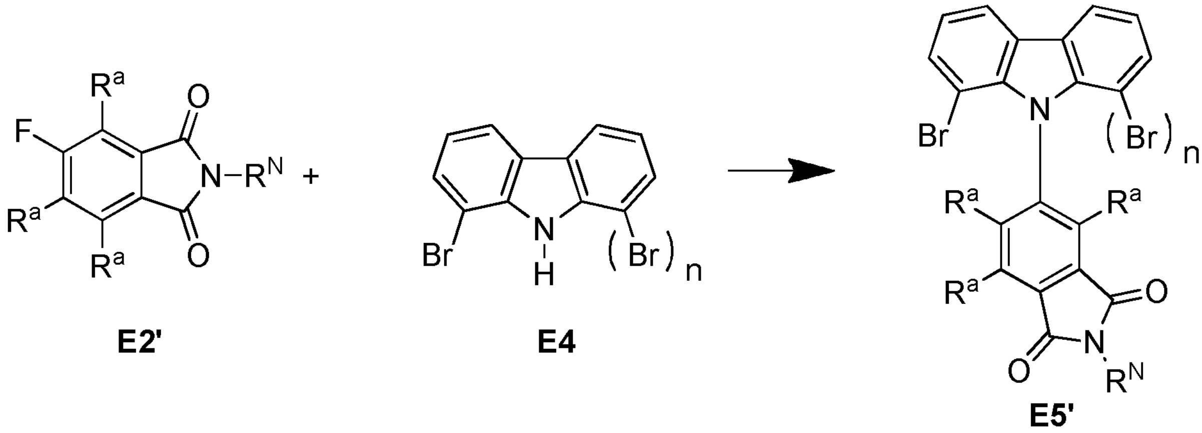

E2' E4 E5'

n = 0 oder 1

In einem Rundkolben werden **E2'** (1,2 Äquivalente), ein Brom-substituiertes Carbazol **E4** (1 Äquivalent) und $K_3PO_4$ (2 Äquivalente) vorgelegt und für 5 min evakuiert. Unter Inertgasatmosphäre wird DMSO (trocken) zugegeben und die Reaktionslösung für 16 h bei 100 °C gerührt. Nach abkühlen wird die Reaktionslösung auf Wasser geschüttet und mit $CH_2Cl_2$ extrahiert. Nach erneuter Extraktion mit $CH_2Cl_2$ werden die vereinigten organischen Phasen 2x mit Wasser und mit gesättigter NaCl-Lösung gewaschen. Anschließend wird über $MgSO_4$ getrocknet und das Lösemittel am Rotationsverdampfer entfernt. Das jeweilige Produkt kann durch Umkristallisation gereinigt werden.

Erfindungsgemäß kann statt Brom-substituiertem Carbazol auch Chlor- oder Iodsubstituiertes Carbazol verwendet werden.

**Beispielhafte E4 und E5' Kombinationen**

| E4 | E5' |
|---|---|
| 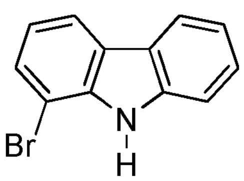 | 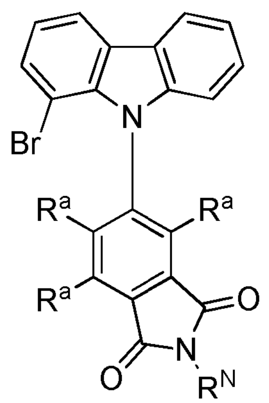 |

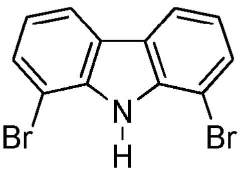

E5'

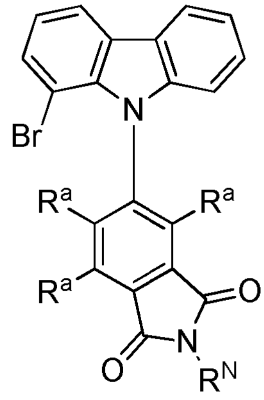

E5'

AAV4:

**Stufe 1**

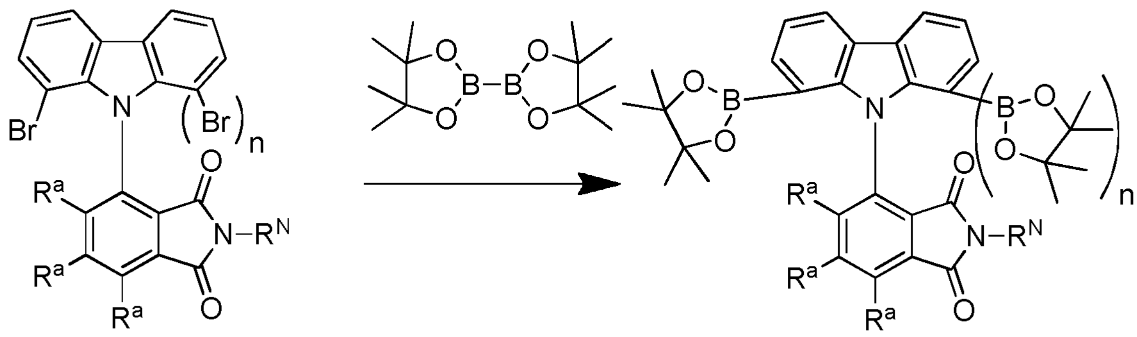

E5 E6

**E5** (1,00 Äquivalente), Bis(pinacolato)diboron (1,5 + 1,5n) Äquivalente), Tris(dibenzylideneaceton)dipalladium (0,01 Äquivalente), 2-(Dicyclohexylphosphino)-2',4',6'-triisopropylbiphenyl (0,04 Äquivalente) und tribasisches Kaliumphosphat (3 + 3n Äquivalente) werden unter Stickstoff in Dioxan für 12 - 24 h bei 110 °C gerührt. Das erhaltene Rohprodukt kann durch Umkristallisation gereinigt werden.

**Stufe 2:**

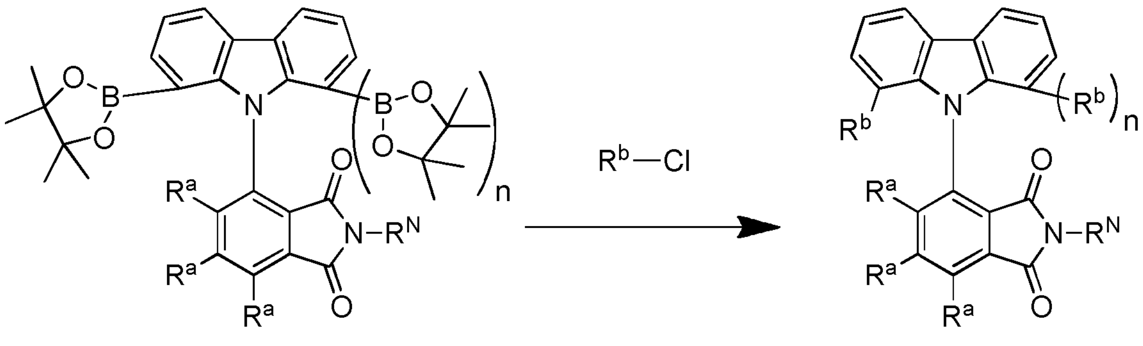


E6

**E6** (1,00 Äquivalente), $R^b$-Cl (1,3 + 1,3n Äquivalente), Tris(dibenzylideneaceton)dipalladium (0,01 Äquivalente), 2-(Dicyclohexylphosphino)-2',4',6'-triisopropylbiphenyl (0,04 Äquivalente) und tribasisches Kaliumphosphat (2,5 + 2,5n Äquivalente) werden unter Stickstoff in einem Toluol:/Wasser (10:1) Gemisch für 12 - 24 h bei 100 °C gerührt. Das erhaltene Rohprodukt wird durch Flash-Chromatographie oder durch Umkristallisation gereinigt.

Erfindungsgemäß kann statt $R^b$-Cl auch $R^b$-Br oder $R^b$-I verwendet werden.

AAV4':

**Stufe 1**

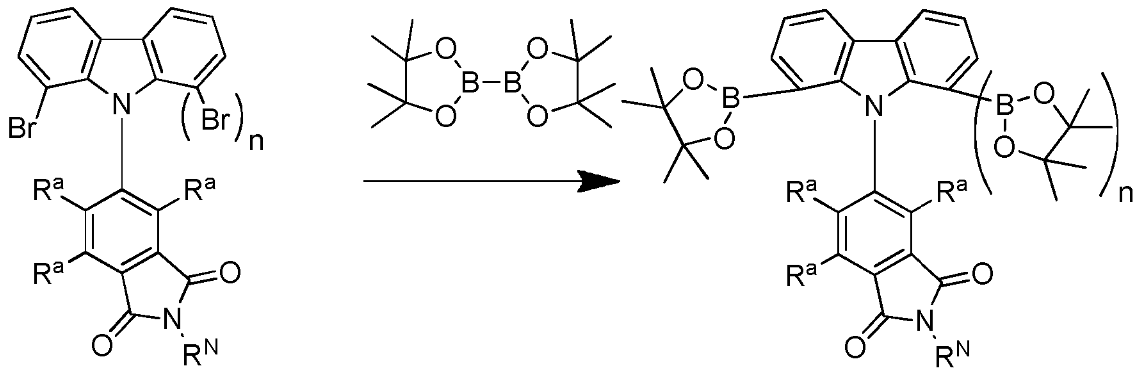

E5' E6'

**E5'** (1,00 Äquivalente), Bis(pinacolato)diboron (1,5 + 1,5n) Äquivalente), Tris(dibenzylideneaceton)dipalladium (0,01 Äquivalente), 2-(Dicyclohexylphosphino)-2',4',6'-triisopropylbiphenyl (0,04 Äquivalente) und tribasisches Kaliumphosphat (3 + 3n Äquivalente) werden unter Stickstoff in Dioxan für 12 - 24 h bei 110 °C gerührt. Das erhaltene Rohprodukt kann durch Umkristallisation gereinigt werden.

**Stufe 2:**

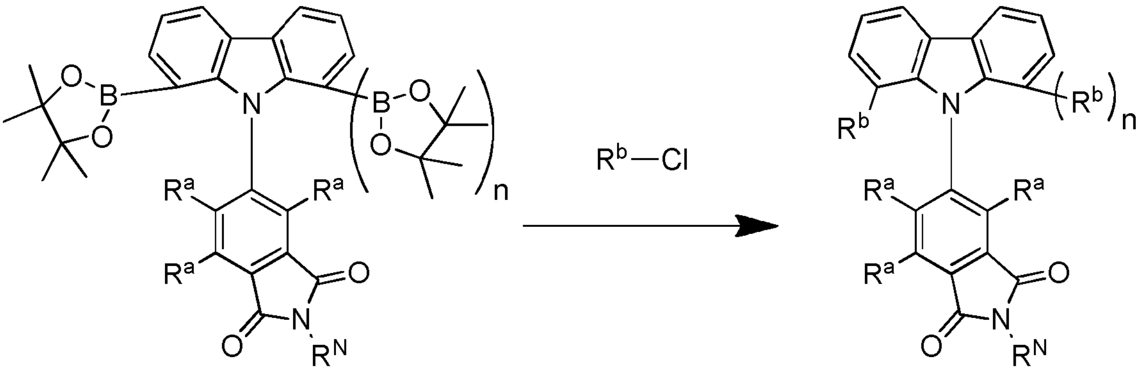


E6'

**E6'** (1,00 Äquivalente), $R^b$-Cl (1,3 + 1,3n Äquivalente), Tris(dibenzylideneaceton)dipalladium (0,01 Äquivalente), 2-(Dicyclohexylphosphino)-2',4',6'-triisopropylbiphenyl (0,04 Äquivalente) und tribasisches Kaliumphosphat (2,5 + 2,5n Äquivalente) werden unter Stickstoff in einem Toluol:/Wasser (10:1) Gemisch für 12 - 24 h bei 100 °C gerührt. Das erhaltene Rohprodukt wird durch Flash-Chromatographie oder durch Umkristallisation gereinigt.

Erfindungsgemäß kann statt $R^b$-Cl auch $R^b$-Br oder $R^b$-I verwendet werden.

AAV5:

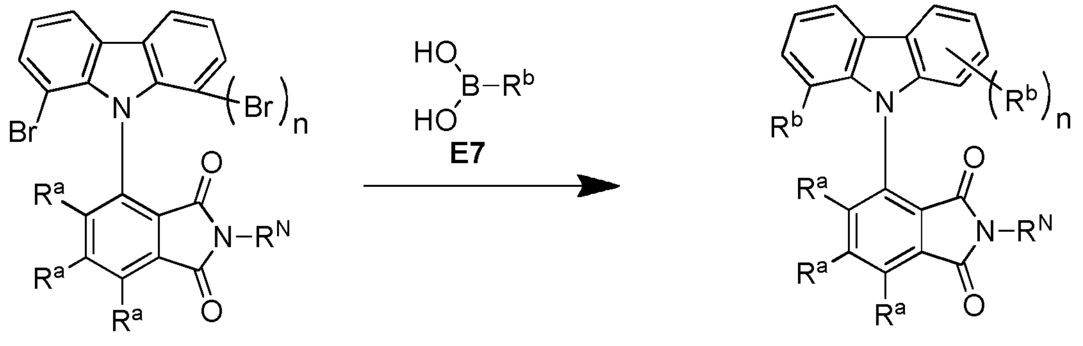

E7

E5

**E5** (1,00 Äquivalente), die entsprechende Boronsäure des Restes $R^b$ **E7** (1,3n + 1,3m Äquivalente), Tris(dibenzylideneaceton)dipalladium (0,01 Äquivalente), 2-(Dicyclohexylphosphino)-2',4',6'-triisopropylbiphenyl (0,04 Äquivalente) und tribasisches Kaliumphosphat (3n + 3m Äquivalente) werden unter Stickstoff in Dioxan/Wasser (10:1) für 12 —24 h bei 110 °C gerührt. Das erhaltene Rohprodukt wird durch Flash-Chromatographie oder durch Umkristallisation gereinigt.

Erfindungsgemäß kann statt einer Boronsäure auch ein entsprechender Boronsäureester verwendet werden.

**AAV5':**

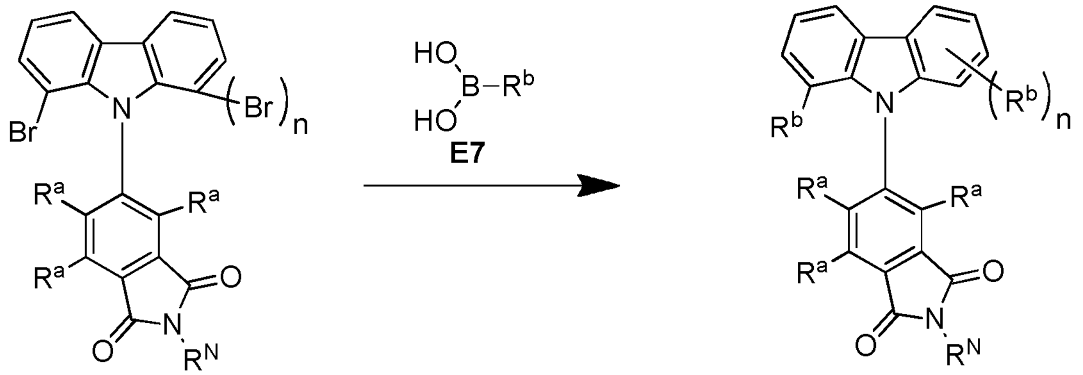

E7

E5

**E5'** (1,00 Äquivalente), die entsprechende Boronsäure des Restes $R^b$ **E7** (1,3n + 1,3m Äquivalente), Tris(dibenzylideneacetone)dipalladium (0,01 Äquivalente), 2-(Dicyclohexylphosphino)-2',4',6'-triisopropylbiphenyl (0,04 Äquivalente) und tribasisches Kaliumphosphat (3n + 3m Äquivalente) werden unter Stickstoff in Dioxan/Wasser (10:1) für 12 —24 h bei 110 °C gerührt. Das erhaltene Rohprodukt wird durch Flash-Chromatographie oder durch Umkristallisation gereinigt.

Erfindungsgemäß kann statt einer Boronsäure auch ein entsprechender Boronsäureester verwendet werden.

Die erfindungsgemäßen Moleküle können jeweils gemäß der AAV2 oder einer Kombination von AAV3 und AAV4 oder AAV3 und AAV5 bzw. gemäß der AAV2' oder einer Kombination von AAV3' und AAV4' oder AAV3' und AAV5' erhalten werden. Die Produkte der Synthesewege unterscheiden sich jeweils lediglich durch die erhaltenen Ausbeuten bzw. Reinheit vor der Aufreinigung. Nach entsprechender Aufreinigung sind die Produkte von äquivalenter Qualität.

**Berechnungen nach der Dichtefunktionaltheorie**

Für die Optimierung der Molekülstrukturen wurde das BP86-Funktional (Becke, A. D. Phys. Rev. A1988, 38, 3098-3100; Perdew, J. P. Phys. Rev. B1986, 33, 8822-8827) verwendet, wobei die resolution-of-identity-Näherung (RI) (Sierka, M.; Hogekamp, A.; Ahlrichs, R. J. Chem. Phys. 2003, 118, 9136-9148; Becke, A.D., J.Chem.Phys. 98 (1993) 5648-5652; Lee, C; Yang, W; Parr, R.G. Phys. Rev. B 37 (1988) 785-789) zum Einsatz kam. Anregungsenergien wurden bei der mit BP86 optimierten Struktur mit der Time-Dependent DFT-Methode (TD-DFT) unter Verwendung des B3LYP-Funktionals (Becke, A.D., J.Chem.Phys. 98 (1993) 5648-5652; Lee, C; Yang, W; Parr, R.G. Phys. Rev. B 37 (1988)

785-789; Vosko, S. H.; Wilk, L.; Nusair, M. Can. J. Phys. 58 (1980) 1200-1211; Stephens, P. J.; Devlin, F. J.; Chabalowski, C. F.; Frisch, M. J. J.Phys.Chem. 98 (1994) 11623-11627) berechnet. In allen Rechnungen wurden def2-SV(P)-Basissätze (Weigend, F.; Ahlrichs, R. Phys. Chem. Chem. Phys. 2005, 7, 3297-3305; Rappoport, D.; Furche, F. J. Chem. Phys.2010, 133, 134105/1-134105/11) und ein m4 -Grid zur numerischen Integration verwendet. Alle DFT-Rechnungen wurden mit dem Turbomole-Programmpaket (Version 6.5) (TURBOMOLE V6.4 2012, eine Entwicklung der Universität Karlsruhe und Forschungszentrum Karlsruhe GmbH, 1989-2007, TURBOMOLE GmbH, seit 2007; http://www. turbomole.com) durchgeführt.

Die in den Figuren dargestellten Grenzorbitale wurden mit dem B3LYP-Funktional für die optimierten Grundzustandsgeometrien berechnet.

**Photophysikalische Messungen**

*Vorbehandlung von optischen Gläsern*

Alle Gläser (Küvetten und Substrate aus Quarzglas, Durchmesser: 1 cm) wurden nach jeder Benutzung gereinigt: Je dreimaliges Spülen mit Dichlormethan, Aceton, Ethanol, demineralisiertem Wasser, Einlegen in 5 % Hellmanex-Lösung für 24 h, gründliches Ausspülen mit demineralisiertem Wasser. Zum Trocknen wurden die optischen Gläser mit Stickstoff abgeblasen.

***Probenvorbereitung: Lösungen***

1-2 mg der Probe wurden in 100 ml des jeweiligen Lösemittels gelöst, Konzentration $10^{-5}$ mol/L. Die Küvette wurde luftdicht verschlossen und 10 min. entgast.

***Probenvorbereitung, Film: Spin-Coating*** (Gerät: Spin150, SPS euro.)

Die Probenkonzentration entsprach 10mg/ml, angesetzt in Toluol oder Chlorbenzol. Programm: 1) 3 s bei 400 U/min; 2) 20 Sek. bei 1000 U/min bei 1000 Upm/ s. 3) 10 s bei 4000 U/min bei 1000 Upm/ s. Die Filme wurden nach dem Beschichten für 1 min bei 70 °C an Luft auf einer Präzisionsheizplatte von LHG getrocknet.

***Absorptionsspektroskopie***

Lösungen: UV-VIS-Spektren wurden auf einem Gerät der Firma Thermo Scientific, Modell Evolution 201 aufgenommen. (Siehe Probenvorbereitung: Lösungen)
Film: UV-VIS-Spektren wurden auf einem Gerät der Firma Thermo Scientific, Modell Evolution 201 aufgenommen. (Siehe Probenvorbereitung, Film: Spin-Coating)

***Photolumineszenzspektroskopie und TCSPC***

Steady-state Emissionsspektroskopie wurde mit einem Fluoreszenzspektrometer der Firma Horiba Scientific, Modell FluoroMax-4 durchgeführt, ausgestattet mit einer 150 W Xenon-Arc Lampe, Anregungs- und Emissionsmonochromatoren und einer Hamamatsu R928 Photomultiplier-Röhre, sowie einer TCSPC-Option. Emissions- und Anregungsspektren wurden korrigiert durch Standardkorrekturkurven.

Die Emissionsabklingzeiten wurden ebenfalls auf diesem System gemessen unter Verwendung der TCSPC-Methode mit dem FM-2013 Zubehör und einem TCSPC-Hub von Horiba Yvon Jobin. Anregungsquellen: NanoLED 370 (Wellenlänge: 371 nm, Pulsdauer: 1.1 ns), NanoLED 290 (Wellenlänge: 294 nm, Pulsdauer: <1 ns), SpectraLED 310 (Wellenlänge: 314nm), SpectraLED 355 (Wellenlänge: 355nm).

Die Auswertung (exponentielles Fitten) erfolgte mit dem Softwarepaket DataStation und der DAS 6 Auswertungssoftware. Der Fit wurde über die Chi-Quadrat-Methode angegeben

$$c^2 = \sum_{k=1}^{i} \frac{(e_i - o_i)^2}{e_i}$$

mit ei: Durch den Fit vorhergesagte Größe und $o_i$: Gemessenen Größe.

*Quanteneffizienzbestimmung*

Die Messung der Photolumineszenzquantenausbeute (PLQY) erfolgte mittels eines *Absolute PL Quantum Yield Measurement C9920-03G*-Systems der Firma *Hamamatsu Photonics.*

Dieses besteht aus einer 150 W Xenon-Gasentladungslampe, automatisch justierbaren Czerny-Turner Monochromatoren (250 - 950 nm) und einer Ulbricht-Kugel mit hochreflektierender Spektralon-Beschichtung (einem Teflon-Derivat), die über ein Glasfaserkabel mit einem PMA-12 Vielkanaldetektor mit BT- *(back thinned-)* CCD-Chip mit 1024 x 122 Pixeln (Größe 24 x 24 $\mu$m) verbunden ist. Die Auswertung der Quanteneffizienz und der CIE-Koordinaten erfolgte mit Hilfe der Software U6039-05 Version 3.6.0

Für G9920-OXG (PMA-12). Das Emissionsmaximum wird in nm, die Quantenausbeute $\Phi$ in % und die CIE-Farbkoordinaten als x,y-Werte angegeben.

Die PLQY wurde für Polymerfilme, Lösungen und Pulverproben nach folgendem Protokoll bestimmt:

1) Durchführung der Qualitätssicherung: Als Referenzmaterial dient Anthracen in Ethanol mit bekannter Konzentration.
2) Ermitteln der Anregungswellenlänge: Es wurde zuerst das Absorptionsmaximum des organischen Moleküls bestimmt und mit diesem angeregt.
3) Durchführung der Probenmessung: Es wurde von entgasten Lösungen und Filmen unter Stickstoff-Atmosphäre die absolute Quantenausbeute bestimmt. Die Berechnung erfolgte systemintern nach folgender Gleichung:

$$\Phi_{PL} = \frac{n_{photon}, emittiert}{n_{photon}, absorbiert} = \frac{\int \frac{\lambda}{hc}\left[Int_{emittiert}^{Probe}(\lambda) - Int_{absorbiert}^{Probe}(\lambda)\right]d\lambda}{\int \frac{\lambda}{hc}\left[Int_{emittiert}^{Referenz}(\lambda) - Int_{absorbiert}^{Referenz}(\lambda)\right]d\lambda}$$

mit der Photonenzahl $n_{photon}$ und der Intensität Int.

**Herstellung und Charakterisierung von organischen Elektrolumineszenzvorrichtungen aus der Gasphase**

Mit den erfindungsgemäßen organischen Molekülen können OLED-Devices mittels Vakuum-Sublimationstechnik erstellt werden. Enthält eine Schicht mehrere Komponenten, so ist das Verhältnis dieser in Massenprozent angegeben.

Diese noch nicht optimierten OLEDs können standardmäßig charakterisiert werden; hierfür werden die Elektrolumineszenzspektren, die externe Quanteneffizienz (gemessen in %) in Abhängigkeit von der Helligkeit, berechnet aus dem von der Fotodiode detektiertem Licht, und dem Strom aufgenommen. Aus dem zeitlichen Verlauf der Elektrolumineszenzspektren kann die Lebensdauer der OLEDs bestimmt werden. Der angegebene LT50-Wert entspricht hierbei der Zeit, bei der die Leuchtdichte auf 50 % des Startwertes abgefallen ist. Analog entspricht der LT70-Wert der Zeit, bei der die Leuchtdichte auf 70 % des Startwertes abgefallen ist.

Beispiel 1

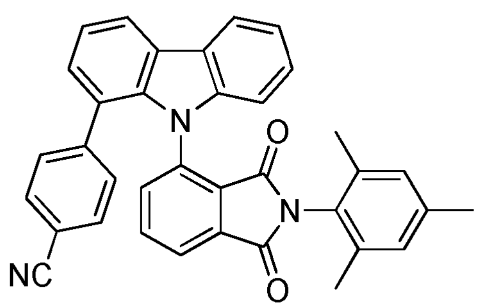

Beispiel 1 wurde nach AAV 5 aus 3-(1-Bromcarbazolyl)-*N*-mesitylphthalimid und 4-Cyanophenylboronsäure in einer Ausbeute von 22 % hergestellt.

$^1$H NMR (500 MHz, Chloroform-d) $\delta$ 8.22 (dd, J = 7.8, 1.2 Hz, 1H), 8.19-8.16 (m, 1H), 7.80 (dd, J = 7.4, 0.9 Hz,

1H), 7.56 (t, J = 7.7 Hz, 1H), 7.44 - 7.37 (m, 3H), 7.37 - 7.24 (m, 7H), 7.05 (dt, J = 8.2, 0.9 Hz, 1H), 6.96 (s, 1H), 6.90 (s, 1H), 2.27 (s, 3H), 2.15 (s, 3H), 1.99 (s, 3H).

Figur 1 zeigt das Emissionsspektrum von Beispiel 1 (10% in PMMA). Das Emissionsmaximum liegt bei 482 nm. Die Photolumineszenzquantenausbeute (PLQY) beträgt 61 % und die Halbwertsbreite (FWHM) 94 nm (0,48 eV). Es ergibt sich eine $CIE_y$ von 0,33 und ein BMI von 185.

Beispiel 2

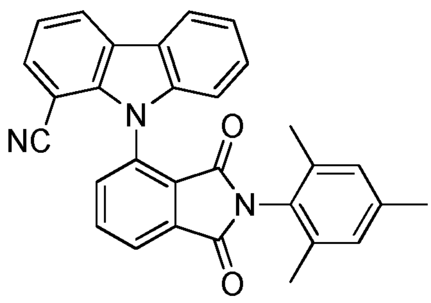

Beispiel 2 wurde gemäß der folgenden Vorschrift hergestellt:
In einem Zweihalskolben mit Rückflusskühler wurden unter Stickstoffatmosphäre 3-(1-Bromcarbazolyl)-*N*-mesitylphthalimid (1 Äquivalent) und CuCN (1,5 Äquivalente) in trockenem DMF während 15 h auf 250 °C erhitzt. Nach dem Abkühlen auf RT wurde aus der Reaktionslösung durch Zugabe des gleichen Volumens an Wasser das Rohprodukt ausgefällt. Der Niederschlag wurde abfiltriert und mit $CH_2Cl_2$ behandelt. Die resultierende Lösung wurde mit Wasser gewaschen, über $MgSO_4$ getrocknet und unter vermindertem Druck vom Lösungsmittel befreit. Der Rückstand wurde mit heißem Ethanol gewaschen. Die Waschlösung wurde verworfen und der Rückstand aus Toluol umkristallisiert. Ausbeute: 69 %. Figur 2 zeigt das Emissionsspektrum von Beispiel 2 (10% in PMMA). Das Emissionsmaximum liegt bei 453 nm. Die Photolumineszenzquantenausbeute (PLQY) beträgt 44 % und die Halbwertsbreite (FWHM) 84 nm (0,49 eV). Es ergibt sich eine $CIE_y$ von 0,16 und ein BMI von 275.

Beispiel 3

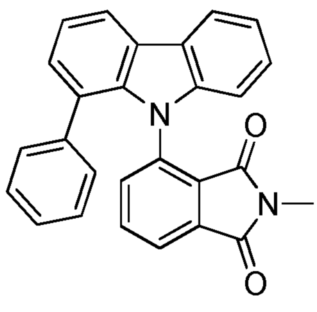

Figur 3 zeigt das Emissionsspektrum von Beispiel 3 (10 % in PMMA). Das Emissionsmaximum liegt bei 481 nm. Die Photolumineszenzquantenausbeute (PLQY) beträgt 56 % und die Halbwertsbreite (FWHM) 96 nm (0,49 eV). Es ergibt sich eine $CIE_y$ von 0,32 und ein BMI von 175. Die Emissionslebensdauer beträgt 5,5 μs.

**Beispiel 4**

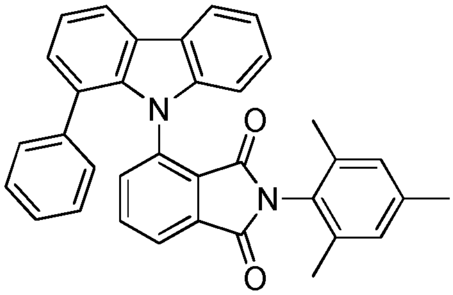

Figur 4 zeigt das Emissionsspektrum von Beispiel **4** (10 % in PMMA). Das Emissionsmaximum liegt bei 485 nm. Die Photolumineszenzquantenausbeute (PLQY) beträgt 60 % und die Halbwertsbreite (FWHM) 96 nm (0,48 eV). Es ergibt sich eine $CIE_y$ von 0,36 und ein BMI von 167. Die Emissionslebensdauer beträgt 4,9 µs.

**Beispiel 5**

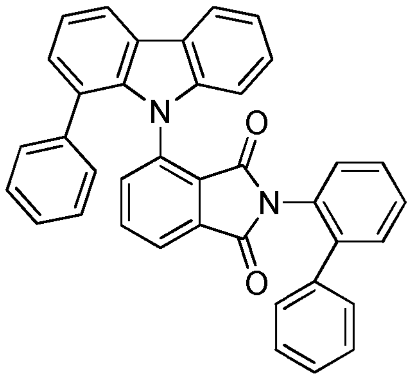

Figur 5 zeigt das Emissionsspektrum von Beispiel **5** (10 % in PMMA). Das Emissionsmaximum liegt bei 487 nm. Die Photolumineszenzquantenausbeute (PLQY) beträgt 61 % und die Halbwertsbreite (FWHM) 97 nm (0,49 eV). Es ergibt sich eine $CIE_y$ von 0,36 und ein BMI von 169. Die Emissionslebensdauer beträgt 3,8 µs

**Beispiel 6**

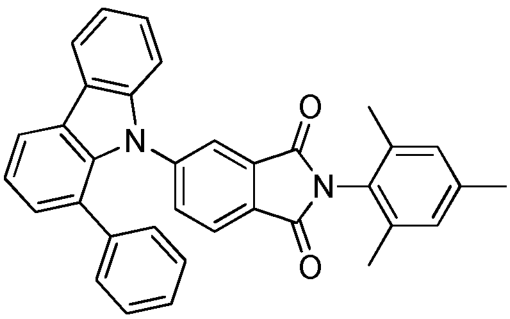

Figur 6 zeigt das Emissionsspektrum von Beispiel **6** (10 % in PMMA). Das Emissionsmaximum liegt bei 474 nm. Die Photolumineszenzquantenausbeute (PLQY) beträgt 67 % und die Halbwertsbreite (FWHM) 88 nm (0,47 eV). Es ergibt sich eine $CIE_y$ von 0,26 und ein BMI von 258.

**Beispiel 7**

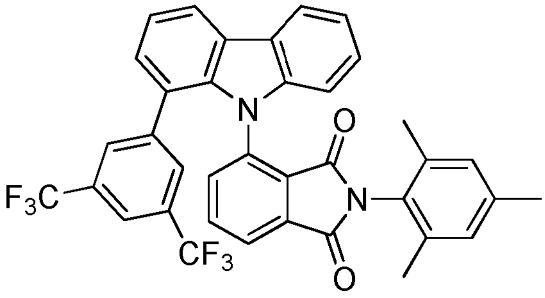

Beispiel 7 wurde nach AAV 5 aus 3-(1-Bromcarbazolyl)-*N*-mesitylphthalimid und 3,5-Bis(trifluormethyl)benzolboronsäure in einer Ausbeute von 49 % hergestellt.

$^1H$ NMR (500 MHz, Chloroform-d) δ 8.26 (dd, J = 7.8, 1.2 Hz, 1H), 8.20 - 8.16 (m, 1H), 7.76 (dd, J = 7.4, 0.9 Hz, 1H), 7.79 - 7.47 (breites Signal, 2H), 7.61 (s, 1H), 7.45 (t, J = 7.6 Hz, 1H), 7.41 (t, J =7.41 Hz, 1H), 7.39 - 7.32 (m, 3H), 7.25 (dd, J = 8.0, 0.9 Hz, 1H), 7.01 - 6.97 (m, 1H), 6.95 - 6.92 (m, 1H), 6.91-6.89 (m, 1H), 2.27 (s, 3H), 2.10 (s, 3H), 2.03 (s, 3H).

$^{19}F$ NMR (471 MHz, CDCl3) δ -62.5.

Figur 7 zeigt das Emissionsspektrum von Beispiel **7** (10 % in PMMA). Das Emissionsmaximum liegt bei 477 nm. Die Photolumineszenzquantenausbeute (PLQY) beträgt 65 % und die Halbwertsbreite (FWHM) 93 nm (0,48 eV). Es ergibt sich eine $CIE_y$ von 0,31 und ein BMI von 210.

**Weitere Beispielmoleküle**:

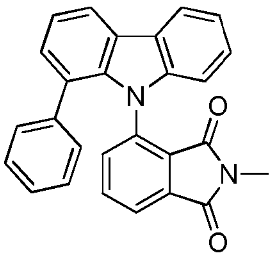

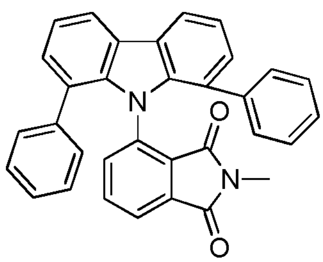

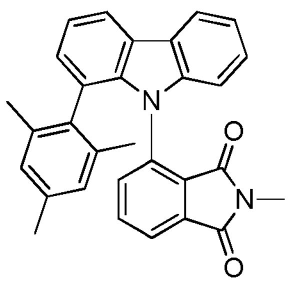

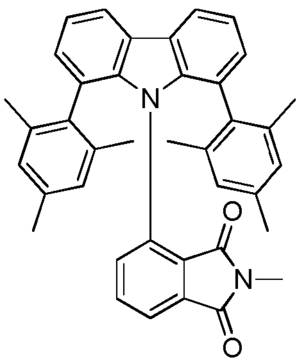

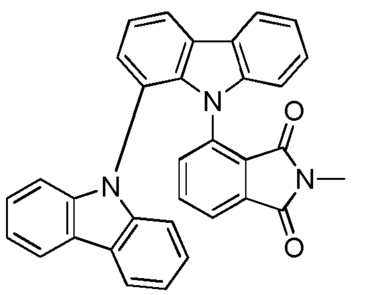

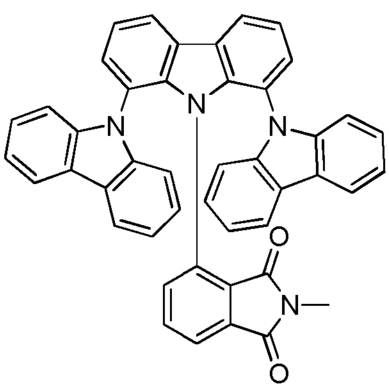

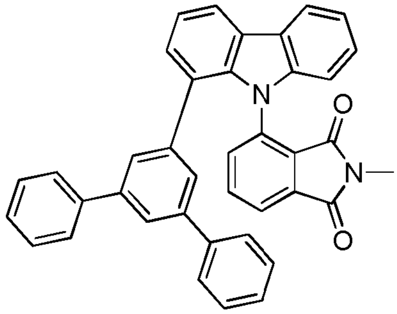

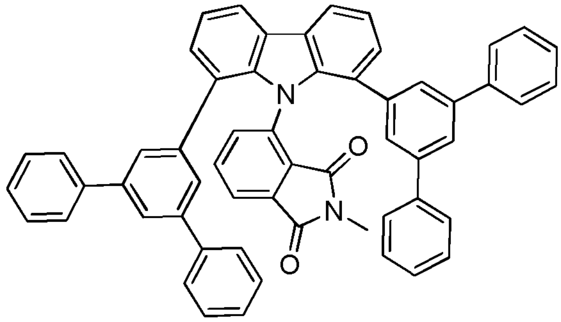

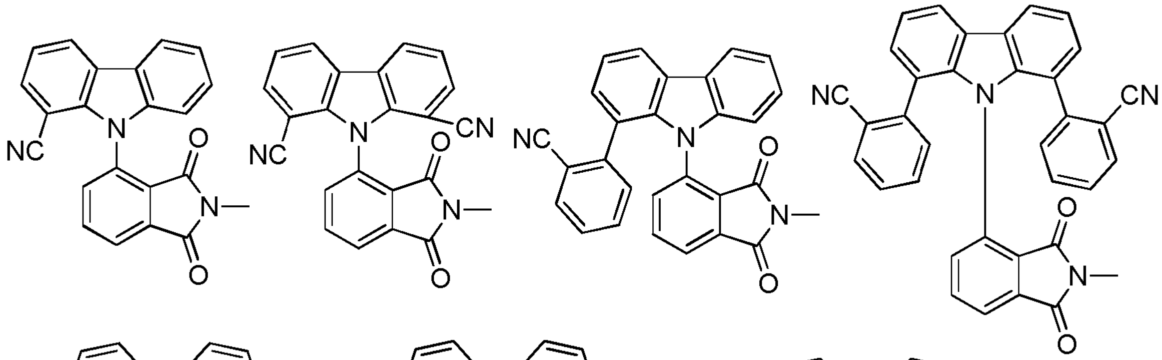

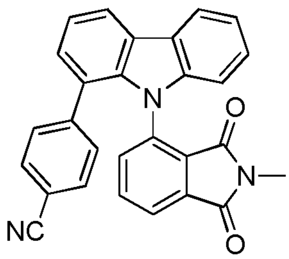

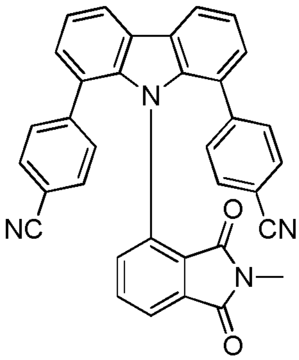

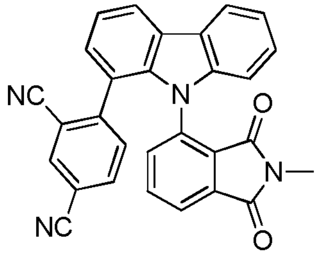

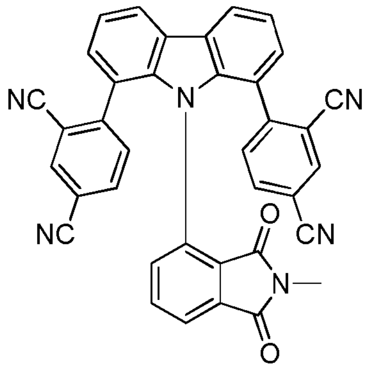

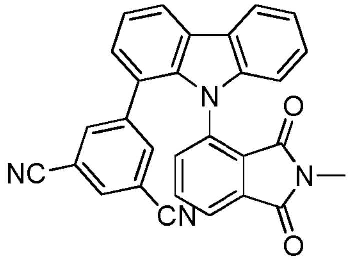

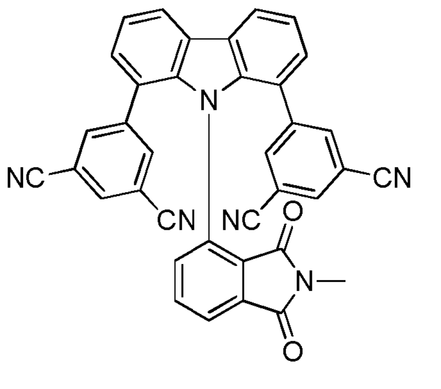

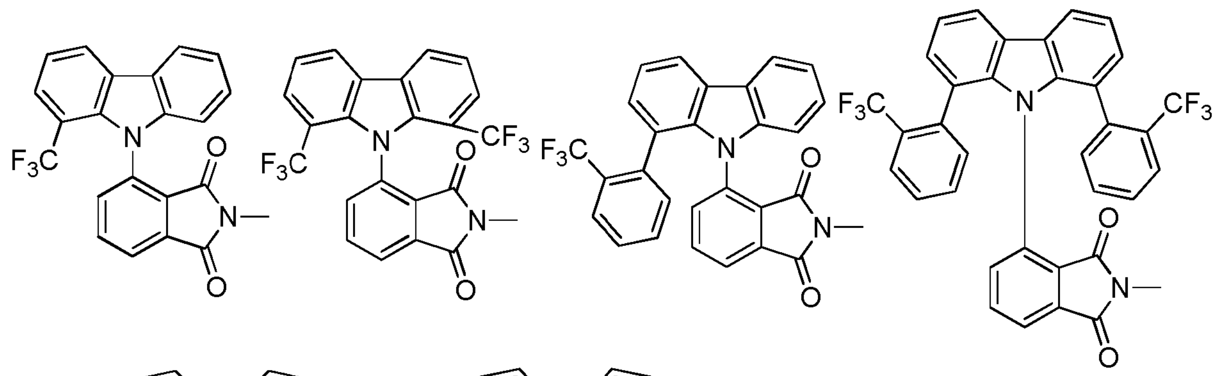

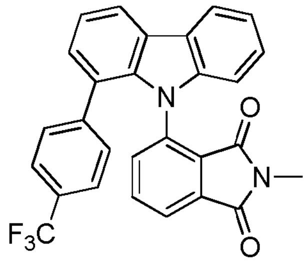

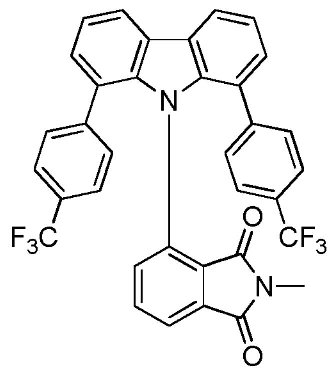

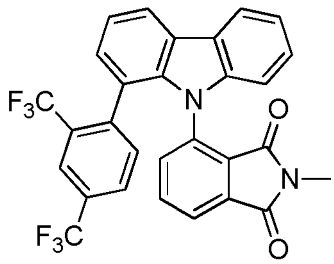

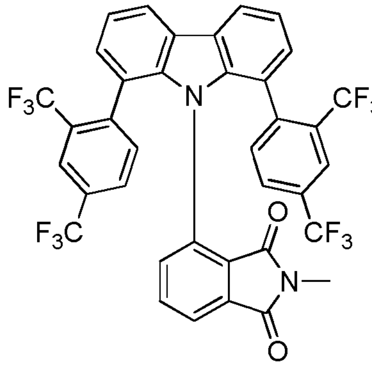

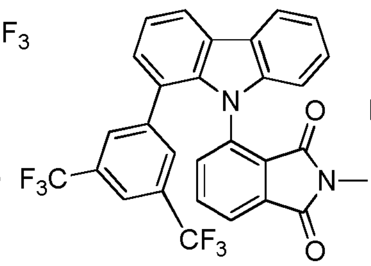

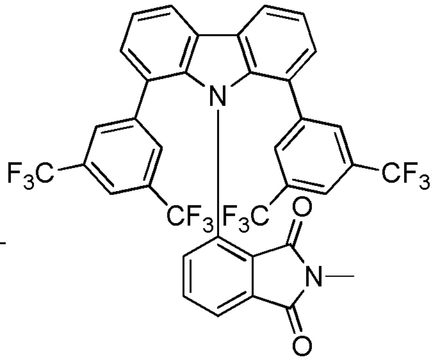

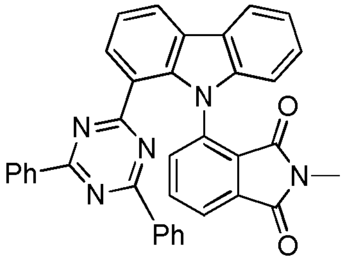

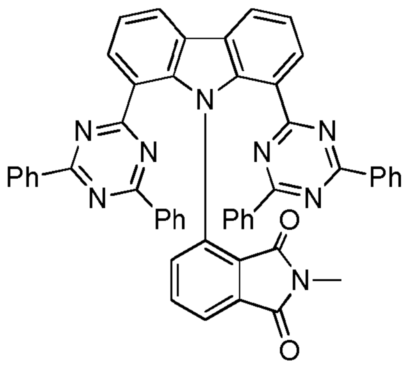

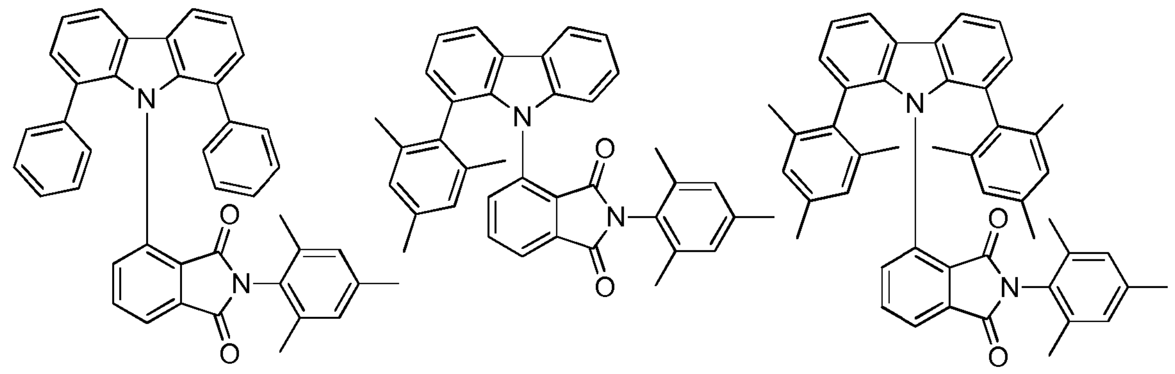

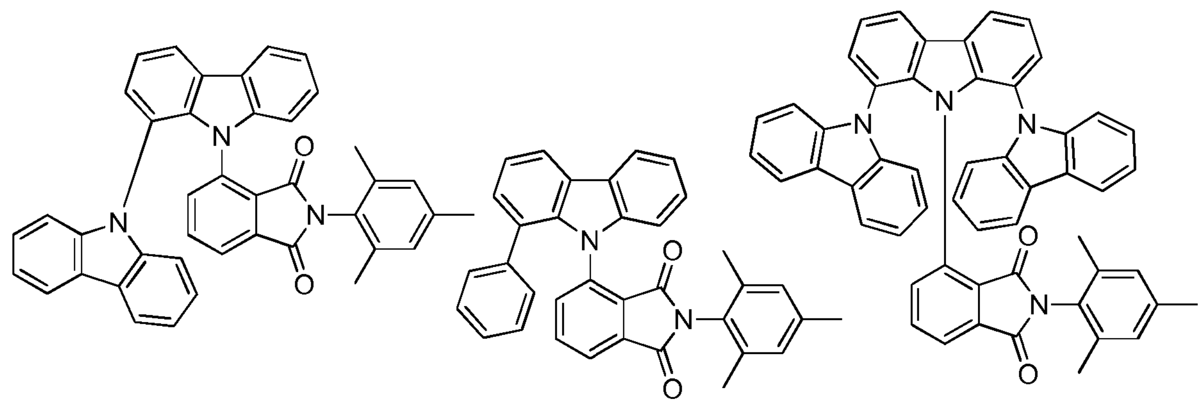

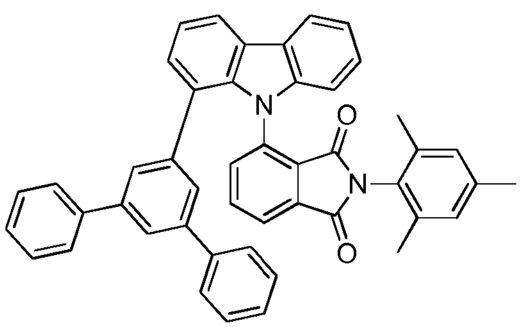

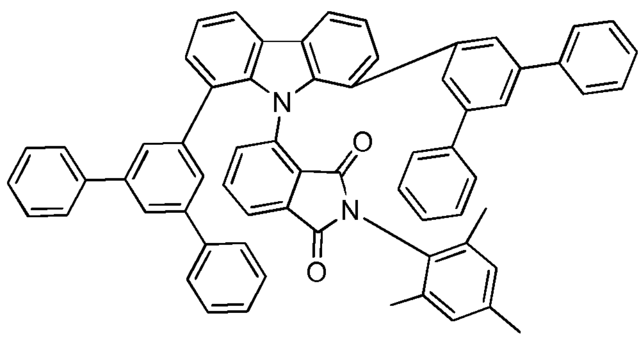

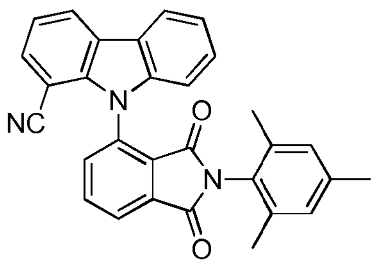

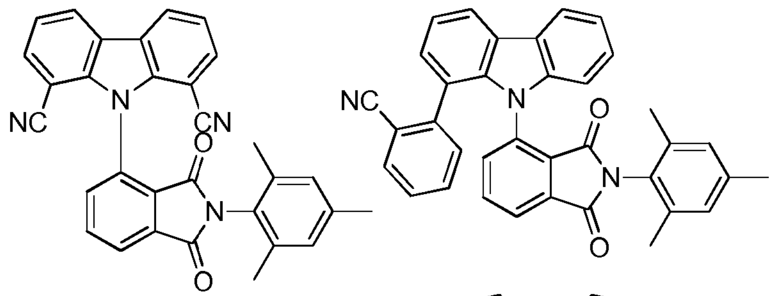

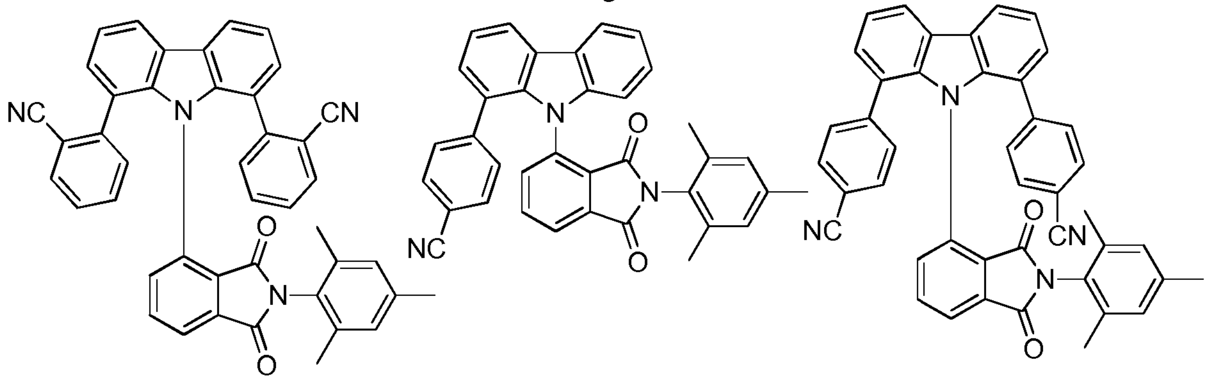

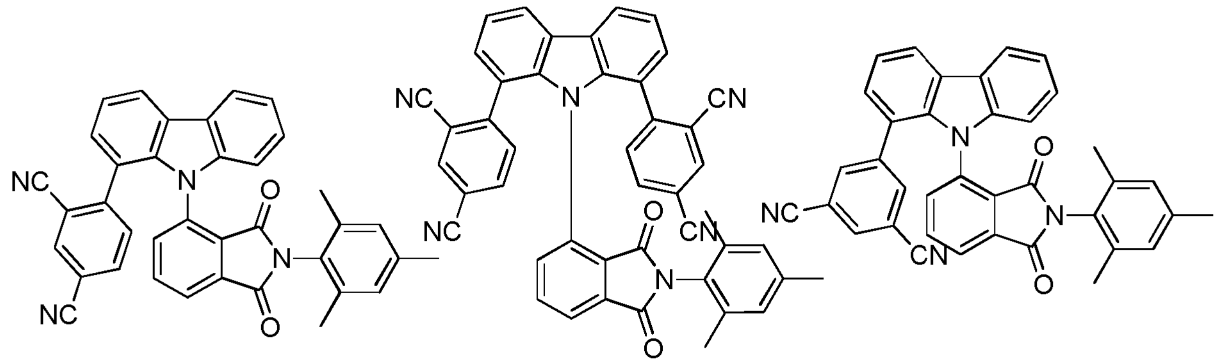

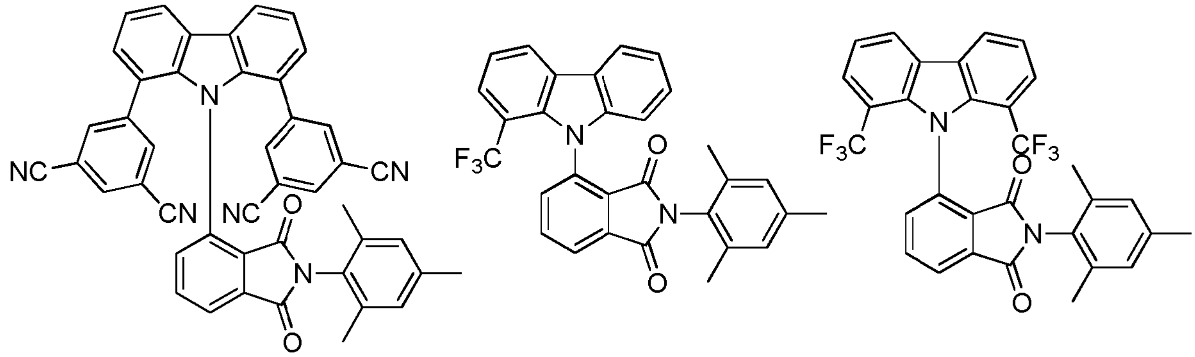

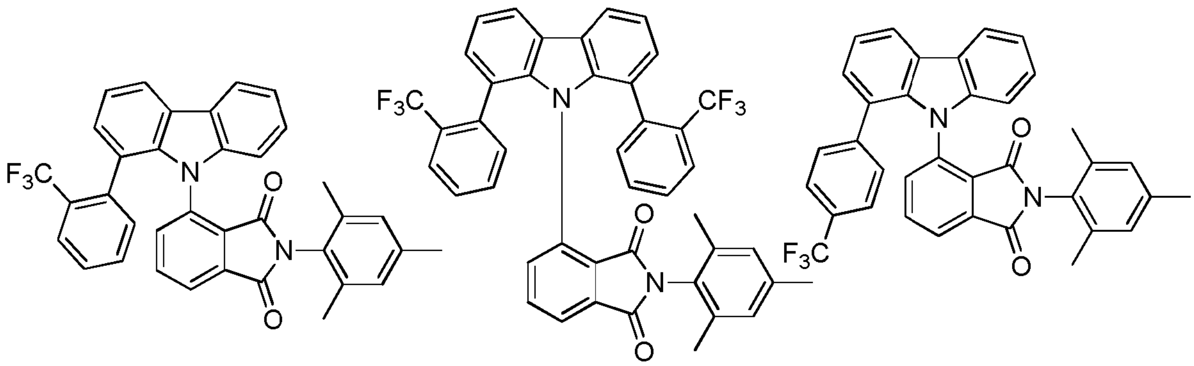

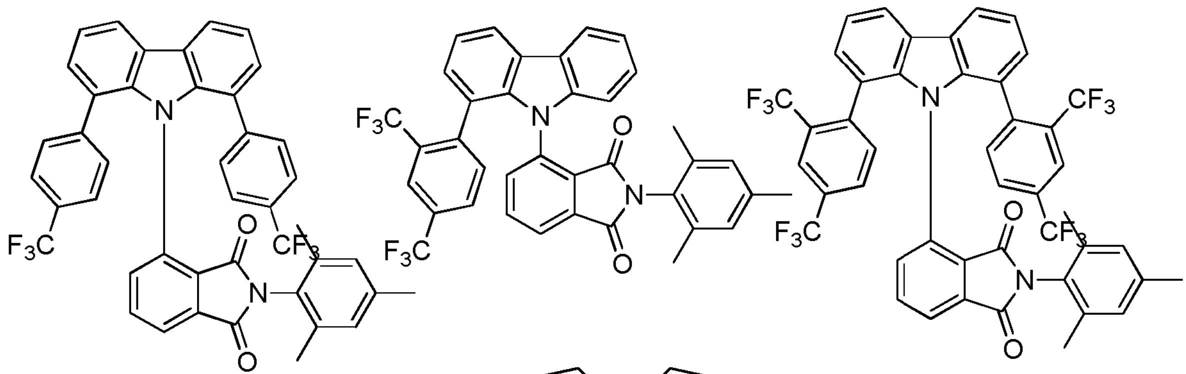

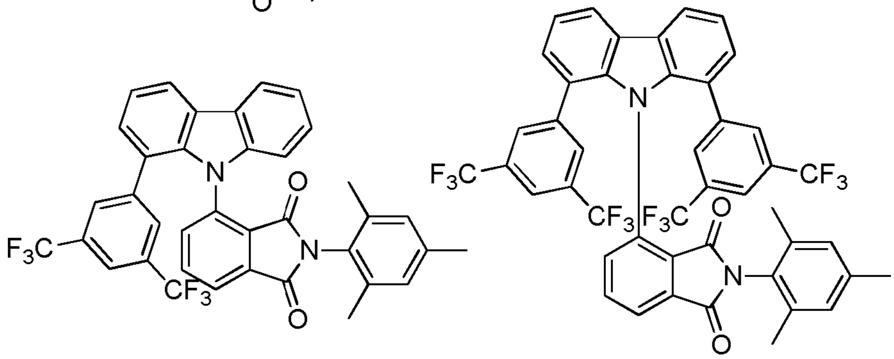

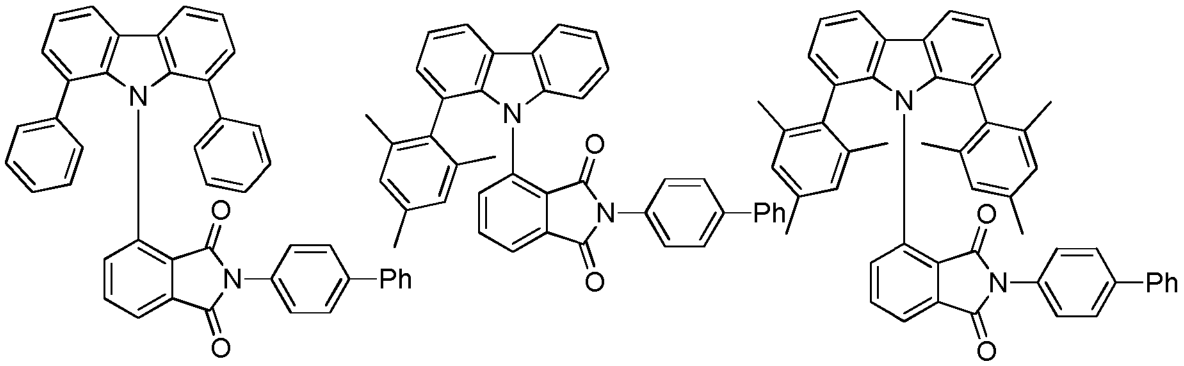

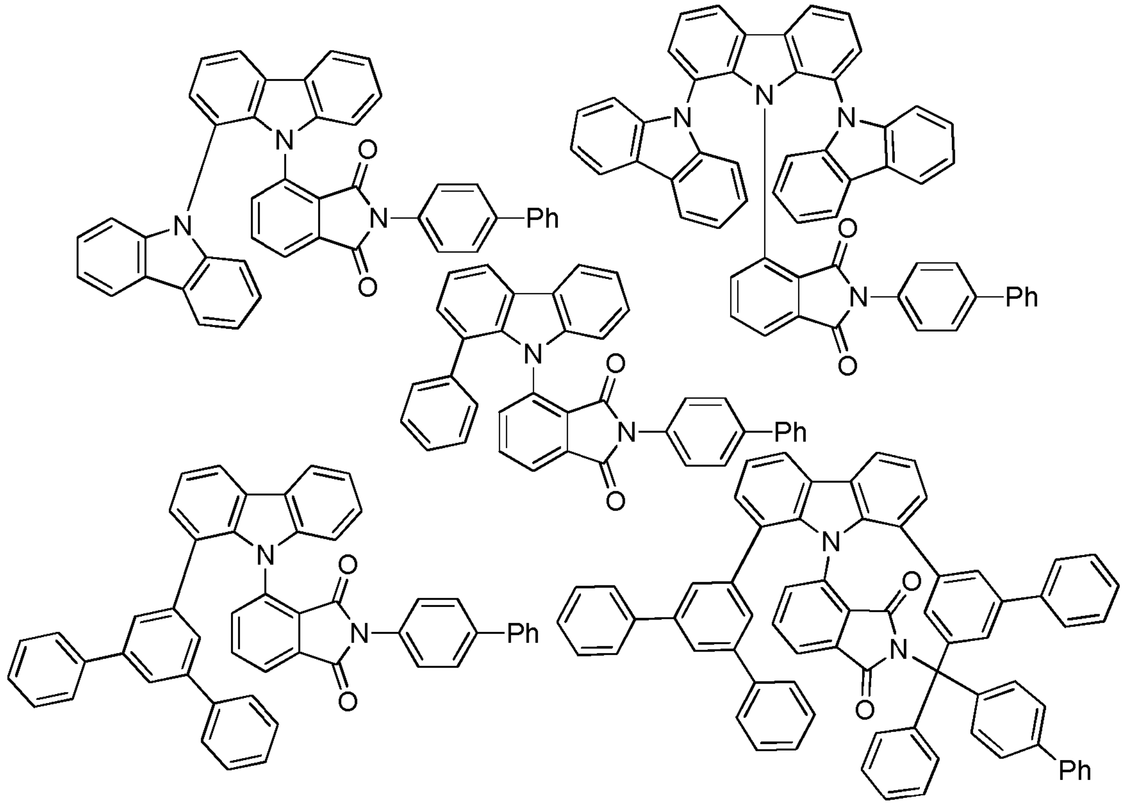

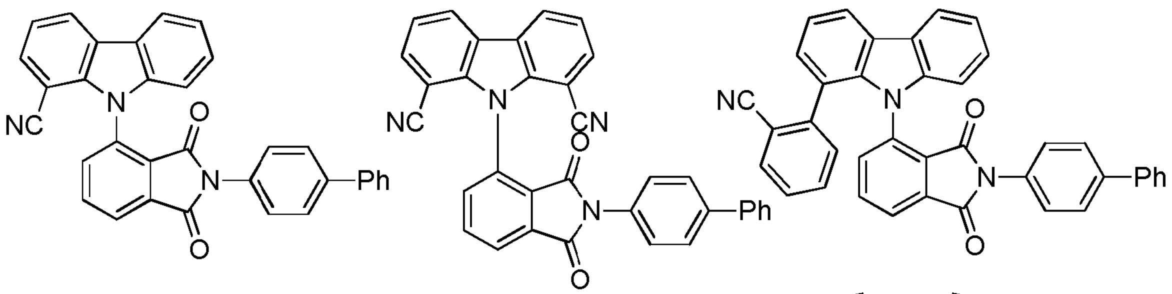

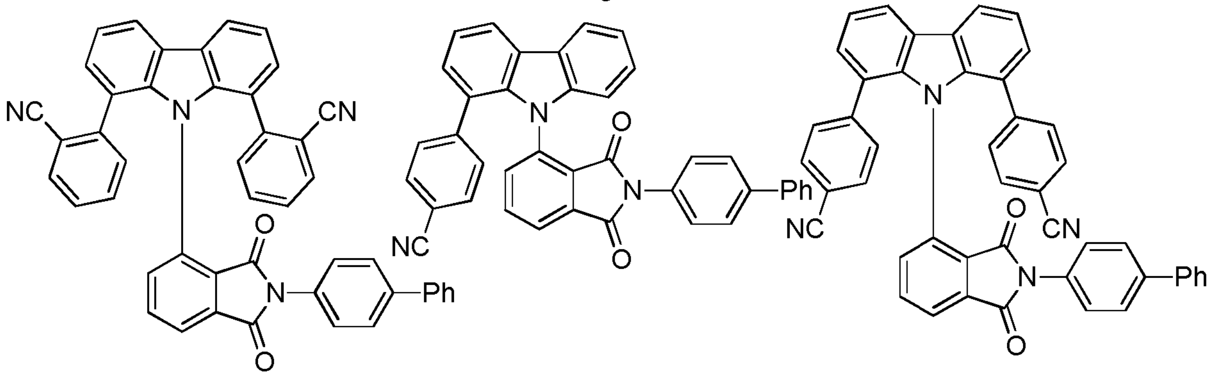

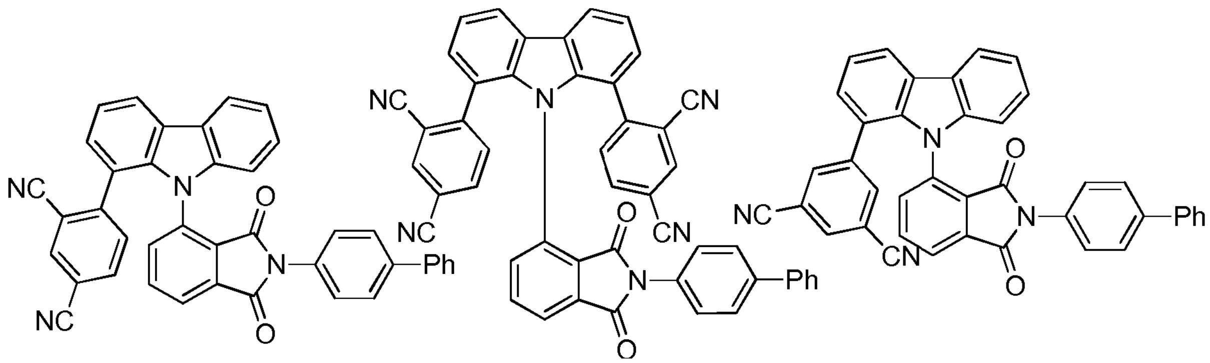

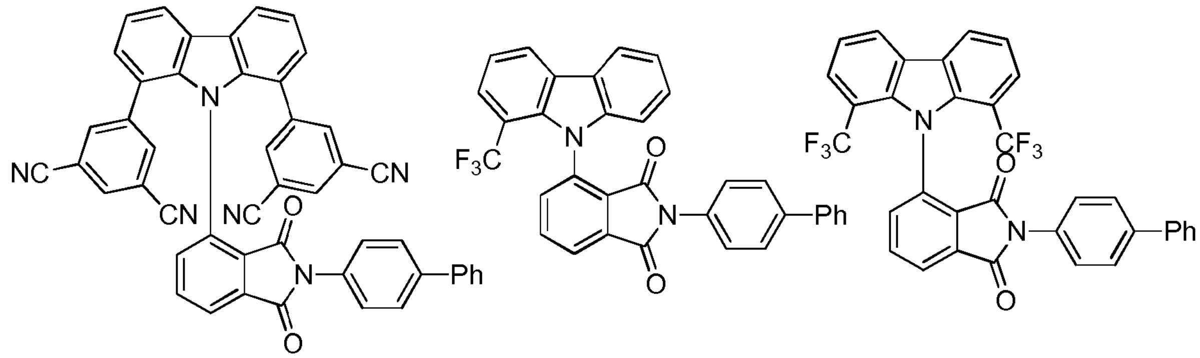

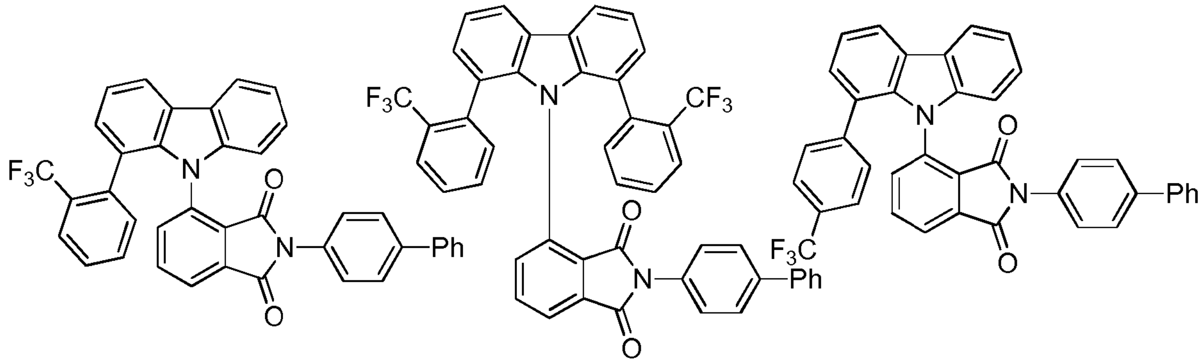

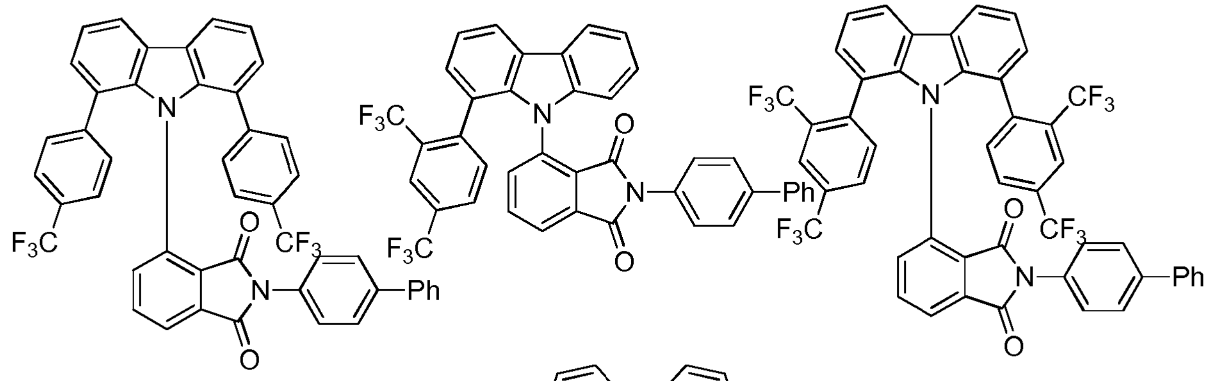

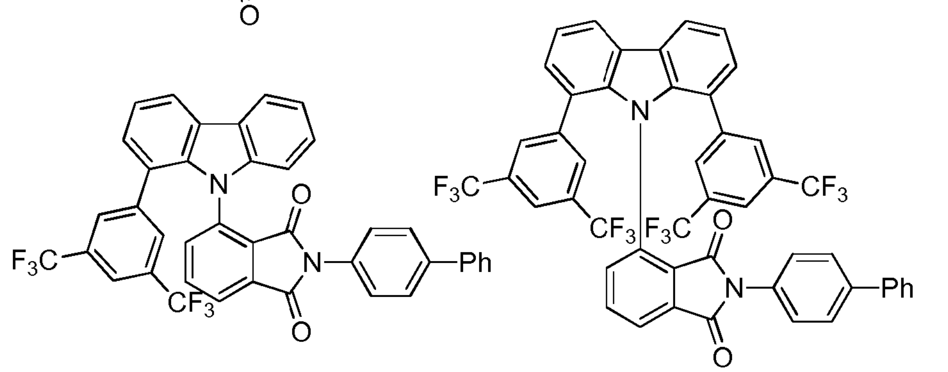

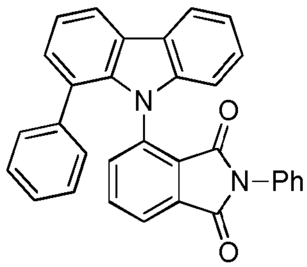

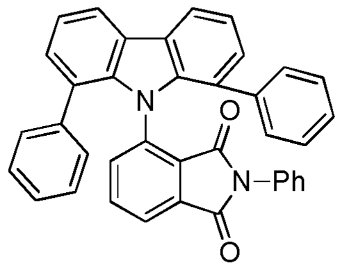

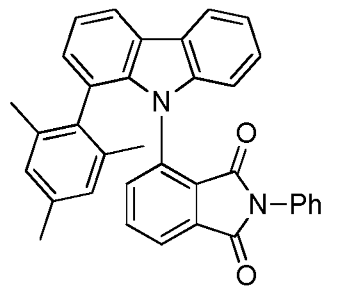

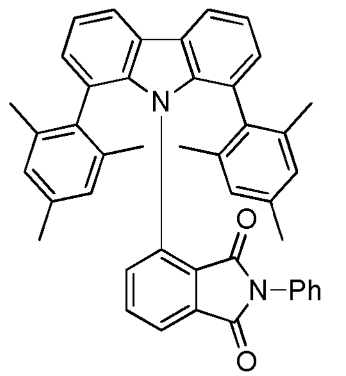

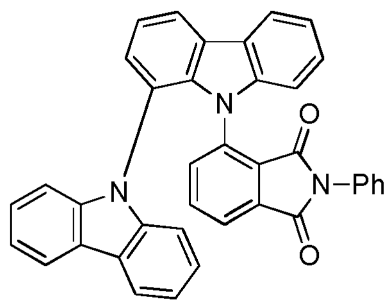

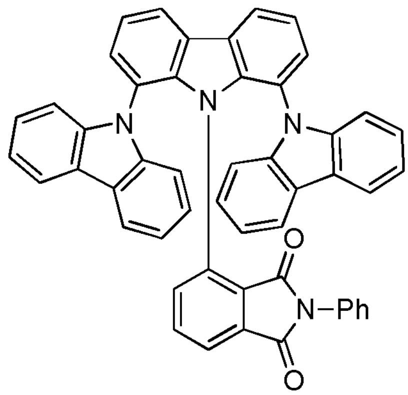

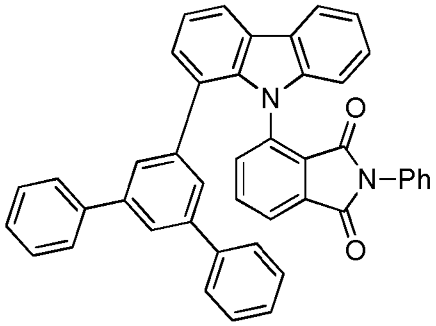

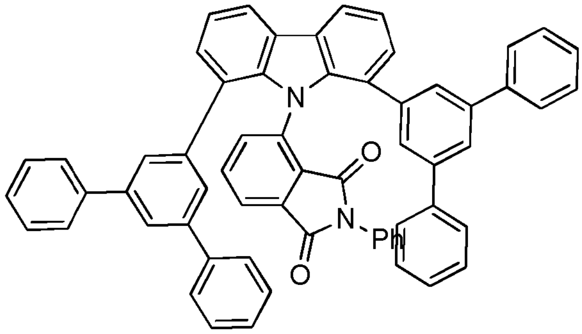

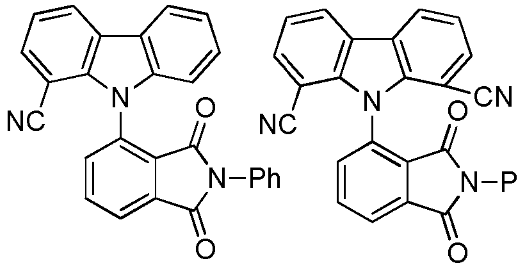

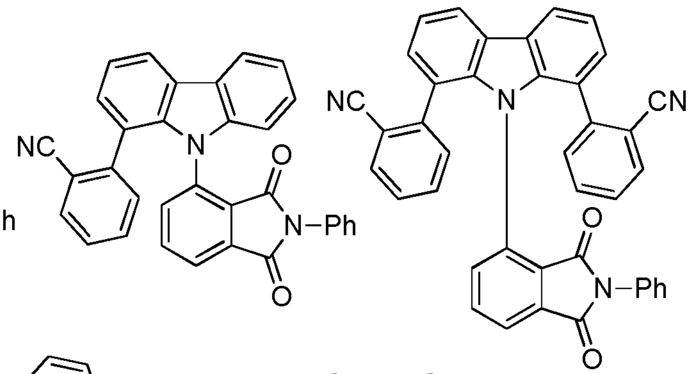

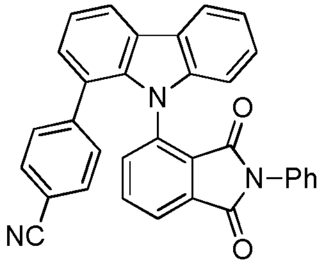

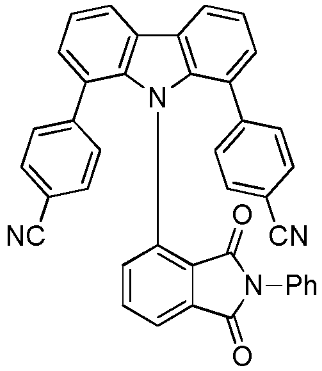

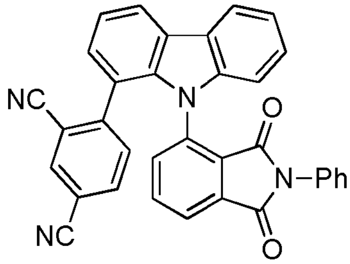

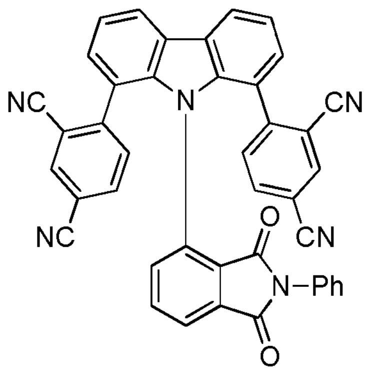

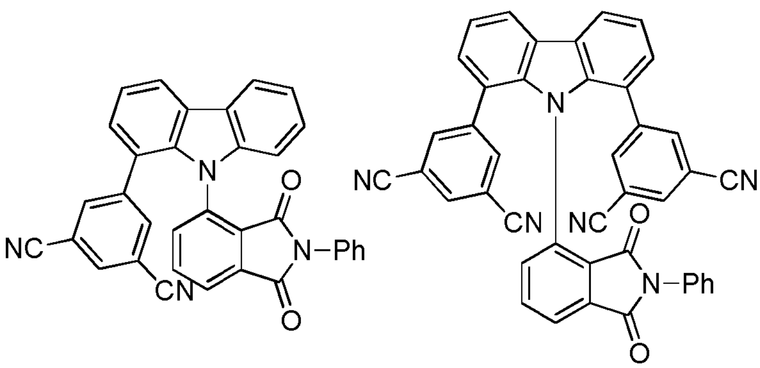

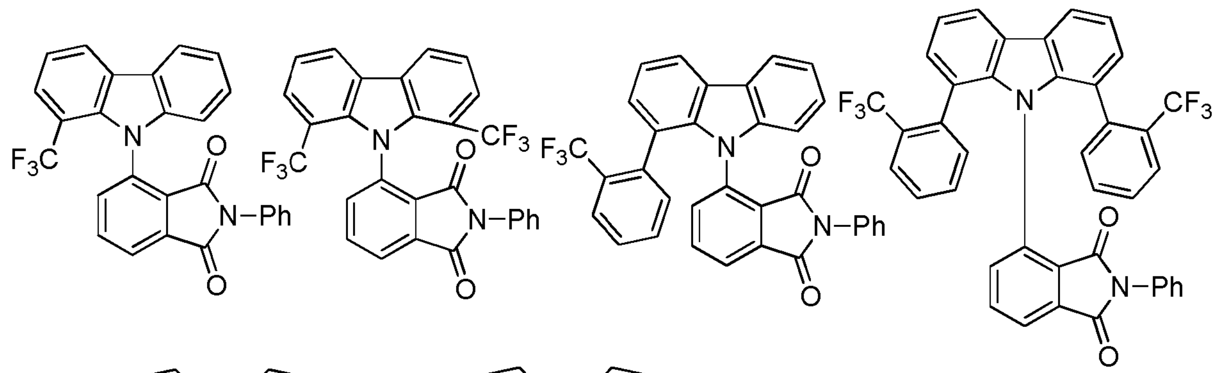

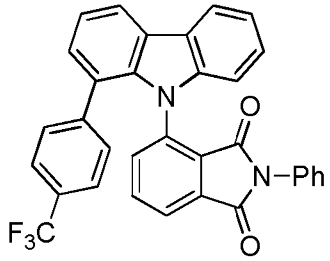

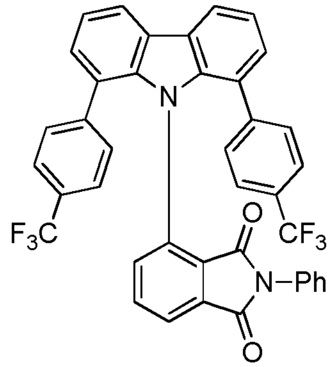

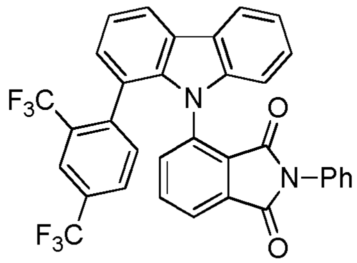

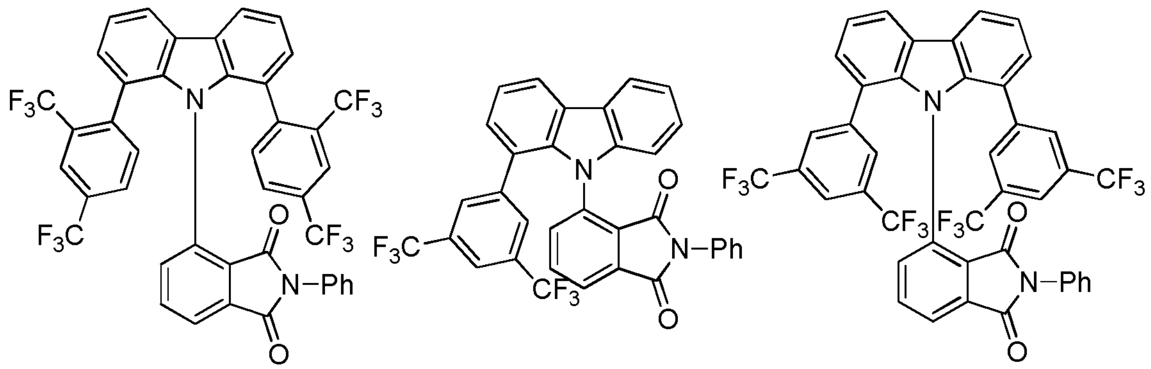

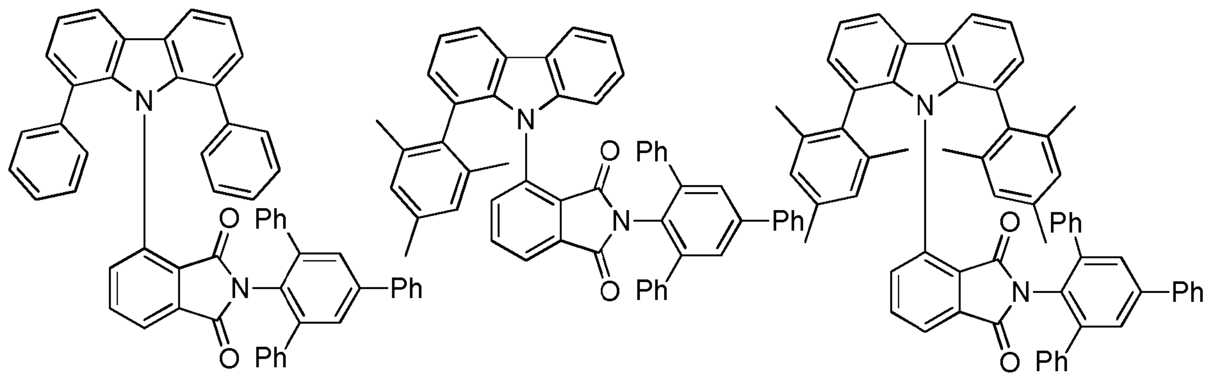

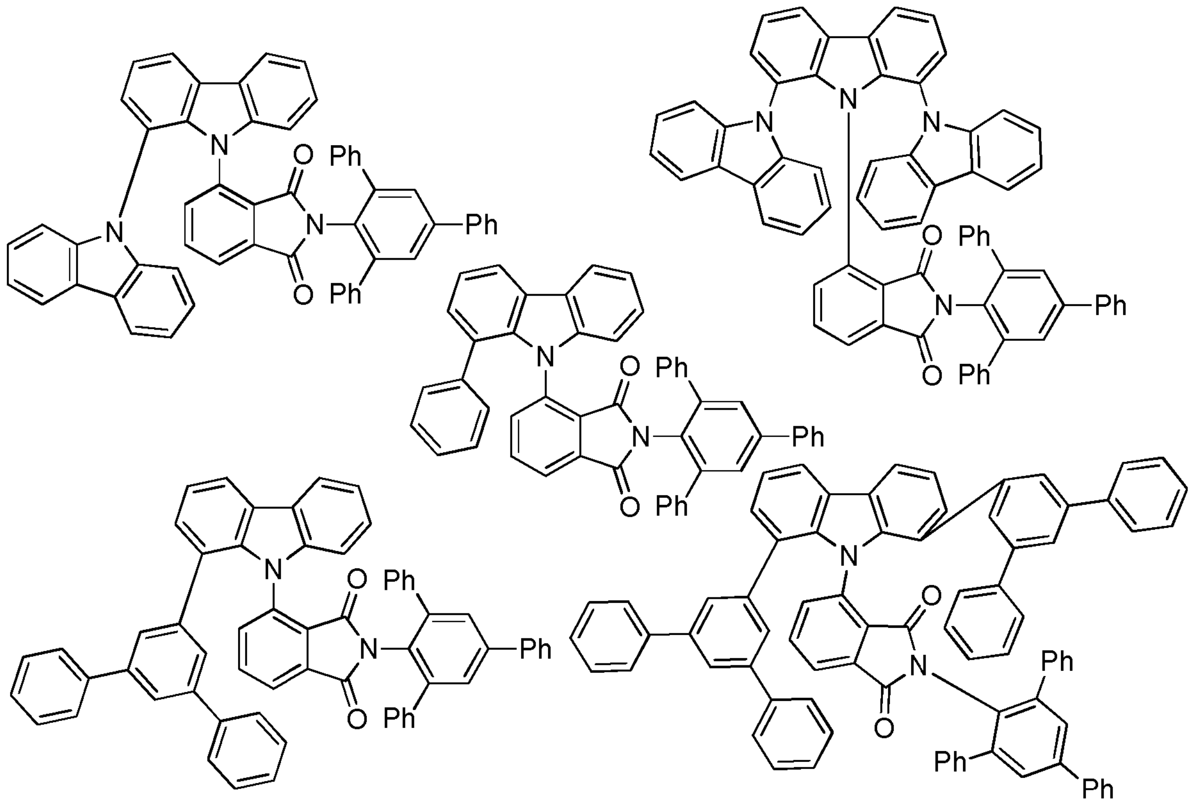

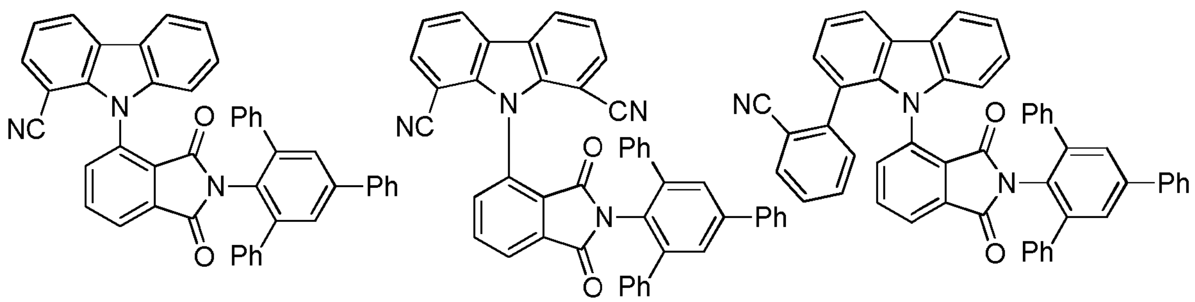

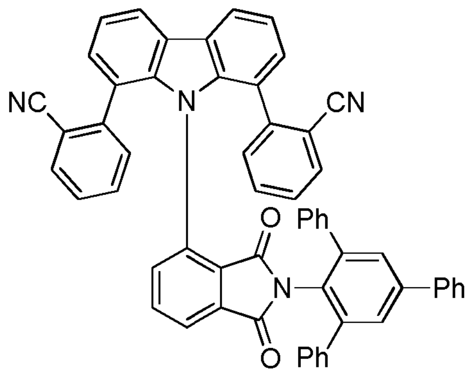

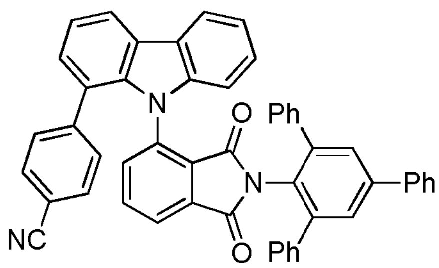

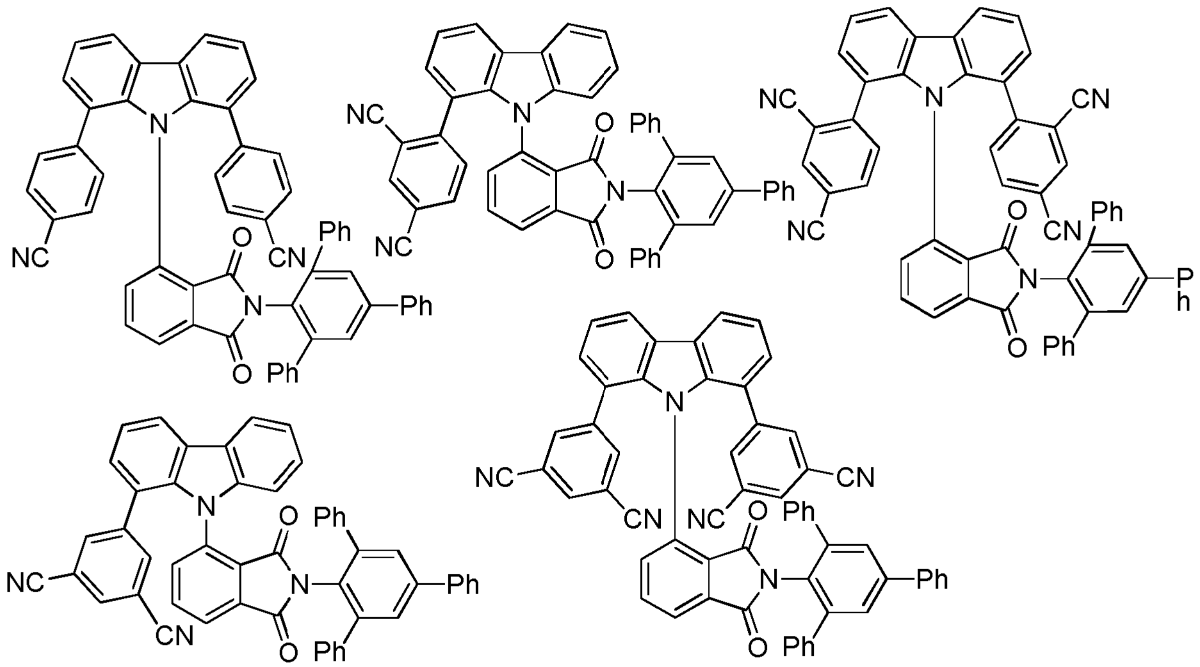

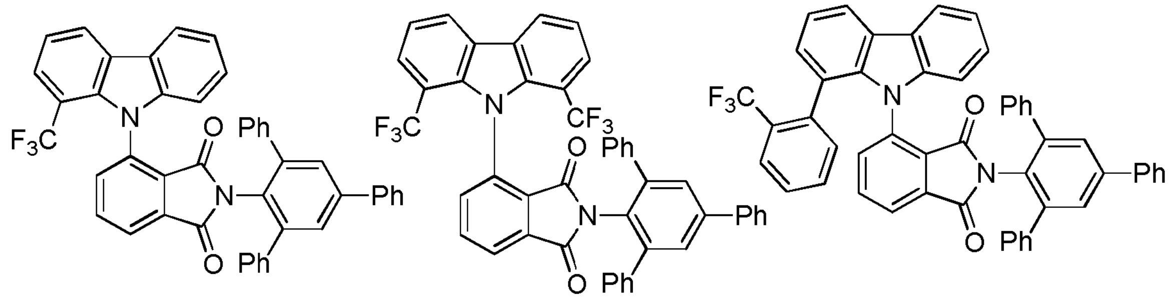

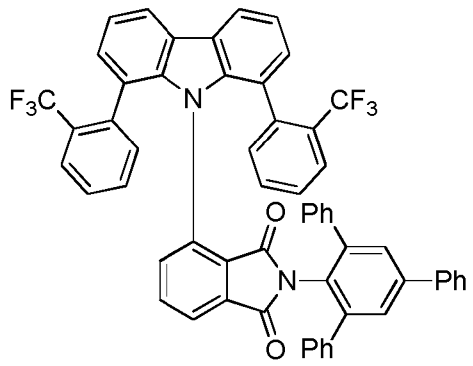

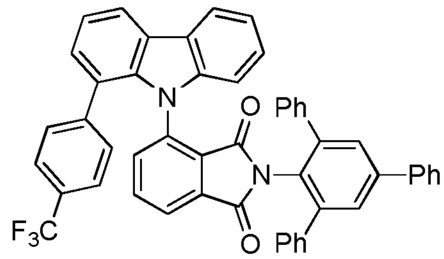

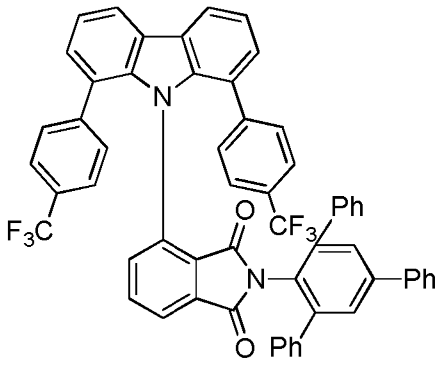

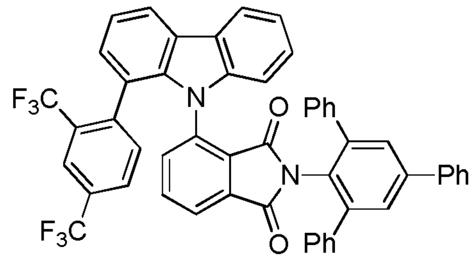

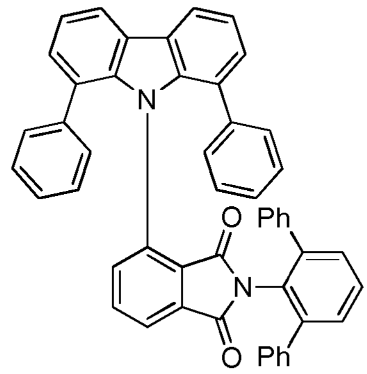

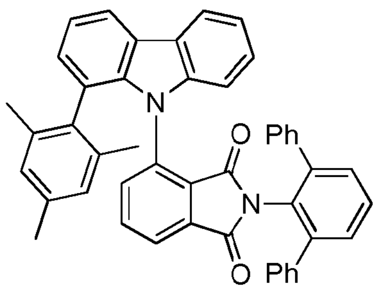

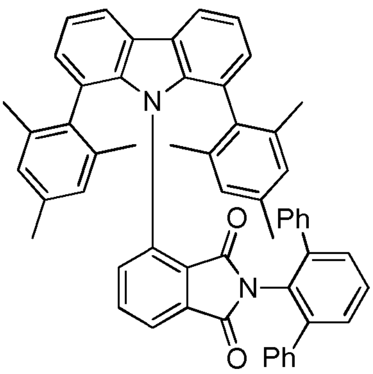

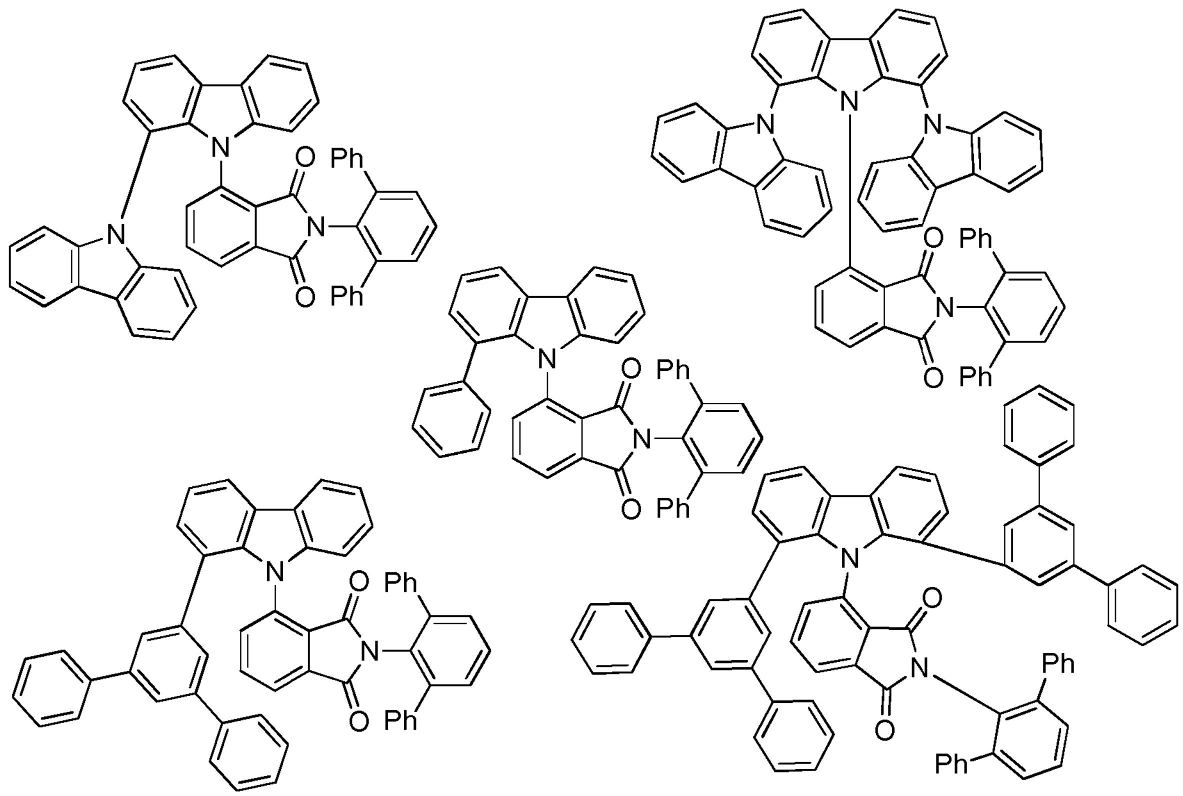

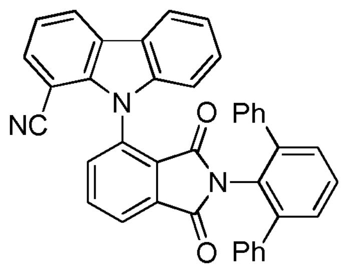

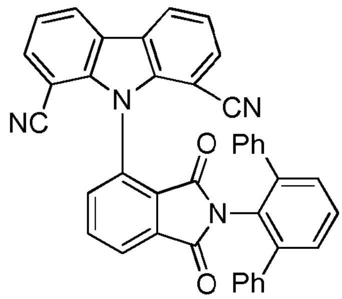

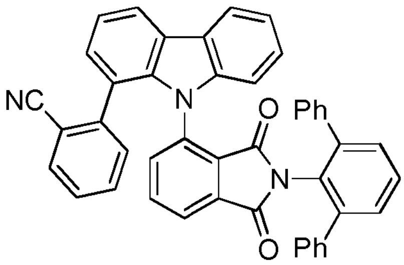

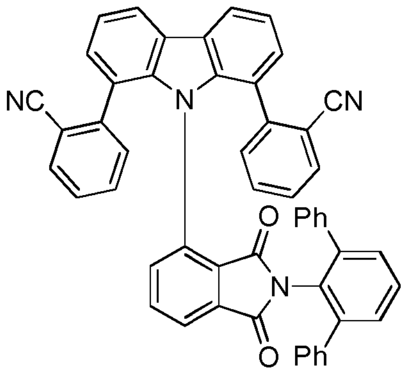

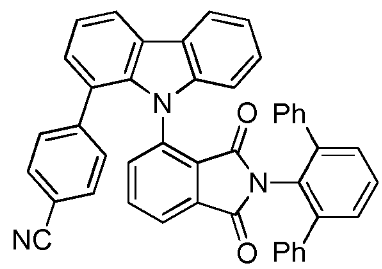

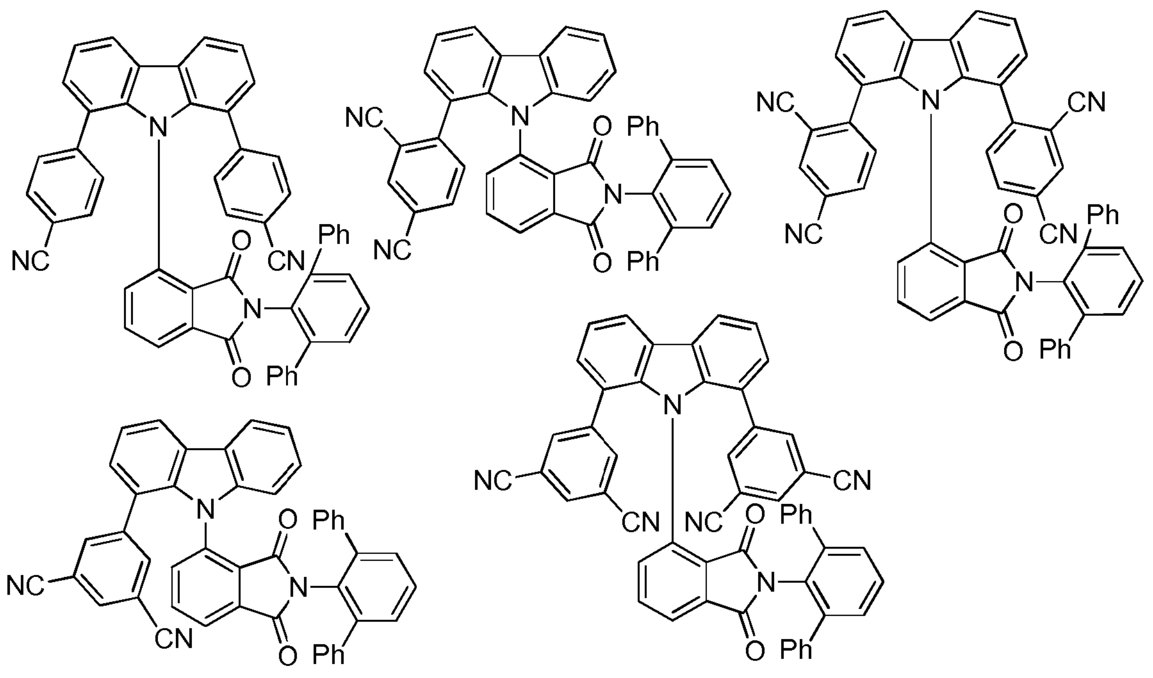

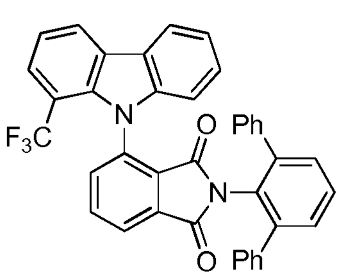

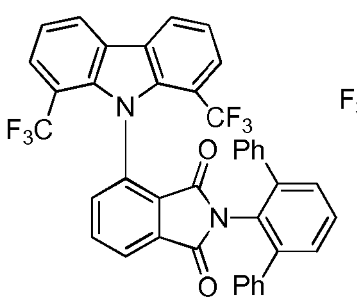

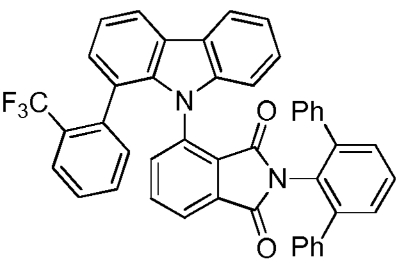

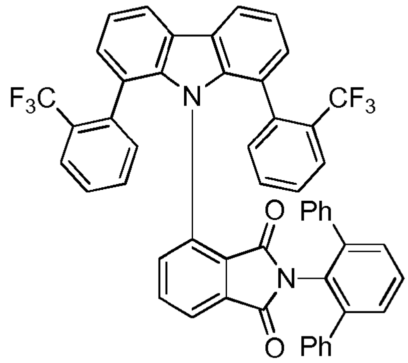

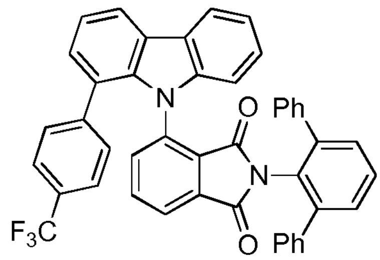

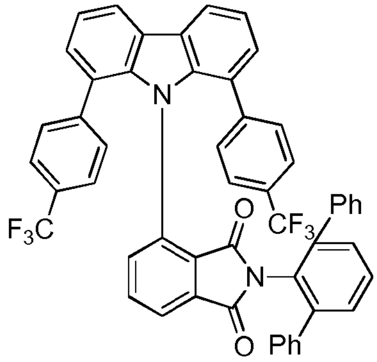

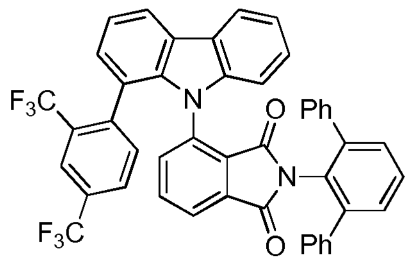

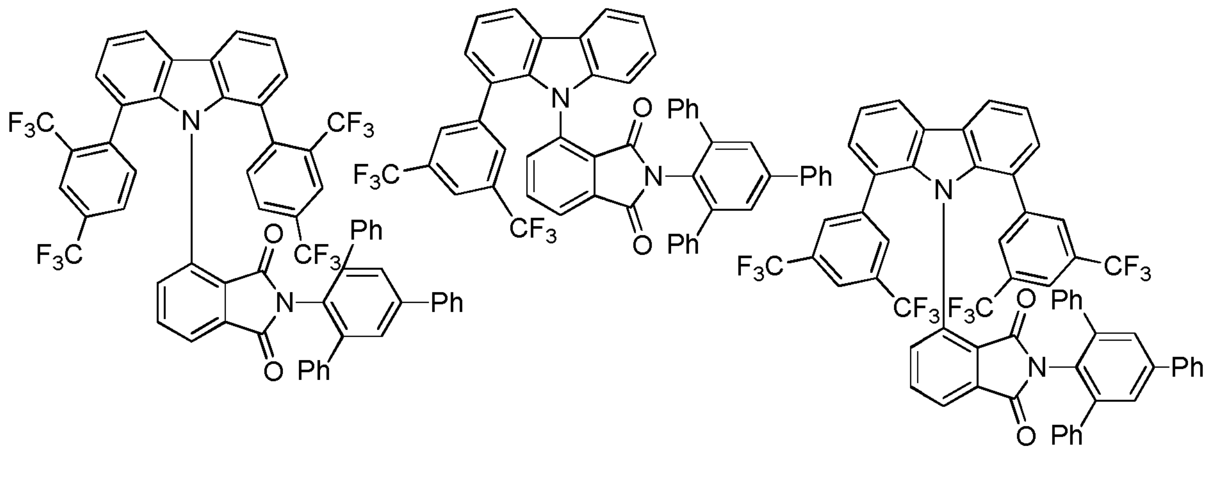

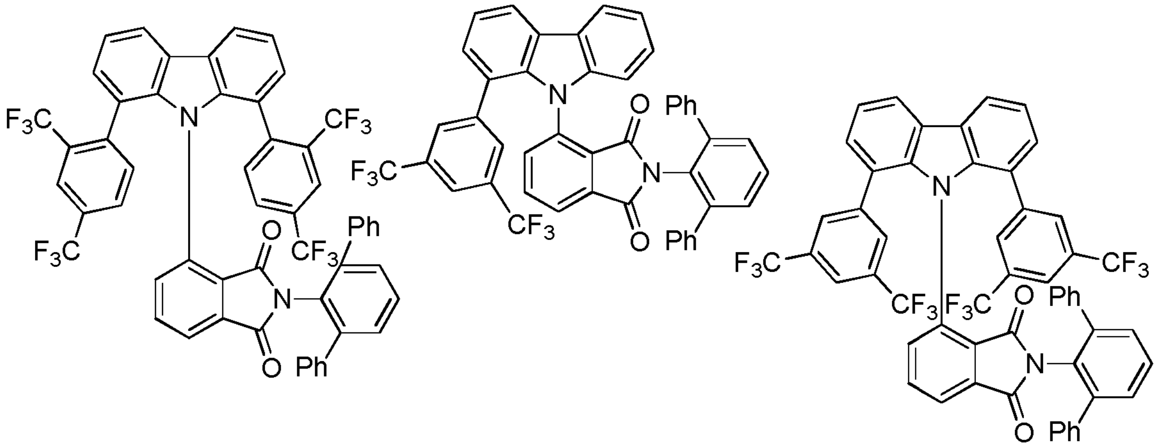

**Figuren**

Es zeigen:

Figur 1 Emissionsspektrum von 1 (10 % in PMMA).
Figur 2 Emissionsspektrum von 2 (10 % in PMMA).
Figur 3 Emissionsspektrum von 3 (10 % in PMMA).
Figur 4 Emissionsspektrum von 4 (10 % in PMMA).
Figur 5 Emissionsspektrum von 5 (10 % in PMMA).
Figur 6 Emissionsspektrum von 6 (10 % in PMMA).
Figur 7 Emissionsspektrum von 7 (10 % in PMMA).

**Patentansprüche**

**1.** Organisches Molekül, aufweisend

- eine erste chemische Einheit aufweisend eine oder bestehend aus einer Struktur der Formel A1

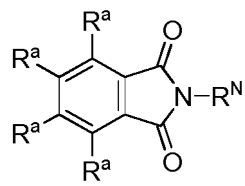

Formel A1

- eine zweite chemische Einheit oder zwei zweite chemische Einheiten aufweisend eine oder bestehend aus einer Struktur der Formel D1

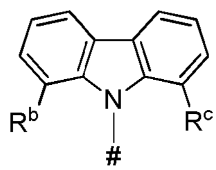

Formel D1

mit

# = Anknüpfungspunkt der zweiten chemischen Einheit an die erste chemische Einheit;
$R^N$ ist Methyl, Phenyl, Xylyl, Mesityl, Naphtyl, Biphenyl, Naphthylphenyl, Terphenyl oder 2,4,6-Triphenylphenyl;
$R^a$ ist bei jedem Auftreten gleich oder verschieden ausgewählt aus der Gruppe bestehend aus:

Anknüpfungspunkt der ersten chemischen Einheit an die zweite chemische Einheit, H, Deuterium, eine Alkylgruppe, und eine unsubstituierte oder mit einem oder mehreren $R^4$ substituierte Arylgruppe, wobei ein oder zwei $R^a$ einen Anknüpfungspunkt der ersten chemischen Einheit an die zweite chemische Einheit darstellt;
$R^b$ ist ausgewählt aus der Gruppe bestehend aus $CF_3$, $C(=O)R^1$, CN, unsubstituiertem oder mit einem oder mehreren $R^2$ substituiertem Carbazolyl, einer unsubstituierten oder mit einem oder mehreren $R^2$ substituierten Arylgruppe und einer unsubstituierten oder mit einem oder mehreren $R^2$ substituierten Heteroarylgruppe;
$R^c$ ist H, Deuterium oder $R^b$;
$R^1$ ist bei jedem Auftreten eine unsubstituierte oder mit einer oder mehreren Resten $R^2$ substituierte Arylgruppe;
$R^2$ ist bei jedem Auftreten unabhängig voneinander ausgewählt aus der Gruppe bestehend aus $CF_3$,

C(=O)$R^1$, CN, einer unsubstituierten oder mit einem oder mehreren $R^3$ substituierten Alkylgruppe, einer unsubstituierten oder mit einem oder mehreren $R^3$ substituierten Arylgruppe und einer unsubstituierten oder mit einem oder mehreren $R^3$ substituierten Heteroarylgruppe;
$R^3$ ist bei jedem Auftreten unabhängig voneinander ausgewählt aus der Gruppe bestehend aus $CF_3$, C(=O)$R^1$, CN, einer unsubstituierten Alkylgruppe, einer unsubstituierten Arylgruppe und einer unsubstituierten Heteroarylgruppe;
$R^4$ ist bei jedem Auftreten unabhängig voneinander ausgewählt aus der Gruppe bestehend aus F, $CF_3$, C(=O)$R^1$, CN, einer unsubstituierten Alkylgruppe, einer unsubstituierten Arylgruppe und einer unsubstituierten Heteroarylgruppe;
dabei können zwei oder mehrere dieser Substituenten $R^1$, $R^2$ und $R^3$ auch miteinander ein mono- oder polycyclisches, aliphatisches, aromatisches und/oder benzoannelliertes Ringsystem bilden.

**2.** Organisches Molekül nach Anspruch 1, aufweisend eine Struktur der Formel A2

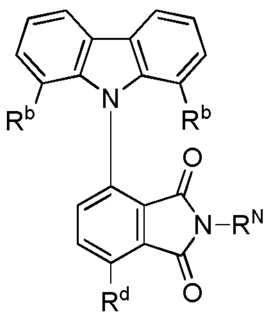

Formel A2

wobei

$R^d$ ausgewählt ist aus der Gruppe bestehend aus H und einem Anknüpfungspunkt an eine Gruppe der Formel A2a

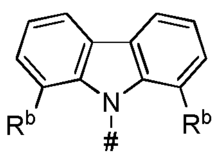

Formel A2a

mit # = Anknüpfungspunkt der chemischen Einheit der Formel A2a an die Einheit der Formel A2; und
$R^b$ und $R^N$ wie in Anspruch 1 definiert sind.

**3.** Organisches Molekül nach Anspruch 1, aufweisend eine Struktur der Formel A3

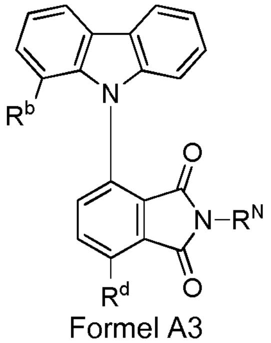

Formel A3

wobei

$R^d$ ausgewählt ist aus der Gruppe bestehend aus H und einem Anknüpfungspunkt an eine Gruppe der Formel A2b

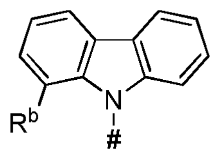

Formel A2b

mit
# = Anknüpfungspunkt der chemischen Einheit der Formel A2b an die Einheit der Formel A3; und
$R^b$ und $R^N$ wie in Anspruch 1 definiert sind.

**4.** Organisches Molekül nach Anspruch 1, aufweisend eine Struktur der Formel A4

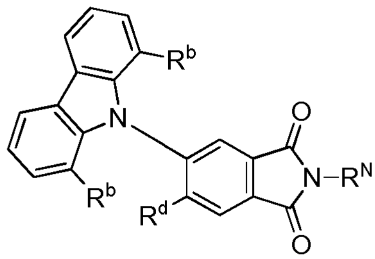

Formel A4

wobei

$R^d$ ausgewählt ist aus der Gruppe bestehend aus H und einem Anknüpfungspunkt an eine Gruppe der Formel A2a

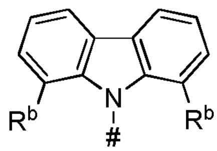

Formel A2a

mit
# = Anknüpfungspunkt der chemischen Einheit der Formel A2a an die Einheit der Formel A4; und
$R^b$ und $R^N$ wie in Anspruch 1 definiert sind.

**5.** Organisches Molekül nach Anspruch 1, aufweisend eine Struktur der Formel A5

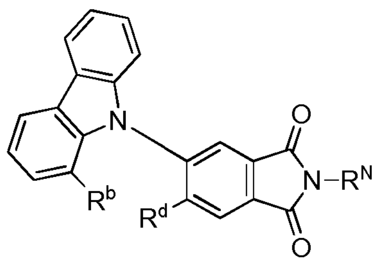

Formel A5

wobei

$R^d$ ausgewählt ist aus der Gruppe bestehend aus H und einem Anknüpfungspunkt an eine Gruppe der Formel A2b

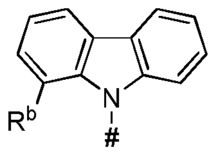

Formel A2b

mit
# = Anknüpfungspunkt der chemischen Einheit der Formel A2b an die Einheit der Formel A5; und
$R^b$ und $R^N$ wie in Anspruch 1 definiert sind.

**6.** Organisches Molekül nach Anspruch 1 bis 5, wobei $R^b$ bei jedem Auftreten unabhängig voneinander ausgewählt ist aus der Gruppe bestehend aus einer unsubstituierten Arylgruppe, einer mit einem oder mehreren $R^2$ substituierten Arylgruppe, einer unsubstituierten Hetreroarylgruppe und einer mit $R^2$ substituierten Heteroarylgruppe, wobei ansonsten die in Anspruch 1 angegebenen Definitionen gelten.

**7.** Organisches Molekül nach Anspruch 1 bis 5, wobei $R^b$ bei jedem Auftreten unabhängig voneinander ausgewählt ist aus der Gruppe bestehend aus einer unsubstituierten Arylgruppe, einer mit einem oder mehreren $R^5$ substituierten Arylgruppe, einer unsubstituierten Heteroarylgruppe und einer mit $R^5$ substituierten Heteroarylgruppe, wobei $R^5$ bei jedem Auftreten unabhängig voneinander ausgewählt ist aus der Gruppe bestehend aus $CF_3$, CN, einer unsubstituierten Alkylgruppe, einer unsubstituierten Arylgruppe und einer unsubstituierten Heteroarylgruppe und ansonsten die in Anspruch 1 angegebenen Definitionen gelten.

**8.** Verwendung eines organischen Moleküls nach Anspruch 1 bis 7 als Emitter und/ oder Host.

**9.** Zusammensetzung aufweisend oder bestehend aus:

(a) mindestens einem organischen Molekül nach einem der Ansprüche 1 bis 7, insbesondere als Emitter und/oder Host, und
(b) einer oder mehrerer von dem Molekül nach einem der Ansprüche 1 bis 7 verschiedenen Emitter- und/oder Hostmaterialien und
(c) optional einem oder mehreren Farbstoffen und/ oder einem oder mehreren Lösungsmitteln.

**10.** Optoelektronisches Bauelement, aufweisend ein organisches Molekül nach Anspruch 1 bis 7 oder eine Zusammensetzung nach Anspruch 9.

**11.** Optoelektronisches Bauelement nach Anspruch 10, aufweisend

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die das organisches Molekül nach Anspruch 1 bis 7 oder eine Zusammensetzung nach Anspruch 9 aufweist.

**12.** Verfahren zur Herstellung eines optoelektronischen Bauelements, wobei ein organisches Molekül nach Anspruch 1 bis 7 verwendet wird.

**13.** Verfahren nach Anspruch 12, wobei das organische Molekül durch ein Verdampfungsverfahren oder aus einer Lösung aufgebracht wird.

**Claims**

1. Organic molecule comprising

- a first chemical unit comprising or consisting of a structure of formula A1

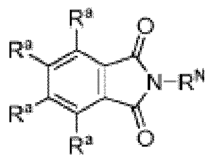

Formula A1

- one second chemical unit or two second chemical units comprising or consisting of a structure of formula D1

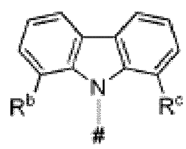

Formula D1

with

# = point of attachment of the second chemical unit to the first chemical unit;
$R^N$ is methyl, phenyl, xylyl, mesityl, naphthyl, biphenyl, naphthylphenyl, terphenyl or 2,4,6-triphenylphenyl;
$R^a$ is the same or different at each instance and is selected from the group consisting of:

point of attachment of the first chemical unit to the second chemical unit, H, deuterium, an alkyl group, and an aryl group which is unsubstituted or substituted by one or more $R^4$;
where one or two $R^a$ constitute(s) a point of attachment of the first chemical unit to the second chemical unit;
$R^b$ is selected from the group consisting of $CF_3$, $C(=O)R^1$, CN, carbazolyl which is unsubstituted or substituted by one or more $R^2$, an aryl group which is unsubstituted or substituted by one or more $R^2$, and a heteroaryl group which is unsubstituted or substituted by one or more $R^2$;
$R^c$ is H, deuterium or $R^b$;
$R^1$ at each instance is an aryl group which is unsubstituted or substituted by one or more $R^2$ radicals;
$R^2$ at each instance is independently selected from the group consisting of $CF_3$, $C(=O)R^1$, CN, an alkyl group which is unsubstituted or substituted by one or more $R^3$, an aryl group which is unsubstituted or substituted by one or more $R^3$, and a heteroaryl group which is unsubstituted or substituted by one or more $R^3$;
$R^3$ at each instance is independently selected from the group consisting of $CF_3$, $C(=O)R^1$, CN, an unsubstituted alkyl group, an unsubstituted aryl group and an unsubstituted heteroaryl group;
$R^4$ at each instance is independently selected from the group consisting of F, $CF_3$, $C(=O)R^1$, CN, an unsubstituted alkyl group, an unsubstituted aryl group and an unsubstituted heteroaryl group;
wherein two or more of these substituents $R^1$, $R^2$ and $R^3$ may form a mono- or polycyclic, aliphatic, aromatic and/or benzofused ring system.

2. Organic molecule according to Claim 1, comprising a structure of formula A2

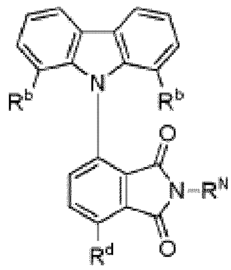

Formula A2

where

$R^d$ is selected from the group consisting of H and a point of attachment to a group of formula A2a

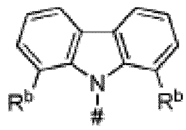

Formula A2a

with # = point of attachment of the chemical unit of the formula A2a to the unit of the formula A2; and $R^b$ and $R^N$ are as defined in Claim 1.

**3.** Organic molecule according to Claim 1, comprising a structure of formula A3

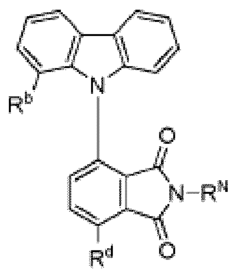

Formula A3

where

$R^d$ is selected from the group consisting of H and a point of attachment to a group of formula A2b

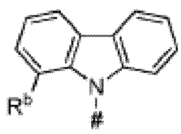

Formula A2b

with

# = point of attachment of the chemical unit of the formula A2b to the unit of the formula A3; and

$R^b$ and $R^N$ are as defined in Claim 1.

**4.** Organic molecule according to Claim 1, comprising a structure of formula A4

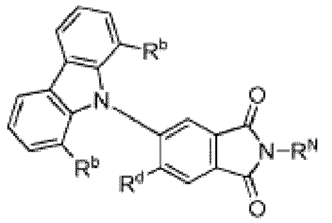

Formula A4

where

$R^d$ is selected from the group consisting of H and a point of attachment to a group of formula A2a

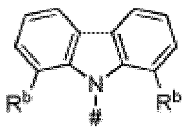

Formula A2a

with
# = point of attachment of the chemical unit of the formula A2a to the unit of the formula A4; and
$R^b$ and $R^N$ are as defined in Claim 1.

**5.** Organic molecule according to Claim 1, comprising a structure of formula A5

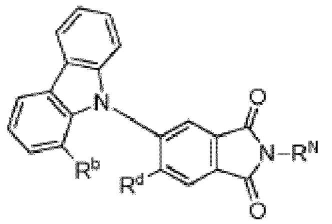

Formula A5

where

$R^d$ is selected from the group consisting of H and a point of attachment to a group of formula A2b

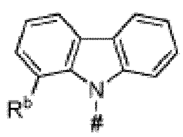

Formula A2b

with
# = point of attachment of the chemical unit of the formula A2b to the unit of the formula A5; and
$R^b$ and $R^N$ are as defined in Claim 1.

**6.** Organic molecule according to Claims 1 to 5, where $R^b$ at each instance is independently selected from the group consisting of an unsubstituted aryl group, an aryl group substituted by one or more $R^2$, an unsubstituted heteroaryl group, and a heteroaryl group substituted by $R^2$, where the definitions given in Claim 1 are otherwise applicable.

**7.** Organic molecule according to Claims 1 to 5, where $R^b$ at each instance is independently selected from the group consisting of an unsubstituted aryl group, an aryl group substituted by one or more $R^5$, an unsubstituted heteroaryl

group, and a heteroaryl group substituted by $R^5$,

where $R^5$ at each instance is independently selected from the group consisting of $CF_3$, CN, an unsubstituted alkyl group, an unsubstituted aryl group and an unsubstituted heteroaryl group,
and the definitions given in Claim 1 are otherwise applicable.

**8.** Use of an organic molecule according to Claims 1 to 7 as emitter and/or host.

**9.** Composition including or consisting of:

(a) at least one organic molecule according to any of Claims 1 to 7, in particular as emitter and/or host, and
(b) one or more emitter and/or host materials other than the molecule according to any of Claims 1 to 7 and
(c) optionally one or more dyes and/or one or more solvents.

**10.** Optoelectronic component including an organic molecule according to Claims 1 to 7 or a composition according to Claim 9.

**11.** Optoelectronic component according to Claim 10, comprising

- a substrate,
- an anode and
- a cathode, wherein the anode or the cathode is applied to the substrate,
and
- at least an light-emitting layer which is disposed between the anode and the cathode and which comprises the organic molecule according to Claims 1 to 7 or the composition according to Claim 9.

**12.** Method for producing an optoelectronic component, wherein an organic molecule according to Claims 1 to 7 is used.

**13.** Method according to Claim 12, wherein the organic molecule is applied by an evaporation method or from a solution.

**Revendications**

**1.** Molécule organique, comprenant

- un premier motif chimique ou constitué d'une structure de formule A1

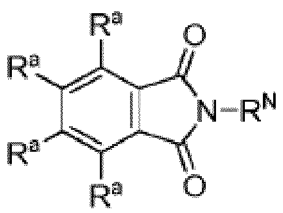

formule A1

- un deuxième motif chimique ou deux deuxièmes motifs chimiques comprenant ou constitué(s) d'une structure de formule D1

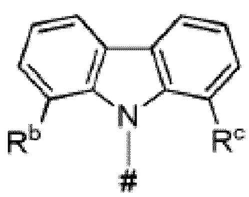

formule D1

avec

# = point de connexion du deuxième motif chimique au premier motif chimique ;
$R^N$ étant méthyle, phényle, xylyle, mésityle, naphtyle, biphényle, naphtylphényle, terphényle ou 2,4,6-triphénylphényle ;
$R^a$, identique ou différent en chaque occurrence, étant choisi dans le groupe constitué par :

un point de connexion du premier motif chimique au deuxième motif chimique, H, deutérium, un groupe alkyle, et un groupe aryle non substitué ou substitué par un ou plusieurs $R^4$,
un ou deux $R^a$ recomprenant un point de connexion du premier fragment chimique au deuxième fragment chimique ;
$R^b$ étant choisi dans le groupe constitué par $CF_3$, C(=O)$R^1$, CN, carbazolyle non substitué ou substitué par un ou plusieurs $R^2$, un groupe aryle non substitué ou substitué par un ou plusieurs $R^2$ et un groupe hétéroaryle non substitué ou substitué par un ou plusieurs $R^2$ ;
$R^c$ étant H, deutérium ou $R^b$ ;
$R^1$ étant en chaque occurrence un groupe aryle non substitué ou substitué par un ou plusieurs $R^2$ ;
$R^2$ étant en chaque occurrence indépendamment l'un de l'autre choisi dans le groupe constitué par $CF_3$, C(=O)$R^1$, CN, un groupe alkyle non substitué ou substitué par un ou plusieurs $R^3$, un groupe aryle non substitué ou substitué par un ou plusieurs $R^3$ et un groupe hétéroaryle non substitué ou substitué par un ou plusieurs $R^3$ ;
$R^3$ étant en chaque occurrence indépendamment l'un de l'autre choisi dans le groupe constitué par $CF_3$, C(=O)$R^1$, CN, un groupe alkyle non substitué, un groupe aryle non substitué et un groupe hétéroaryle non substitué ;
$R^4$ étant en chaque occurrence indépendamment l'un de l'autre choisi dans le groupe constitué par F, $CF_3$, C(=O)$R^1$, CN, un groupe alkyle non substitué, un groupe aryle non substitué et un groupe hétéroaryle non substitué ;
à cet égard, deux ou plus de ces substituants $R^1$, $R^2$ et $R^3$ peuvent également former ensemble un système cyclique monocyclique ou polycyclique, aliphatique, aromatique et/ou benzoannelé.

**2.** Molécule organique selon la revendication 1, comprenant une structure de formule A2

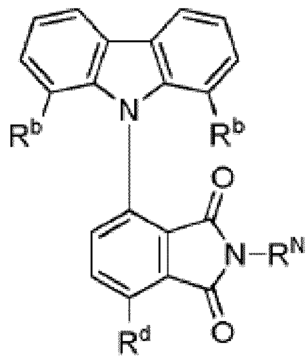

formule A2

$R^d$ étant choisi dans le groupe constitué par H et un point de connexion à un groupe de formule A2a

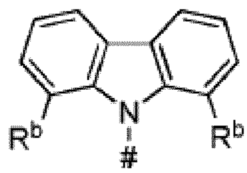

formule A2a

avec # = point de connexion du motif chimique de formule A2a au motif de formule A2 ; et
$R^b$ et $R^N$ étant définis comme dans la revendication 1.

**3.** Molécule organique selon la revendication 1, comprenant une structure de formule A3

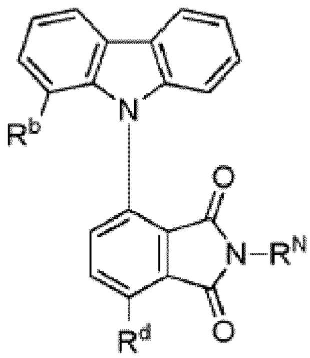

formule A3

$R^d$ étant choisi dans le groupe constitué par H et un point de connexion à un groupe de formule A2b

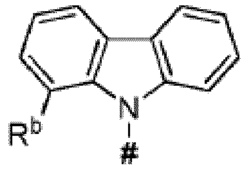

formule A2b

avec
# = point de connexion du motif chimique de formule A2b au motif de formule A3 ; et
$R^b$ et $R^N$ étant définis comme dans la revendication 1.

**4.** Molécule organique selon la revendication 1, comprenant une structure de formule A4

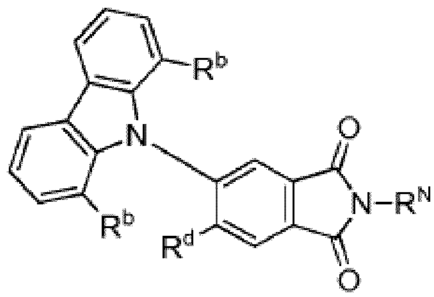

formule A4

$R^d$ étant choisi dans le groupe constitué par H et un point de connexion à un groupe de formule A2a

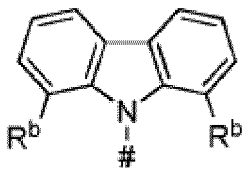

formule A2a

avec # = point de connexion du motif chimique de formule A2a au motif de formule A4 ; et

$R^b$ et $R^N$ étant définis comme dans la revendication 1.

**5.** Molécule organique selon la revendication 1, comprenant une structure de formule A5

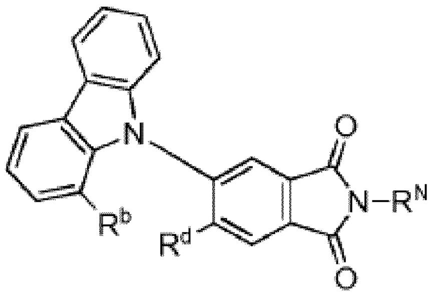

formule A5

$R^d$ étant choisi dans le groupe constitué par H et un point de connexion à un groupe de formule A2b

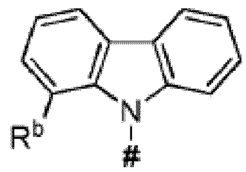

formule A2b

avec
# = point de connexion du motif chimique de formule A2b au motif de formule A5 ; et
$R^b$ et $R^N$ étant définis comme dans la revendication 1.

**6.** Molécule organique selon les revendications 1 à 5, $R^b$ étant choisi indépendamment l'un de l'autre en chaque occurrence dans le groupe constitué par un groupe aryle non substitué, un groupe aryle substitué par un ou plusieurs $R^2$, un groupe hétéroaryle non substitué et un groupe hétéroaryle substitué par $R^2$, mis à part cela, les définitions indiquées dans la revendication 1 s'appliquant.

**7.** Molécule organique selon les revendications 1 à 5, $R^b$ étant choisi indépendamment l'un de l'autre en chaque occurrence dans le groupe constitué par un groupe aryle non substitué, un groupe aryle substitué par un ou plusieurs $R^5$, un groupe hétéroaryle non substitué et un groupe hétéroaryle substitué par $R^5$, $R^5$ étant choisi indépendamment l'un de l'autre en chaque occurrence dans le groupe constitué par $CF_3$, CN, un groupe alkyle non substitué, un groupe aryle non substitué et un groupe hétéroaryle non substitué
et mis à part cela, les définitions indiquées dans la revendication 1 s'appliquant.

**8.** Utilisation d'une molécule organique selon les revendications 1 à 7 en tant qu'émetteur et/ou hôte.

**9.** Composition comprenant ou constituée de :

(a) au moins une molécule organique selon l'une quelconque des revendications 1 à 7, en particulier en tant qu'émetteur et/ou hôte, et
(b) un ou plusieurs matériaux émetteurs et/ou hôtes différents de la molécule selon l'une quelconque des revendications 1 à 7 et
(c) éventuellement un ou plusieurs colorants et/ou un ou plusieurs solvants.

**10.** Composant optoélectronique, comprenant une molécule organique selon les revendications 1 à 7 ou une composition selon la revendication 9.

**11.** Composant optoélectronique selon la revendication 10, comprenant

- un substrat,
- une anode et
- une cathode, l’anode ou la cathode étant déposée sur le substrat, et
- au moins une couche luminescente, qui est agencée entre l’anode et la cathode et qui présente la molécule organique selon les revendications 1 à 7 ou une composition selon la revendication 9.

**12.** Procédé pour la préparation d’un composant optoélectronique, une molécule organique selon les revendications 1 à 7 étant utilisée.

**13.** Procédé selon la revendication 12, la molécule organique étant déposée par un procédé d’évaporation ou à partir d’une solution.

**Figur 1**

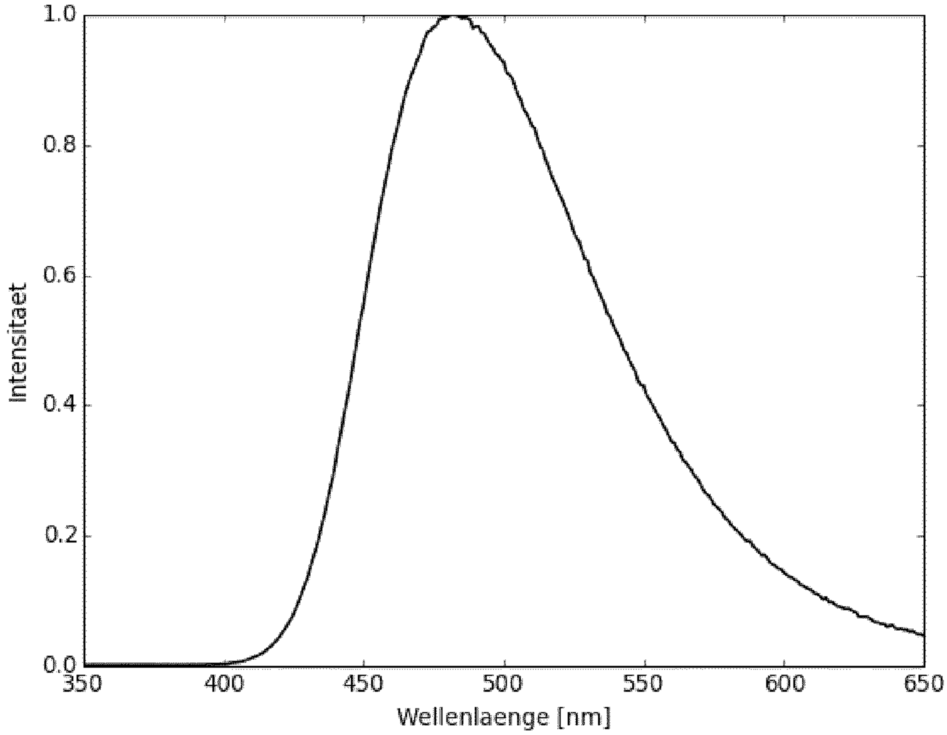


**Figur 2**

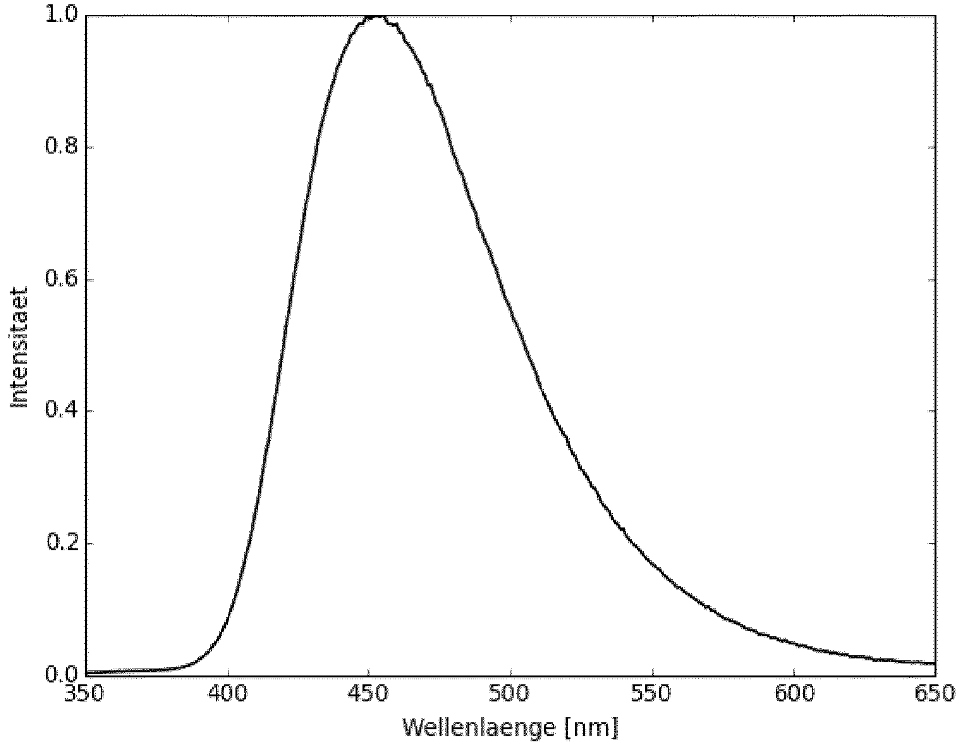

**Figur 3**

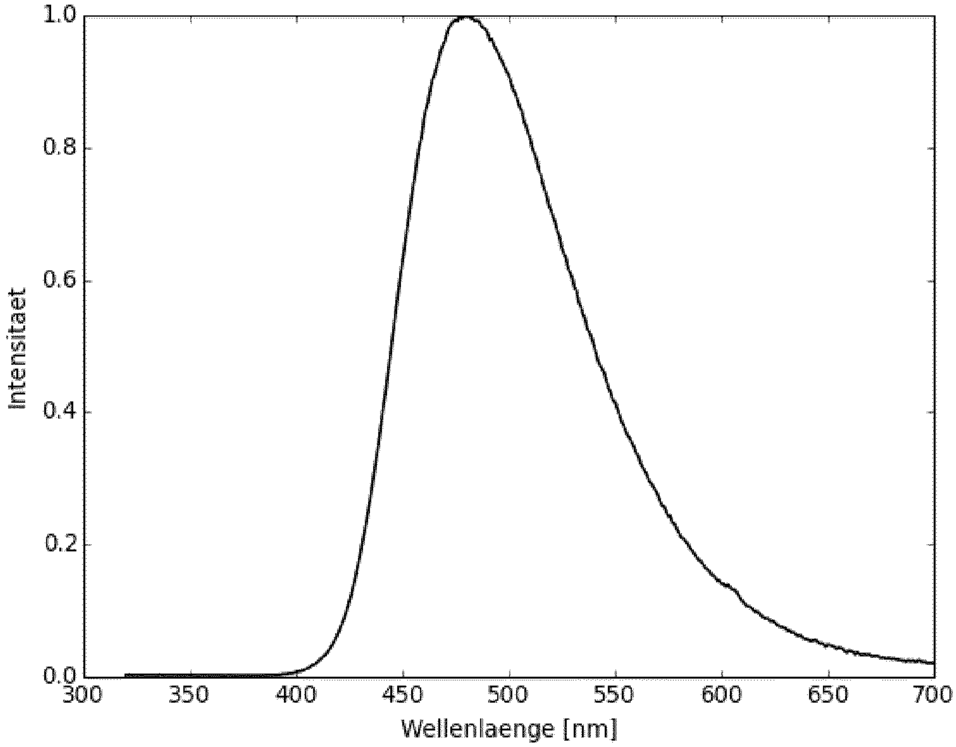


**Figur 4**

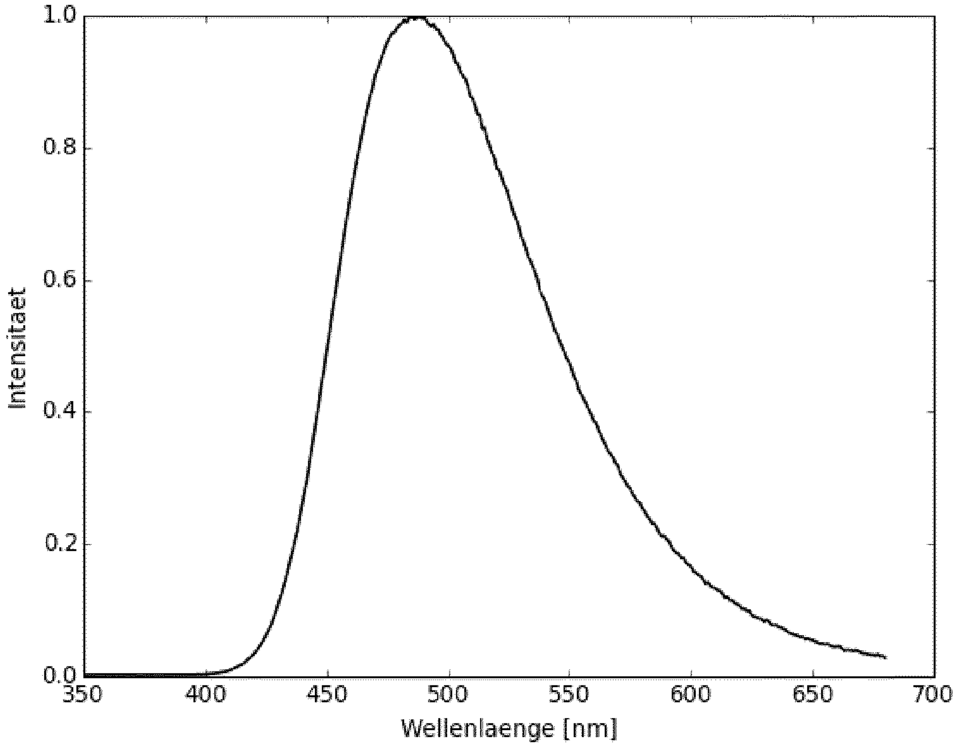

Figur 5

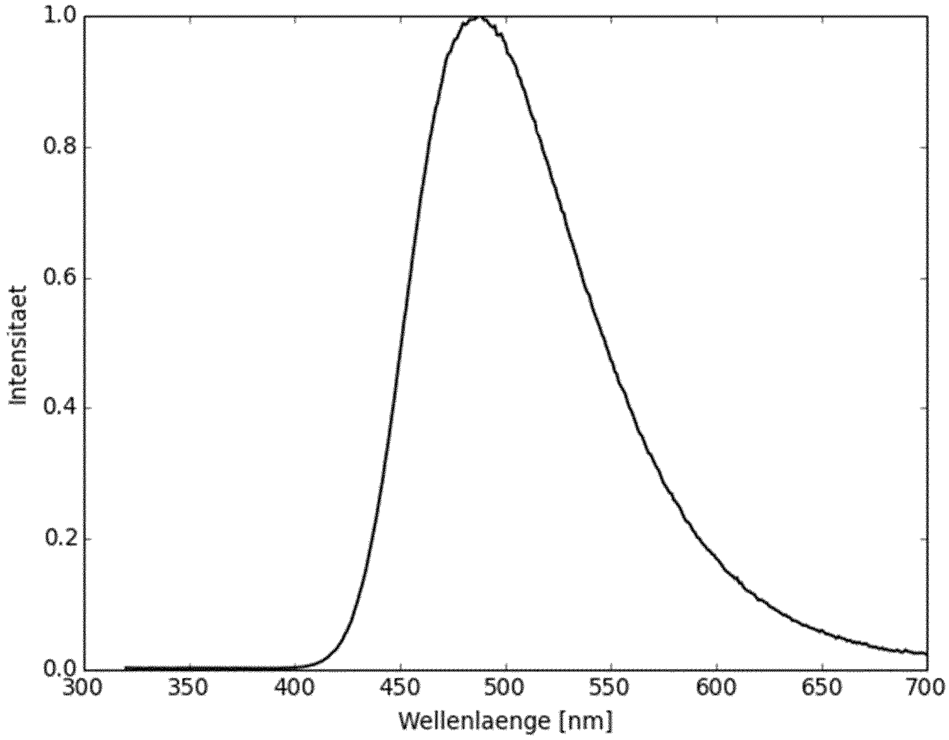


Figur 6

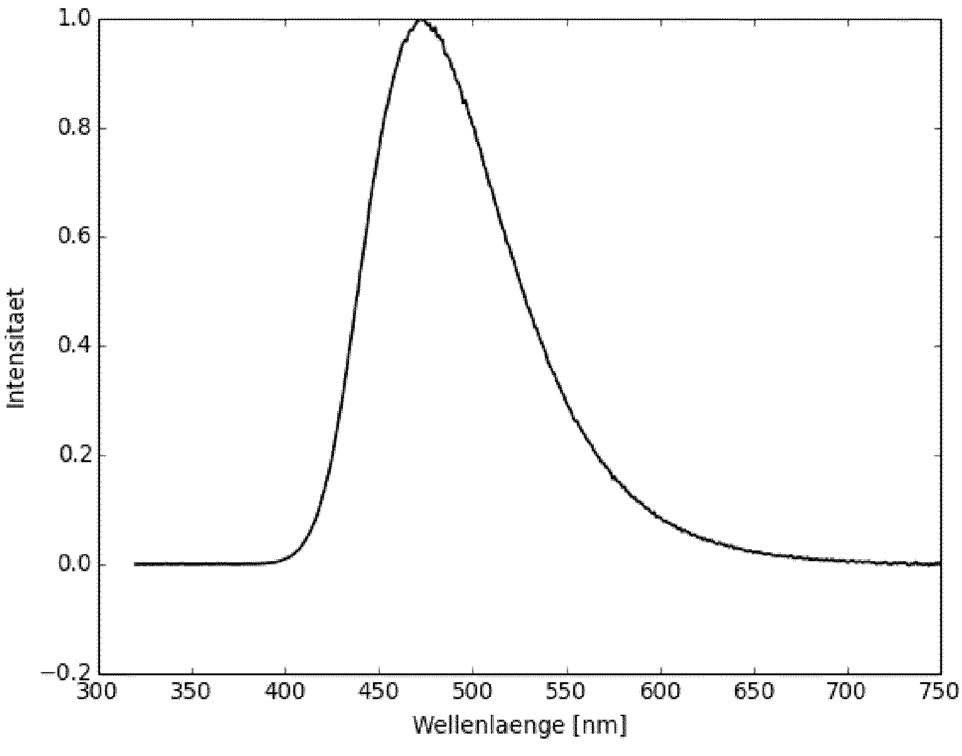

Figur 7

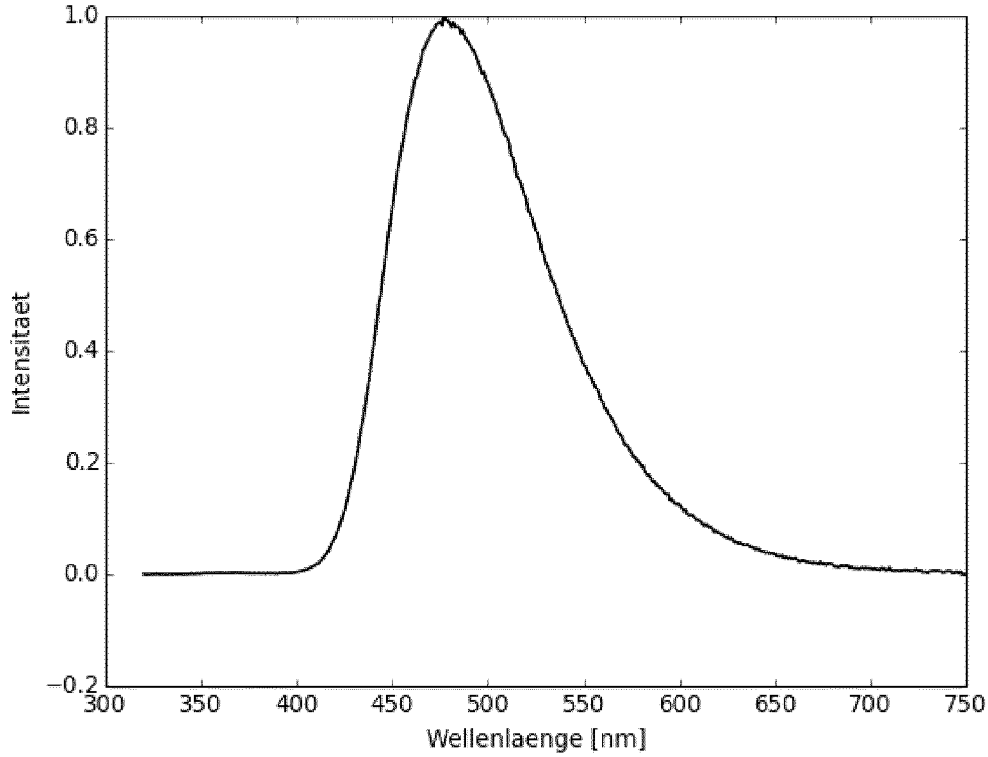
1.0
0.8
0.6
0.4
0.2
0.0
−0.2
Intensitaet
300
350
400
450
500
550
600
650
700
750
Wellenlaenge [nm]

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2010149748 A1, H. Yersin, U. Monkowius, T. Fischer, T. Hofbeck
- WO 2013161437 A1
- WO 2016042070 A


### In der Beschreibung aufgeführte Nicht-Patentliteratur


- **Q. ZHANG et al.** *J. Am. Chem. Soc.,* 2012, vol. 134, 14706
- **B. M.E. THOMPSON et al.** *Chem. Mater.,* 2004, vol. 16, 4743
- **BECKE, A. D.** *Phys. Rev.,* vol. A1988 (38), 3098-3100
- **PERDEW, J. P.** *Phys. Rev. B,* 1986, vol. 33, 8822-8827
- **SIERKA, M. ; HOGEKAMP, A. ; AHLRICHS, R.** *J. Chem. Phys.,* 2003, vol. 118, 9136-9148
- **BECKE, A.D.** *J.Chem.Phys.,* 1993, vol. 98, 5648-5652
- **LEE, C ; YANG, W ; PARR, R.G.** *Phys. Rev. B,* 1988, vol. 37, 785-789
- **VOSKO, S. H. ; WILK, L. ; NUSAIR, M.** *Can. J. Phys.,* 1980, vol. 58, 1200-1211
- **STEPHENS, P. J. ; DEVLIN, F. J. ; CHABALOWSKI, C. F. ; FRISCH, M. J.** *J.Phys.Chem.,* 1994, vol. 98, 11623-11627
- **WEIGEND, F. ; AHLRICHS, R.** *Phys. Chem. Chem. Phys.,* 2005, vol. 7, 3297-3305
- **RAPPOPORT, D. ; FURCHE, F.** *J. Chem. Phys.,* 2010, vol. 133, 134105, , 1-134105, 11